# EUROPEAN PATENT SPECIFICATION

(11) **EP 3 233 831 B1**
(45) Date of publication and mention of the grant of the patent: **20.02.2019**
(21) Application number: 15816666.0
(22) Date of filing: 14.12.2015
(51) Int. Cl.: C07D 401/14, C07D 413/12, C07D 413/14, C07D 417/14, C07D 471/08, A61K 31/454, A61P 1/00, A61P 3/00, A61P 9/00, A61P 29/00, A61P 35/00

(54) **NOVEL FXR (NR1H4) MODULATING COMPOUNDS**
NEUARTIGE FXR (NR1H4) MODULIERENDE VERBINDUNGEN
NOUVEAUX COMPOSÉS DE MODULATION (FXR NR1H4)

(30) Priority: 17.12.2014 EP 14004260
(43) Date of publication of application: 25.10.2017
(73) Proprietor: Gilead Sciences, Inc., Foster City, CA 94404 (US)
(72) Inventor: KINZEL, Olaf, 69117 Heidelberg (DE); KREMOSER, Claus, 69121 Heidelberg (DE); BLOMGREN, Peter, A., Foster City, CA 94404 (US); CURRIE, Kevin, S., Foster City, CA 94404 (US); KROPF, Jeffrey, E., Foster City, CA 94404 (US); SCHMITT, Aaron, Hamden, Connecticut 06518 (US); WATKINS, William, J., Foster City, CA 94404 (US); XU, Jianjun, Foster City, CA 94404 (US); GEGE, Christian, 89584 Ehingen (DE)
(74) Representative: Wallace, Sheila Jane
(86) International application number: PCT/EP2015/002512
(87) International publication number: WO 2016/096116

(56) References cited:
- WO-A1-2009/012125
- WO-A1-2009/081197
- WO-A1-2012/087520
- WO-A1-2012/087521
- WO-A1-2013/007387

### SEQUENCE LISTING

<110> Gilead sciences, Inc. Christian Gege
<120> Novel FXR (NR1H4) Modulating Compounds
<130> PCT98221kG030pau
<140> not yet assigned
   <141> 2014-12-17
<150> EP14004260.7
   <151> 2014-12-17
<160> 1
<170> PatentIn version 3.5
<210> 1
   <211> 25
   <212> PRT
   <213> Artificial sequence
<220>
   <223> synthetic biotinylated peptide based on residues 676-700 of SRC-1 (LCD2, 676-700)
<400> 1
   Cys Pro Ser Ser His Ser Ser Leu Thr Glu Arg His Lys Ile Leu His
   1               5                   10                  15
   Arg Leu Leu Gln Glu Gly Ser Pro Ser
               20                  25

## Description

The present invention relates to compounds which bind to the NR1H4 receptor (FXR) and act as agonists or modulators of FXR. The invention further relates to the use of the compounds for the treatment and/or prophylaxis of diseases and/or conditions through binding of said nuclear receptor by said compounds.

Multicellular organisms are dependent on advanced mechanisms of information transfer between cells and body compartments. The information that is transmitted can be highly complex and can result in the alteration of genetic programs involved in cellular differentiation, proliferation, or reproduction. The signals, or hormones, are often low molecular weight molecules, such as peptides, fatty acid, or cholesterol derivatives.

Many of these signals produce their effects by ultimately changing the transcription of specific genes. One well-studied group of proteins that mediate a cell's response to a variety of signals is the family of transcription factors known as nuclear receptors, hereinafter referred to often as "NR". Members of this group include receptors for steroid hormones, vitamin D, ecdysone, cis and trans retinoic acid, thyroid hormone, bile acids, cholesterol-derivatives, fatty acids (and other peroxisomal proliferators), as well as so-called orphan receptors, proteins that are structurally similar to other members of this group, but for which no ligands are known. Orphan receptors may be indicative of unknown signalling pathways in the cell or may be nuclear receptors that function without ligand activation. The activation of transcription by some of these orphan receptors may occur in the absence of an exogenous ligand and/or through signal transduction pathways originating from the cell surface (D. J. Mangelsdorf et al., Cell 1995, 83, 835; R. M. Evans, Mol. Endocrinol. 2005, 19, 1429).

In general, three functional domains have been defined in NRs. An amino terminal domain is believed to have some regulatory function. It is followed by a DNA-binding domain hereinafter referred to as "DBD" which usually comprises two zinc finger elements and recognizes a specific Hormone Responsive Element hereinafter referred to as "HRE" within the promoters of responsive genes. Specific amino acid residues in the "DBD" have been shown to confer DNA sequence binding specificity (M. Schena and K. R. Yamamoto, Science 1988, 241, 965). A ligand-binding-domain hereinafter referred to as "LBD" is at the carboxy-terminal region of known NRs.

In the absence of hormone, the LBD appears to interfere with the interaction of the DBD with its HRE. Hormone binding seems to result in a conformational change in the NR and thus opens this interference (A. M. Brzozowski et al., Nature 1997, 389, 753). A NR without the LBD constitutively activates transcription but at a low level.

Coactivators or transcriptional activators are proposed to bridge between sequence specific transcription factors, the basal transcription machinery and in addition to influence the chromatin structure of a target cell. Several proteins like SRC-1, ACTR, and Grip1 interact with NRs in a ligand enhanced manner (D. M. Heery et al., Nature 1997, 387, 733; T. Heinzel et al., Nature 1997, 387, 43; K. W. Nettles and G. L. Greene, Annu. Rev. Physiol. 2005, 67, 309).

Nuclear receptor modulators like steroid hormones affect the growth and function of specific cells by binding to intracellular receptors and forming nuclear receptor-ligand complexes. Nuclear receptor-hormone complexes then interact with a HRE in the control region of specific genes and alter specific gene expression (A. Aranda and A. Pascual, Physiol. Rev. 2001, 81, 1269).

The Farnesoid X Receptor alpha (hereinafter also often referred to as NR1H4 when referring to the human receptor) is a prototypical type 2 nuclear receptor which activates genes upon binding to promoter region of target genes in a heterodimeric fashion with Retinoid X Receptor (B. M. Forman et al., Cell 1995, 81, 687). The relevant physiological ligands of NR1H4 are bile acids (D. J. Parks et al., Science 1999, 284, 1365; M. Makishima et al., Science 1999, 284, 1362). The most potent one is chenodeoxycholic acid (CDCA), which regulates the expression of several genes that participate in bile acid homeostasis. Farnesol and derivatives, together called farnesoids, are originally described to activate the rat orthologue at high concentration but they do not activate the human or mouse receptor. FXR is expressed in the liver, throughout the entire gastrointestinal tract including the esophagus, stomach, duodenum, small intestine, colon, ovary, adrenal gland and kidney. Beyond controlling intracellular gene expression, FXR seems to be also involved in paracrine and endocrine signalling by upregulating the expression of the cytokine Fibroblast Growth Factor 15 (rodents) or 19 (monkeys, humans, J. A. Holt et al., Genes Dev. 2003, 17, 1581; T. Inagaki et al., Cell Metab. 2005, 2, 217).

Small molecule compounds which act as FXR modulators have been disclosed in the following publications: WO 2000/037077, WO 2003/015771, WO 2004/048349, WO 2007/076260, WO 2007/092751, WO 2007/140174, WO 2007/140183, WO 2008/051942, WO 2008/157270, WO 2009/005998, WO 2009/012125, WO 2008/025539, WO 2008/025540, WO 2009/005998, WO 2009/012125, WO 2011/020615, WO 2012/087519, WO 2012/087520 and WO 2012/087521. Further small molecule FXR modulators have been recently reviewed (M. L. Crawley, Expert Opin Ther. Pat. 2010, 20,1047; D. Merk et al., Future Med. Chem. 2012, 4, 1015 and C. Gege et al., Curr. Top. Med. Chem. 2014, 14, 2143).

In WO 2013/007387 we disclosed hydroxy containing cyclobutyl and azetidine derivatives of the following general formula 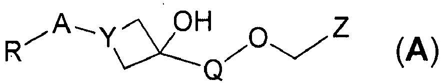 wherein the variables are defined similar as in this application.

Although numerous FXR agonists are disclosed to date, here is still a need to deliver improved FXR agonist.

Said problem has been solved by a compound according to the following Formula (1), an enantiomer, diastereomer, tautomer, solvate or pharmaceutical acceptable salt thereof 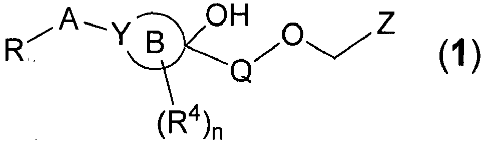 wherein
R is selected from the group consisting of hydrogen, halogen, C₁₋₆-alkyl, C₂₋₆-alkenyl, C₂₋₆-alkynyl, halo-C₁₋₆-alkyl, C₀₋₆-alkylene-R⁷, C₀₋₆-alkylene-O-R⁷, C₀₋₆-alkylene-CN, C₀₋₆-alkylene-NR⁷R⁸, O-C₃₋₁₀-cycloalkyl, O-C₁₋₆-alkylene-O-R⁷, O-C₃₋₁₀-heterocycloalkyl, C₀₋₆-alkylene-CO₂R⁷, C₀₋₆-alkylene-C(O)R⁷, C₀₋₆-alkylene-C(O)NR⁷R⁸, C₀₋₆-alkylene-C(O)NR⁷SO₂R⁷, C₀₋₆-alkylene-N(R⁷)C(O)R⁷, C₀₋₆-alkylene-SOₓ-R⁷, C₀₋₆-alkylene-SO₃H, C₀₋₆-alkylene-SO₂-NR⁷R⁸, C₀₋₆-alkylene-SO₂-NR⁸COR⁷, C₀₋₆-alkylene-N(R⁷)SO₂-R⁸, and C₀₋₆-alkylene-SO₂-C₃₋₁₀-heterocycloalkyl,
   wherein alkylene, cycloalkyl, heterocycloalkyl and the 5- or 6-membered heteroaryl are unsubstituted or substituted by 1 to 4 substituents independently selected from the group consisting of halogen, CN, C₁₋₃-alkyl, halo-C₁₋₃-alkyl, OH, oxo, CO₂H, SO₃H, O-C₁₋₃-alkyl and O-halo-C₁₋₃-alkyl;
R⁷ is independently selected from the group consisting of hydrogen, C₁₋₆-alkyl, halo-C₁₋₆-alkyl, C₀₋₆-alkylene-C₃₋₈-cycloalkyl, C₀₋₆-alkylene-C₃₋₈-heterocycloalkyl, 5- or 6-membered heteroaryl and phenyl, wherein alkyl, alkylene, cyclolalkyl, heterocycloalkyl, phenyl and heteroaryl are unsubstituted or substituted with 1 to 6 substituents independently selected from the group consisting of halogen, CN, OH, oxo, CO₂H, C₁₋₃-alkyl, halo-C₁₋₃-alkyl, O-C₁₋₃-alkyl, O-halo-C₁₋₃-alkyl, SO₃H and SO₂-C₁₋₃-alkyl;
R⁸ is independently selected from the group consisting of hydrogen, C₁₋₆-alkyl, halo-C₁₋₈-alkyl and C₃₋₆-cycloalkyl;
or R⁷ and R⁸ when taken together with the nitrogen to which they are attached may complete a 3- to 8-membered ring containing carbon atoms and optionally containing 1 or 2 heteroatoms selected from O, S or N, wherein the ring is unsubstituted or substituted with 1 to 4 substituents independently selected from the group consisting of fluoro, OH, oxo, C₁₋₄-alkyl and halo-C₁₋₄-alkyl;
A is a 6-10 membered mono- or bicyclic aryl or a 5-10 membered mono- or bicyclic heteroaryl containing 1 to 5 heteroatoms independently selected from the group consisting of N, O and S, wherein aryl and heteroaryl are unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₆-alkyl, C₃₋₆-cycloalkyl and halo-C₃₋₆-cycloalkyl;
B is a C₅₋₈-cycloalkyl ring or, if Y is N, then is B a C₅₋₈-heterocycloalkyl containing one nitrogen atom, and wherein the substituent Q is not directly adjacent to substituent A;
Q is selected from the group consisting of phenyl, pyridyl, thiazolyl, thiophenyl, pyrimidyl, oxazolyl, pyrazolyl, imidazolyl and triazolyl, each unsubstituted or substituted with one or two groups independently selected from the group consisting of halogen, C₁₋₄-alkyl, halo-C₁₋₄-alkyl, C₁₋₄-alkoxy or halo-C₁₋₄-alkoxy;
Y is selected from N, CH or CF;
Z is selected from 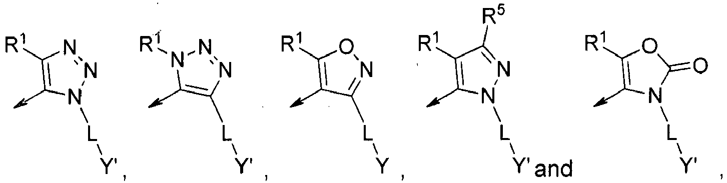 wherein
   L is selected from the group consisting of a bond, C₁₋₃-alkylene and C₁₋₃-alkylene-O-;
   Y' is selected from phenyl, pyridyl, pyridyl-*N*-oxide, pyrimidyl, pyridinonyl, pyrimidinonyl, C₄₋₈-cycloalkyl, and C₄₋₈-heterocycloalkyl, wherein phenyl, pyridyl, pyridyl-*N*-oxide, pyrimidyl, pyridinonyl, pyrimidinonyl, C₄₋₈-cycloalkyl and C₄₋₈-heterocycloalkyl are substituted with R² and R³ and optionally substituted one or two times with a group selected from fluoro, chloro, CN, NH₂, NH(C₁₋₃-alkyl), N(C₁₋₃-alkyl)₂, C₁₋₃-alkyl, fluoro-C₁₋₃-alkyl, OH, C₁₋₃-alkoxy, fluoro-C₁₋₃-alkoxy, C₃₋₆-cycloalkyl and fluoro-C₃₋₆-cycloalkyl;
   R¹ is selected from the group consisting of C₁₋₄-alkyl and C₃₋₆-cycloalkyl, wherein C₁₋₄-alkyl is unsubstituted or substituted with 1 to 3 substituents independently selected from the group consisting of fluoro, hydroxy, C₁₋₃-alkoxy and fluoro-C₁₋₃-alkoxy, and C₃₋₆-cycloalkyl is unsubstituted or substituted with 1 to 3 substituents independently selected from the group consisting of fluoro, hydroxy, C₁₋₃-alkyl, fluoro-C₁₋₃-alkyl, C₁₋₃-alkoxy and fluoro-C₁₋₃-alkoxy;
   R² and R³ are independently selected from the group consisting of hydrogen, halogen, C₁₋₃-alkyl, halo-C₁₋₃-alkyl, C₁₋₃-alkoxy, halo-C₁₋₃-alkoxy, cyclopropyl and fluoro-cyclopropyl;
   R⁴ is independently selected from halogen, C₁₋₃-alkyl, halo-C₁₋₃-alkyl, C₁₋₃-alkoxy, halo-C₁₋₃-alkoxy, C₃₋₆-cycloalkyl, and fluoro-C₃₋₆-cycloalkyl;
   R⁵ is selected from the group consisting of hydrogen, fluoro, CH₃, CHF₂ and CF₃;
   n is selected from 0, 1, 2, 3 and 4; and
   x is selected from 0, 1 and 2.

In another embodiment, the present invention is directed to a compound according to Formula (**1**) as a medicament.

In a further embodiment, the present invention is directed to a compound according to Formula (**1**) for use in the prophylaxis and/or treatment of diseases mediated by FXR.

In another embodiment, the present invention is directed to the use of a compound according to Formula (**1**) for the preparation of a medicament for the prophylaxis and/or treatment of diseases mediated by FXR.

Also disclosed is a method for treating or preventing diseases mediated by FXR in a subject in need thereof, the method comprising administering an effective amount of a compound according to Fromula (1) to the subject.

In another embodiment in combination with any of the above or below embodiments, the disease is selected from chronic intrahepatic or some forms of extrahepatic cholestatic conditions; liver fibrosis; obstructive or chronic inflammatory disorders of the liver; liver cirrhosis; liver steatosis and associated syndromes, cholestatic or fibrotic effects that are associated with alcohol-induced cirrhosis or with viral-borne forms of hepatitis; liver failure or liver ischemia after major liver resection; chemotherapy associated steatohepatitis (CASH); acute liver failure; and/or Inflammatory Bowel Diseases.

In another embodiment in combination with any of the above or below embodiments, the disease is selected from lipid and lipoprotein disorders; Type II Diabetes and clinical complications of Type I and Type II Diabetes, including diabetic nephropathy, diabetic neuropathy, diabetic retinopathy and other observed effects of clinically manifest long term Diabetes; conditions and diseases which result from chronic fatty and fibrotic degeneration of organs due to enforced lipid and specifically triglyceride accumulation and subsequent activation of profibrotic pathways, such as Non-Alcoholic Fatty Liver Disease (NAFLD), or Non-Alcoholic Steatohepatitis (NASH); obesity or metabolic syndrome (combined conditions of dyslipidemia, diabetes or abnormally high body-mass index); and/or cute myocardial infarction, acute stroke or thrombosis which occurs as an endpoint of chronic obstructive atherosclerosis. In another embodiment in combination with any of the above or below embodiments, the disease is selected from non-malignant hyperproliferative disorders and malignant hyperproliferative disorders, specifically of hepatocellular carcinoma, colon adenoma and polyposis, colon adenocarcinoma, breast cancer, pancreas adenocarcinoma, Barrett's esophagus or other forms of neoplastic diseases of the gastrointestinal tract and the liver.

The compounds of the present invention share a common chemical structure according to Formula (**1**) in claim 1.

In a preferred embodiment in combination with any of the above or below embodiments, R in Formula (**1**) is selected from the group consisting of CO₂H, SO₃H, CONR⁷R⁸, tetrazolyl, 1,2,4-oxadiazol-5(4H)-one-3-yl and SO₂NHCOR⁷.

In a further preferred embodiment in combination with any of the above or below embodiments,R⁷ is selected from the group consisting of hydrogen, C₁₋₆-alkyl, halo-C₁₋₆-alkyl, C₁₋₆-alkylene-R⁹, SO₂-C₁₋₃-alkyl.

In a preferred embodiment in combination with any of the above or below embodiments, R⁸ is selected from the group consisting of hydrogen, C₁₋₆-alkyl, halo-C₁₋₆-alkyl.

In another preferred embodiment in combination with any of the above or below embodiments, R⁹ is selected from the group consisting of COOH, OH and SO₃H.

In a preferred embodiment in combination with any of the above or below embodiments, A is selected from the group consisting of phenyl, pyridyl, pyrimidyl, pyrazolyl, indolyl, thienyl, benzothienyl, indazolyl, benzisoxazolyl, benzofuranyl, benzotriazolyl, furanyl, benzothiazolyl, thiazolyl, oxadiazolyl, oxazolyl, naphthyl, quinolyl, isoquinolyl, benzimidazolyl, each unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₆-akyl, C₃₋₆-cycloalkyl and halo-C₃₋₆-cycloalkyl.

More preferably, A is selected from the group consisting of phenyl, pyridyl, indolyl, indazolyl, benzisothiazolyl, triazolopyridinyl, benzothiazolyl, thiazolyl, oxazolyl, quinolyl, each unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₆-alkyl, C₃₋₆-cycloalkyl and halo-C₃₋₆-cycloalkyl.

In a further preferred embodiment in combination with any of the above or below embodiments, R-A is selected from

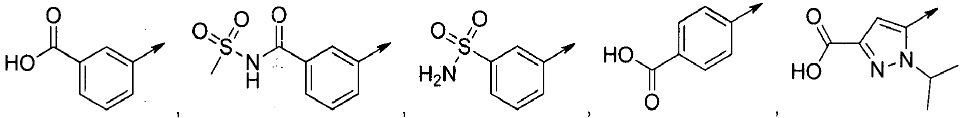

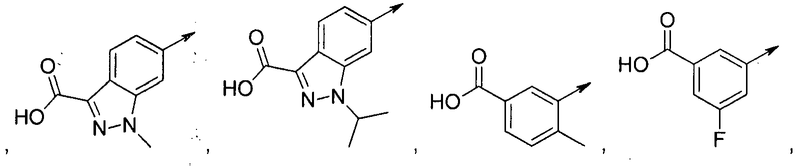

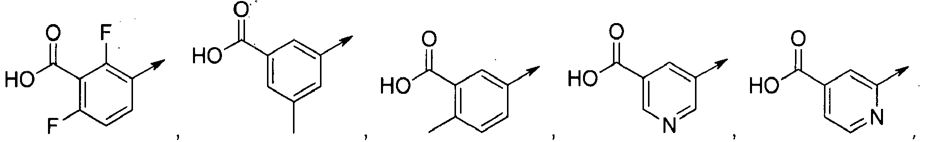

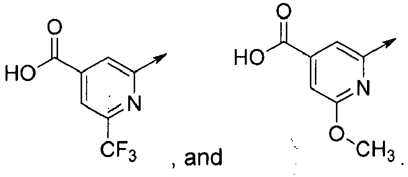

In a further preferred embodiment in combination with any of the above or below embodiments, Z is selected from 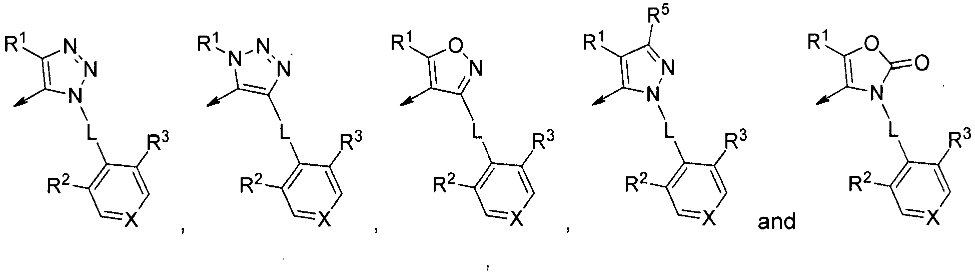 wherein
L is selected from the group consisting of a bond, C₁₋₃-alkylene and C₁₋₃-alkylene-O-;
X is selected from the group consisting of CH, CF, N and NO;
R¹ is selected from the group consisting of C₁₋₄-alkyl and C₃₋₆-cycloalkyl, wherein C₁₋₄-alkyl is unsubstituted or substituted with 1 to 3 substituents independently selected from the group consisting of fluoro, hydroxy, C₁₋₃-alkoxy and fluoro-C₁₋₃-alkoxy, and C₃₋₆-cycloalkyl is unsubstituted or substituted with 1 to 3 substituents independently selected from the group consisting of fluoro, hydroxy, C₁₋₃-alkyl, fluoro-C₁₋₃-alkyl, C₁₋₃-alkoxy and fluoro-C₁₋₃-alkoxy;
R² and R³ are independently selected from the group consisting of hydrogen, halogen, C₁₋₃-alkyl, halo-C₁₋₃-alkyl, C₁₋₃-alkoxy, halo-C₁₋₃-alkoxy, cyclopropyl and fluoro-cyclopropyl; and
R⁵ is selected from the group consisting of hydrogen, fluoro, CH₃, CHF₂ and CF₃,

In a more preferred embodiment in combination with any of the above or below embodiments, Z is selected from 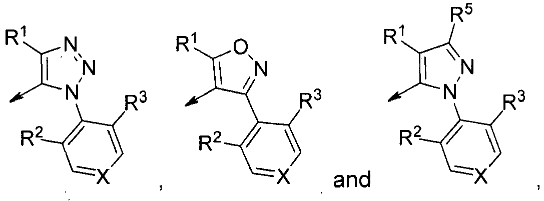 wherein
X is selected from the group consisting of CH, CF, N and NO;
R¹ is selected from the group consisting of CF₃, CHF₂, isopropyl and cyclopropyl, wherein isopropyl and cyclopropyl are unsubstituted or substituted with one or two fluoro or one hydroxy;
R² is selected from the group consisting of fluoro, chloro, CH₃, CHF₂, CF₃, OCHF₂ and OCF₃;
R³ is selected from the group consisting of hydrogen, fluoro, chloro, CH₃, CHF₂, CF₃, OCHF₂ and OCF₃; and
R⁵ is selected from the group consisting of hydrogen, fluoro, CH₃, CHF₂ and CF₃.

In a further preferred embodiment in combination with any of the above or below embodiments, the moiety 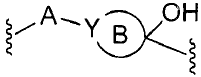 is selected from 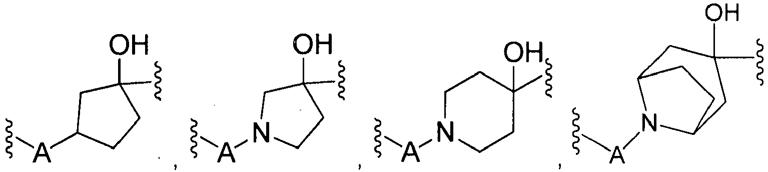 and

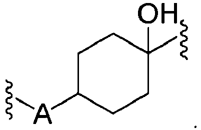

In a more preferred embodiment, there is provided a compound according to Formula (**2**) 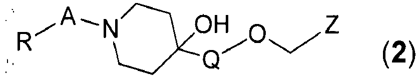 wherein
A is selected from the group consisting of phenyl, pyridyl, pyrimidyl, pyrazolyl, indolyl, thienyl, benzothienyl, indazolyl, benzisoxazolyl, benzofuranyl, benzotriazolyl, furanyl, benzothiazolyl, thiazolyl, oxadiazolyl, oxazolyl, naphthyl, quinolyl, isoquinolyl, benzimidazolyl, each unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₆-alkyl, C₃₋₆-cycloalkyl and halo-C₃₋₆-cycloalkyl;
R is selected from the group consisting of CO₂H, SO₃H, CONR⁷R⁸, tetrazolyl, 1,2,4-oxadiazol-5(4H)-one-3-yl and SO₂NHCOR⁷, wherein
   R⁷ selected from the group consisting of hydrogen, C₁₋₆-alkyl, halo-C₁₋₆-alkyl, C₁₋₆-alkylene-R⁹, SO₂-C₁₋₆-alkyl;
   R⁸ selected from the group consisting of hydrogen, C₁₋₆-alkyl, halo-C₁₋₆-alkyl; and
   R⁹ is selected from the group consisting of COOH, OH and SO₃H;
Q is selected from the group consisting of phenyl, pyridyl, thiazolyl, thiophenyl and pyrimidyl, each unsubstituted or substituted with one or two groups independently selected from the group consisting of fluoro, chloro, CH₃, CHF₂ and CF₃;
Z is selected from 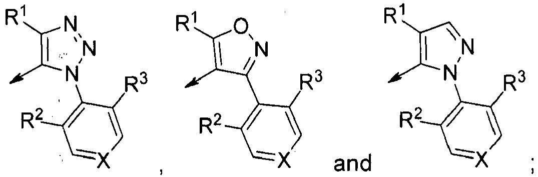
X is selected from the group consisting of CH, N and NO;
R¹ is selected from the group consisting of isopropyl and cyclopropyl, wherein isopropyl and cyclopropyl are unsubstituted or substituted with one or two fluoro or one hydroxy;
R² is selected from the group consisting of fluoro, chloro, CH₃, CHF₂, CF₃, OCHF₂ and OCF₃; and
R³ is selected from the group consisting of hydrogen, fluoro, chloro, CH₃, CHF₂, CF₃, OCHF₂ and OCF₃.

In another preferred embodiment, there is provided a compound of Formula **(3)** 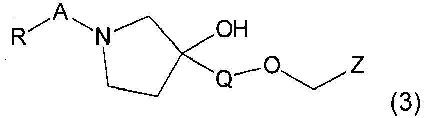 wherein
A is selected from the group consisting of phenyl, pyridyl, pyrimidyl, pyrazolyl, indolyl, thienyl, benzothienyl, indazolyl, benzisoxazolyl, benzofuranyl, benzotriazolyl, furanyl, benzothiazolyl, thiazolyl, oxadiazolyl, oxazolyl, naphthyl, quinolyl, isoquinolyl, benzimidazolyl, each unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₆-alkyl, C₃₋₆-cycloalkyl and halo-C₃₋₆-cycloalkyl;
R is selected from the group consisting of CO₂H, SO₃H, CONR⁷R⁸, tetrazolyl, 1,2,4-oxadiazol-5(4H)-one-3-yl and SO₂NHCOR⁷, wherein
   R⁷ selected from the group consisting of hydrogen, C₁₋₆-alkyl, halo-C₁₋₆-alkyl, C₁₋₆-alkylene-R⁹, SO₂-C₁₋₆-alkyl;
   R⁸ selected from the group consisting of hydrogen, C₁₋₆-alkyl, halo-C₁₋₆-alky!; and
   R⁹ is selected from the group consisting of COOH, OH and SO₃H;
Q is selected from the group consisting of phenyl, pyridyl, thiazolyl, thiophenyl and pyrimidyl, each unsubstituted or substituted with one or two groups independently selected from the group consisting of fluoro, chloro, CH₃, CHF₂ and CF₃;
Z is selected from 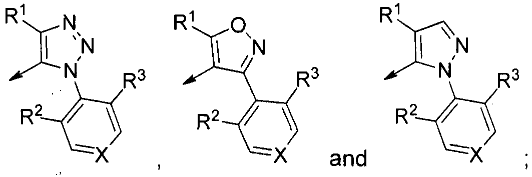
X is selected from the group consisting of CH, N and NO;
R¹ is selected from the group consisting of isopropyl and cyclopropyl, wherein isopropyl and cyclopropyl are unsubstituted or substituted with one or two fluoro or one hydroxy;
R² is selected from the group consisting of fluoro, chloro, CH₃, CHF₂, CF₃, OCHF₂ and OCF₃; and
R³ is selected from the group consisting of hydrogen, fluoro, chloro, CH₃, CHF₂, CF₃, OCHF₂ and OCF₃.

In the context of the present invention "C₁₋₆-alkyl" means a saturated alkyl chain having 1 to 6 carbon atoms which may be straight chained or branched. Examples thereof include methyl, ethyl, propyl, isopropyl, *n*-butyl, isobutyl, *tert*-butyl, n-pentyl, isopentyl, neopentyl and hexyl.

The term "halo-C₁₋₆-alkyl" means that one or more hydrogen atoms in the alkyl chain are replaced by a halogen. A preferred example thereof is CF₃.

"C₂₋₆-alkenyl" means an alkyl chain having 1 to 6 carbon atoms which may be straight chained or branched, containing at least one carbon to carbon double bond. Examples thereof include ethenyl, propenyl, butenyl, pentenyl, hexenyl or (1E,3Z)-2-methylpenta-1,3-dien-1-yl. Preferred examples are ethenyl, propenyl or (1E,3Z)-2-methylpenta-1,3-dien-1-yl.

"C₂₋₆-alkynyl" means an alkyl chain having 1 to 6 carbon atoms which may be straight chained or branched, containing at least one carbon to carbon triple bond. Examples thereof include ethynyl, propynyl, 1-butynyl, 2-butynyl, 1-pentynyl, 2-pentynyl, 1-hexynyl, 2-hexynyl or 3-hexynyl. Preferred examples thereof include ethynyl and propynyl.

A "C₀₋₆-alkylene" means that the respective group is divalent and connects the attached residue with the remaining part of the molecule. Moreover, in the context of the present invention, "C₀-alkylene" is meant to represent a bond.

A C₅₋₁₀-cycloalkyl group means a saturated or partially unsaturated mono-, bi- or spirocyclic ring system comprising 5 to 10 carbon atoms. Bridged carbocyclic ring systems comprise two or more ring systems which share non-adjacent bridgehead ring atoms. Examples include cyclopentyl, cyclohexyl, cyclohexenyl, bicyclo[2.2.2]octyl, bicyclo[3.2.1]octanyl, spiro[3.3]heptyl, bicyclo[2.2.1]heptyl, adamantyl and pentacyclo[4.2.0.0^{2,5}.0^{3,8}.0^{4,7}]octyl.

A C₃₋₁₀-heterocycloalkyl group means a saturated or partially unsaturated 3 to 10 membered carbon mono-, bi- or spirocyclic ring wherein 1, 2 or 3 carbon atoms are replaced by 1, 2 or 3 heteroatoms, respectively, wherein the heteroatoms are independently selected from N, O, S, SO and SO₂. Examples thereof include epoxidyl, oxetanyl, pyrrolidinyl, tetrahydrofuranyl, piperidinyl, piperazinyl tetrahydropyranyl, 1,4-dioxanyl, morpholinyl, 4-quinuclidinyl, 1,4-dihydropyridinyl and 3,6-dihydro-2*H*-thiopyranyl. The C₃₋₁₀-heterocycloalkyl group can be connected with the remaining part of the molecule via a carbon or nitrogen atom.

A 5-10-membered mono- or bicyclic heteroaromatic ring system (within the application also referred to as heteroaryl) containing up to 4 heteroatoms means a monocyclic heteroaromatic ring such as pyrrolyl, imidazolyl, furanyl, thiophenyl, pyridinyl, pyrimidinyl, pyrazinyl, pyrazolyl, oxazolyl, isoxazolyl, triazolyl, oxadiazolyl and thiadiazolyl. It further means a bicyclic ring system wherein the heteroatom(s) may be present in one or both rings including the bridgehead atoms. Examples thereof include quinolinyl, isoquinolinyl, quinoxalinyl, benzimidazolyl, benzisoxazolyl, benzofuranyl, benzoxazolyl, indolyl, indolizinyl and pyrazolo[1,5-a]pyrimidinyl. The nitrogen or sulphur atom of the heteroaryl system may also be optionally oxidized to the corresponding *N-*oxide, *S*-oxide or *S,S*-dioxide. If not stated otherwise, the heteroaryl system can be connected via a carbon or nitrogen atom. Examples for *N*-linked heterocycles are

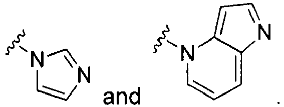

A 6-10-membered mono- or bicyclic aromatic ring system (within the application also referred to as aryl) means an aromatic carbon cycle such as phenyl or naphthalenyl.

The term "*N*-oxide" denotes compounds, where the nitrogen in the heteroaromatic system (preferably pyridinyl) is oxidized. Such compounds can be obtained in a known manner by reacting a compound of the present invention (such as in a pyridinyl group) with H₂O₂ or a peracid in an inert solvent.

Halogen is selected from fluorine, chlorine, bromine and iodine, more preferably fluorine or chlorine and most preferably fluorine.

Furthermore, the compounds of the present invention are partly subject to tautomerism. For example, if a heteroaromatic group containing a nitrogen atom in the ring is substituted with a hydroxy group on the carbon atom adjacent to the nitrogen atom, the following tautomerism can appear:

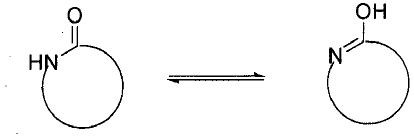

A C₃₋₆-cycloalkyl or C₃₋₆-heterocycloalkyl group can be connected straight or spirocyclic, e.g. when cyclohexane is substituted with the heterocycloalkyl group oxetane, the following structures are possible:

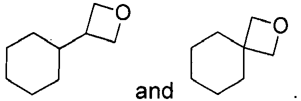

It will be appreciated by the skilled person that when lists of alternative substituents include members which, because of their valency requirements or other reasons, cannot be used to substitute a particular group, the list is intended to be read with the knowledge of the skilled person to include only those members of the list which are suitable for substituting the particular group.

The compounds of the present invention can be in the form of a prodrug compound. "Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods. Other examples of the prodrug are compounds, wherein the carboxylate in a compound of the present invention is, for example, converted into an alkyl-, aryl-, choline-, amino, acyloxymethylester, linolenoylester.

In the human liver, UDP-glucuronosyltransferases act on certain compounds having amino, carbamyl, thio (sulfhydryl) or hydroxyl groups to conjugate uridine diphosphate-α-D-glucuronic acid through glycoside bonds, or to esterify compounds with carboxy or hydroxyl groups in the process of phase II metabolism. Compounds of the present invention may be glucuronidated, that is to say, conjugated to glucuronic acid, to form glucuronides, particularly (β-D)glucuronides.

Metabolites of compounds of the present invention are also within the scope of the present disclosure.

One step in the formation of bile is the conjugation of the individual bile acids with an amino acid, particularly glycine or taurine. Compounds of the present invention may be conjugated with glycine or taurine at a substitutable position.

Where tautomerism, like e.g. keto-enol tautomerism, of compounds of the present invention or their prodrugs may occur, the individual forms, like e.g. the keto and enol form, are each within the scope of the invention as well as their mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases.

Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of the present invention may be obtained from stereoselective synthesis using optically pure starting materials. Another way to obtain pure enantiomers from racemic mixtures would use enantioselective crystallization with chiral counterions.

The compounds of the present invention can be in the form of a pharmaceutically acceptable salt or a solvate. The term "pharmaceutically acceptable salts" refers to salts prepared from pharmaceutically acceptable non-toxic bases or acids, including inorganic bases or acids and organic bases or acids. In case the compounds of the present invention contain one or more acidic or basic groups, the invention also comprises their corresponding pharmaceutically or toxicologically acceptable salts, in particular their pharmaceutically utilizable salts. Thus, the compounds of the present invention which contain acidic groups can be present on these groups and can be used according to the invention, for example, as alkali metal salts, alkaline earth metal salts or ammonium salts. More precise examples of such salts include sodium salts, potassium salts, calcium salts, magnesium salts or salts with ammonia or organic amines such as, for example, ethylamine, ethanolamine, triethanolamine or amino acids. The compounds of the present invention which contain one or more basic groups, i.e. groups which can be protonated, can be present and can be used according to the invention in the form of their addition salts with inorganic or organic acids. Examples of suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present invention simultaneously contain acidic and basic groups in the molecule, the invention also includes, in addition to the salt forms mentioned, inner salts or betaines (zwitterions). The respective salts can be obtained by customary methods which are known to the person skilled in the art like, for example, by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

Further the compounds of the present invention may be present in the form of solvates, such as those which include as solvate water, or pharmaceutically acceptable solvates, such as alcohols, in particular ethanol.

Furthermore, the present invention provides pharmaceutical compositions comprising at least one compound of the present invention, or a prodrug compound thereof, or a pharmaceutically acceptable salt or solvate thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing at least one compound of the present invention and a pharmaceutically acceptable carrier.

The pharmaceutical composition of the present invention may additionally comprise one or more other compounds as active ingredients like a prodrug compound or other nuclear receptor modulators.

The compositions are suitable for oral, rectal, topical, parenteral (including subcutaneous, intramuscular, and intravenous), ocular (ophthalmic), pulmonary (nasal or buccal inhalation) or nasal administration, although the most suitable route in any given case will depend on the nature and severity of the conditions being treated and on the nature of the active ingredient. They may be conveniently presented in unit dosage form and prepared by any of the methods well-known in the art of pharmacy.

The invention further relates to the use of said compounds for the treatment and/or prophylaxis of diseases and/or conditions through binding of said nuclear receptor by said compounds. Further the present invention relates to the use of said compounds for the preparation of a medicament for the treatment and/or prophylaxis of diseases and/or conditions through binding of said nuclear receptor by said compounds. Specifically, the present invention relates to the use of compounds according to Formula (**1**) in the preparation of a medicament for the prophylaxis and/or treatment of chronic intrahepatic or some forms of extrahepatic cholestatic conditions, of liver fibrosis, of acute intraheptic cholestatic conditions, of obstructive or chronic inflammatory disorders that arise out of improper bile composition, of gastrointestinal conditions with a reduced uptake of dietary fat and fat-soluble dietary vitamins, of inflammatory bowel diseases, of lipid and lipoprotein disorders, of Type II Diabetes and clinical complications of Type I and Type II Diabetes, of conditions and diseases which result from chronic fatty and fibrotic degeneration of organs due to enforced lipid and specifically triglyceride accumulation and subsequent activation of profibrotic pathways, of obesity and metabolic syndrome (combined conditions of dyslipidemia, diabetes and abnormally high body-mass index), of acute myocardial infarction, of acute stroke, of thrombosis which occurs as an endpoint of chronic obstructive atherosclerosis, of persistant infections by intracellular bacteria or parasitic protozoae, of non-malignant hyperproliferative disorders, of malignant hyperproliferative disorders, of colon adenocarcinoma and hepatocellular carcinoma in particular, of liver steatosis and associated syndromes, of liver failure or liver malfunction as an outcome of chronic liver diseases or of surgical liver resection, of Hepatitis B infection, of Hepatitis C infection and/or of cholestatic and fibrotic effects that are associated with alcohol-induced cirrhosis or with viral-borne forms of hepatitis.

Medicaments as referred to herein may be prepared by conventional processes, including the combination of a compound according to the present invention and a pharmaceutically acceptable carrier.

FXR is proposed to be a nuclear bile acid sensor. As a result, it modulates both, the synthetic output of bile acids in the liver and their recycling in the intestine (by regulating bile acid binding proteins). But beyond bile acid physiology, FXR seems to be involved in the regulation of many diverse physiological processes which are relevant in the etiology and for the treatment of diseases as diverse as cholesterol gallstones, metabolic disorders such as Type II Diabetes, dyslipidemias or obesity, chronic inflammatory diseases such as Inflammatory Bowel Diseases or chronic intrahepatic forms of cholestasis and many others diseases (T. Claudel et al., Arterioscler. Thromb. Vasc. Biol. 2005, 25, 2020; Y. D. Wang et al., Cell Res. 2008, 18, 1087.

FXR regulates a complex pattern of response genes in the liver and in the gastrointestinal tract. The gene products have impact on diverse physiological processes. In the course of functional analysis of FXR, the first regulatory network that was analyzed was the regulation of bile acid synthesis. While the LXRs induce the key enzyme of the conversion of cholesterol into bile acids, Cyp7A1, via the induction of the regulatory nuclear receptor LRH-1, FXR represses the induction of Cyp7A1 via the upregulation of mRNA encoding SHP, a further nuclear receptor that is dominant repressive over LRH-1. Since FXR binds the end products of this pathway, primary bile acids such as cholic acid (CA) or CDCA, this can be regarded as an example of feedback inhibition on the gene expression level (B. Goodwin et al., Mol. Cell 2000, 6, 517; T. T. Lu et al., Mol. Cell 2000, 6, 507). Parallel to the repression of bile acid synthesis via SHP, FXR induces a range of so-called ABC (for ATP-binding cassette) transporters that are responsible for the export of toxic bile acids from the hepatocyte cytosol into the canaliculi, the small bile duct ramifications where the bile originates. This hepatoprotective function of FXR became first apparent with the analysis of FXR knockout mice (C. J. Sinal et al., Cell 2000, 102, 731). where under- or overexpression of several ABC-transporters in the liver was shown. Further detailed analysis revealed that the major bile salt excretory pump BSEP or ABCB11 (M. Ananthanarayanan et al., J. Biol. Chem. 2001, 276, 28857; J. R. Plass et al., Hepatology 2002, 35, 589) as well as the key enzyme which mediates lipid transfer from lipoproteins to phospholipids, PLTP (N. L. Urizar et al., J. Biol. Chem. 2000, 275, 39313), and the two key canalicular membrane transporters for phospholipids, MRP-2 (ABCC4) (H. R. Kast et al., J. Biol. Chem. 2002, 277, 2908) and MDR-3 (ABCB4); L. Huang et al., J. Biol. Chem. 2003, 278, 51085) are direct targets for ligand-directed transcriptional activation by FXR (summarized in: M. Miyata, J. Pharmacol. Exp. Ther. 2005, 312, 759; G. Rizzo et al., Curr. Drug Targets Immune Endocr. Metabol. Disord. 2005, 5, 289).

The fact that FXR seems to be the major metabolite sensor and regulator for the synthesis, export and re-circulation of bile acids suggested the use of FXR ligands to induce bile flow and change bile acid composition towards more hydrophilic composition. With the development of the first synthetic FXR ligand GW4064 (P. R. Maloney et al., J. Med. Chem. 2000, 43, 2971; T. M. Willson et al., Med. Res. Rev. 2001, 21, 513) as a tool compound and of the semi-synthetic artificial bile acid ligand 6-alpha-ethyl-CDCA, the effects of superstimulation of FXR by potent agonists could be analyzed. It was shown that both ligands induce bile flow in bile duct ligated animals. Moreover, in addition to choleretic effects, also hepatoprotective effects could be demonstrated (R. Pellicciariet al., J. Med. Chem. 2002, 45, 3569; Y. Liu et al., J. Clin. Invest. 2003, 112, 1678). This hepatoprotective effect was further narrowed down to an anti-fibrotic effect that results from the repression of Tissue Inhibitors of Matrix-Metalloproteinases, TIMP-1 and 2, the induction of collagen-deposit resolving Matrix-Metalloproteinase 2 in hepatic stellate cells and the subsequent reduction of alpha-collagen mRNA and Transforming growth factor beta (TGF-beta) mRNA which are both pro-fibrotic factors by FXR agonists (S. Fiorucci et al., Gastroenterology 2004, 127, 1497; S. Fiorucci et al., J. Pharmacol. Exp. Ther. 2005, 314, 584). Furthermore, anti-cholestatic activity was demonstrated in bile-duct ligated animal models as well as in animal models of estrogen-induced cholestasis (S. Fiorucci et al., J. Pharmacol. Exp. Ther. 2005, 313, 604).

Genetic studies demonstrate that in hereditary forms of cholestasis (Progressive Familiar Intrahepatic Cholestasis = PFIC, Type I - IV) either nuclear localization of FXR itself is reduced as a consequence of a mutation in the FIC1 gene (in PFIC Type I, also called Byler's Disease) (F. Chen et al., Gastroenterology 2004, 126, 756; L. Alvarez et al., Hum. Mol. Genet. 2004, 13, 2451) or levels of the FXR target gene encoding MDR-3 phospholipid export pump are reduced (in PFIC Type III). Taken together there is a growing body of evidence that FXR binding compounds will demonstrate substantial clinical utility in the therapeutic regimen of chronic cholestatic conditions such as Primary Biliary Cirrhosis (PBC) or Primary Sclerosing Cholangitis (PSC) (reviewed in: G. Rizzo et al., Curr. Drug Targets Immune Endocr. Metabol. Disord. 2005, 5, 289; G. Zollner et al., Mol. Pharm. 2006, 3, 231; S. Y. Cai et al., Expert Opin. Ther. Targets 2006, 10, 409).

The deep impact that FXR activation has on bile acid metabolism and excretion is not only relevant for cholestatic syndromes but even more directly for a therapy against gallstone formation. Cholesterol gallstones form due to low solubility of cholesterol that is actively pumped out of the liver cell into the lumen of the canaliculi. It is the relative percentage of content of the three major components, bile acids, phospholipids and free cholesterol that determines the formation of mixed micelles and hence apparent solubility of free cholesterol in the bile. FXR polymorphisms map as quantitative trait loci as one factor contributing to gallstone disease (H. Wittenburg, Gastroenterology 2003, 125, 868). Using the synthetic FXR tool compound GW4064 it could be demonstrated that activation of FXR leads to an improvement of the Cholesterol Saturation Index (CSI) and directly to an abolishment of gallstone formation in C57L gallstone susceptible mice whereas drug treatment in FXR knockout mice shows no effect on gallstone formation (A. Moschetta et al., Nature Medicine 2004, 10, 1352).

These results qualify FXR as a good target for the development of small molecule agonists that can be used to prevent cholesterol gallstone formation or to prevent re-formation of gallstones after surgical removal or shockwave lithotripsy (discussed in: S. A. Doggrell, Curr. Opin. Investig. Drugs 2006, 7, 344).

Thus, in one embodiment of the invention, the compound according to Formula (**1**) and pharmaceutical compositions comprising said compound is used for the prophylaxis and/or treatment of obstructive or chronic inflammatory disorders that arise out of improper bile composition such as cholelithiasis also known as cholesterol gallstones.

Beyond its strong hepatoprotective and choleretic as well as anti-fibrotic effects that FXR shows upon small molecule stimulated activation in the liver, FXR seems to have a role in protecting the intestine from neoplastic transformation and from the development of polyps and their transition into adenocarcinoma in the gut (S. Modica et al., Cancer Res. 2008, 68, 9589 and R. R. Maran et al., J. Pharmacol. Exp. Ther. 2009, 328, 469). Similar to the situation in the intestine absence of FXR leads to a high increase in the formation of Hepatocellular Cacrcinoma (HCC), the most prominent form of liver cancer (I. Kim et al., Carcinogenesis 2007, 28, 940 and F. Yang et al., Cancer Res. 2007, 67, 863). Whereas a functional FXR prevents the formation of colon adenocarcinoma and hepatocellular carcinoma, FXR activation induces liver regeneration after hepatectomy (W. Huang et al., Science 2006, 312, 233).

The combined hepatoprotective, anti-neoplastic and liver regenerative effects associated with FXR activation can be therapeutically exploited for the use of FXR agonists in the treatment of sever liver diseases. In one embodiment, the compounds according to the invention and pharmaceutical compositions comprising said compounds are used in the treatment of liver diseases such as HCC, stimulation of liver regrowth and amelioration of side effects associated with major liver resection, liver cirrhosis independent of the etiology and prevention or treatment of liver ischemia in the course of liver transplantation or major liver surgery.

Since the discovery of the first synthetic FXR agonist and its administration to rodents it became evident that FXR is a key regulator of serum triglycerides (P. Maloney et al., J. Med. Chem. 2000, 43, 2971; T. Willson et al., Med. Res. Rev. 2001, 21, 513). Over the past six years accumulating evidence has been published that activation of FXR by synthetic agonists leads to significant reduction of serum triglycerides, mainly in the form of reduced VLDL, but also to reduced total serum cholesterol (H. R. Kast et al., Mol. Endocrinol. 2001, 15, 1720; N. L. Urizar et al., Science 2002, 296, 1703; G. Lambert et al., J. Biol. Chem. 2003, 278, 2563; M. Watanabe et al., J. Clin. Invest. 2004, 113, 1408; A. Figge et al., J. Biol. Chem. 2004, 279, 2790; S. Bilz et al., Am. J. Physiol. Endocrinol. Metab. 2006, 290, E716).

But the lowering of serum triglycerides is not a stand alone effect. Treatment of db/db or ob/ob mice with synthetic FXR agonist GW4064 resulted in marked and combined reduction of serum triglycerides, total cholesterol, free fatty acids, ketone bodies such as 3-OH Butyrate. Moreover, FXR activation engages with the intracellular insulin signaling pathway in hepatocytes, resulting in reduced output of glucose from liver gluconeogenesis but concomitant increase in liver glycogen. Insulin sensitivity as well as glucose tolerance were positively impacted by FXR treatment (K. R. Stayrook et al., Endocrinology 2005, 146, 984; Y. Zhang et al., PNAS 2006, 103, 1006; B. Cariou et al., J. Biol. Chem. 2006, 281, 11039; K. Ma et al., J. Clin. Invest. 2006, 116, 1102; D. Duran-Sandoval et al., Biochimie 2005, 87, 93). An effect on reduction of body weight was also recently observed in mice overfed with a high lipid diet (C. Lihong et al., American Diabetes Association (ADA) 66th annual scientific sessions, June 2006, Abstract Number 856-P). This weight loss effect might results from FXR's induction of FGF-19, a fibroblast growth factor that is known to lead to weight loss and athletic phenotype (J. Holt et al., Genes Dev. 2003, 17, 1581; E. Tomlinson et al., Endocrinology 2002, 143, 1741). In recent patent applications, the effect of FXR agonist on reduction of body weight was demonstrated (WO 2004/087076; WO 2003/080803).

Taken together, these pharmacological effects of FXR agonists can be exploited in different therapeutic ways: FXR binding compounds are thought to be good candidates for the treatment of Type II Diabetes because of their insulin sensitization, glycogenogenic, and lipid lowering effects.

In one embodiment, the compounds according to the invention and pharmaceutical compositions comprising said compounds are used in the prophylaxis and/or treatment of Type II Diabetes which can be overcome by FXR-mediated upregulation of systemic insulin sensitivity and intracellular insulin signalling in liver, increased peripheral glucose uptake and metabolisation, increased glycogen storage in liver, decreased output of glucose into serum from liver-borne gluconeogenesis.

In a further embodiment, said compounds and pharmaceutical compositions are used for the prophylaxis and/or treatment of chronic intrahepatic, such as PBC, PSC, progressive familiar cholestasis (PFIC), alcohol-induced cirrhosis and associated cholestasis, and some forms of extrahepatic cholestatic conditions, or liver fibrosis.

The invention also relates to a compound of Formula (**1**) or to a pharmaceutical composition comprising said compound for the prophylaxis and/or treatment of gastrointestinal conditions with a reduced uptake of dietary fat and fat-soluble dietary vitamins which can be overcome by increased intestinal levels of bile acids and phospholipids.

In a further embodiment, said compound or pharmaceutical composition is used for preventing and/or treating a disease selected from the group consisting of lipid and lipoprotein disorders such as hypercholesterolemia, hypertriglyceridemia, and atherosclerosis as a clinically manifest condition which can be ameliorated by FXR's beneficial effect on lowering total plasma cholesterol, lowering serum triglycerides, increasing conversion of liver cholesterol into bile acids and increased clearance and metabolic conversion of VLDL and other lipoproteins in the liver.

In one further embodiment, said compound and pharmaceutical composition are used for the prophylaxis and/or treatment of diseases where the combined lipid lowering, anti-cholestatic and anti-fibrotic effects of FXR-targeted medicaments can be exploited for the treatment of liver steatosis and associated syndromes such as NASH, or for the treatment of cholestatic and fibrotic effects that are associated with alcohol-induced cirrhosis, or with viral-borne forms of hepatitis.

In conjunction with the hypolipidemic effects it was also shown that loss of functional FXR leads to increased atherosclerosis in ApoE knockout mice (E. A. Hanniman et al., J. Lipid Res. 2005, 46, 2595). Therefore, FXR agonists might have clinical utility as anti-atherosclerotic and cardioprotective drugs. The downregulation of Endothelin-1 in Vascular Smooth Muscle Cells might also contribute to such beneficial therapeutic effects (F. He et al., Circ. Res. 2006, 98, 192).

The invention also relates to a compound according to Formula (**1**) or a pharmaceutical composition comprising said compound for preventive and posttraumatic treatment of cardiovascular disorders such as acute myocardial infarction, acute stroke, or thrombosis which occur as an endpoint of chronic obstructive atherosclerosis.

Beyond controlling intestinal and colonic polyp formation, FXR seems to be expressed in breast cancer tissue and cell lines but not in healthy breast tissue and seems to interact with the Estrogen Receptor in ER positive breast cancer cells (K. E. Swales et al., Cancer Res. 2006, 66, 10120 and F. Journe et al., Breast Cancer Res. Treat. 2009, 115, 523).

This would allow to regard FXR also as a potential target for the treatment of proliferative diseases, especially metastasizing cancer forms that express a small molecule responsive form of FXR.

In a further embodiment, said compounds and pharmaceutical compositions are used for the prophylaxis and/or treatment of malignant hyperproliferative disorders such as different forms of cancer, specifically certain forms of breast, liver or colon cancer where interference with an FXR ligand will have a beneficial impact.

Finally, FXR seems also to be involved in the control of antibacterial defense in the intestine (T. Inagaki et al., PNAS. 2006, 103, 3920) although an exact mechanism is not provided. From these published data, however, one can conclude that treatment with FXR agonists might have a beneficial impact in the therapy of Inflammatory Bowel Disorders (IBD), in particular those forms where the upper (ileal) part of the intestine is affected (e.g. ileal Crohn's disease) because this seems to be the site of action of FXR's control on bacterial growth. In IBD the desensitization of the adaptive immune response is somehow impaired in the intestinal immune system. Bacterial overgrowth might then be the causative trigger towards establishment of a chronic inflammatory response. Hence, dampening of bacterial growth by FXR-borne mechanisms might be a key mechanism to prevent acute inflammatory episodes.

Thus, the invention also relates to a compound according to Formula (**1**) or a pharmaceutical composition comprising said compound for preventing and/or treating a disease related to Inflammatory Bowel Diseases such as Crohn's disease or Colitis ulcerosa. FXR-mediated restoration of intestinal barrier function and reduction in non-commensal bacterial load is believed to be helpful in reducing the exposure of bacterial antigens to the intestinal immune system and can therefore reduce inflammatory responses.

The invention further relates to a compound or pharmaceutical composition for the prophylaxis and/or treatment of obesity and associated disorders such as metabolic syndrome (combined conditions of dyslipidemias; diabetes and abnormally high body-mass index) which can be overcome by FXR-mediated lowering of serum triglycerides, blood glucose and increased insulin sensitivity and FXR-mediated weight loss.

In a further embodiment, the compounds or pharmaceutical composition of the present invention are useful in preventing and/or treating clinical complications of Type I and Type II Diabetes. Examples of such complications include Diabetic Nephropathy, Diabetic Retinopathy, Diabetic Neuropathies, or Peripheral Arterial Occlusive Disease (PAOD). Other clinical complications of Diabetes are also encompassed by the present invention.

Furthermore, conditions and diseases which result from chronic fatty and fibrotic degeneration of organs due to enforced lipid and specifically triglyceride accumulation and subsequent activation of profibrotic pathways may also be prevented and/or treated by applying the compounds or pharmaceutical composition of the present invention. Such conditions and diseases encompass NASH and chronic cholestatic conditions in the liver, Glomerulosclerosis and Diabetic Nephropathy in the kidney, Macula Degeneration and Diabetic Retinopathy in the eye and Neurodegenerative diseases such as Alzheimer's Disease in the brain, or Diabetic Neuropathies in the peripheral nervous system.

In practical use, the compounds of the present invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or non-aqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Since the compounds of the present invention mostly represent carboxylic acids or similar anionic isosters thereof, and since it is well known that salt forms of ionic drug compounds can substantially affect the bioavailability of drug compounds, the compounds of the present invention may also be used as salts with various countercations to yield an orally available formulation. Such pharmaceutically acceptable cations may be amongst others mono- or bivalent ions such as ammonium, the alkaline metals sodium or potassium or the alkaline earth metals magnesium or calcium, certain pharmaceutically acceptable amines such as tris(hydroxymethyl)aminomethane, ethylendiamine, diethylamine, piperazine or others, or certain cationic amino acids such as lysine or arginine.

The compounds of the present invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms. suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the present invention are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing FXR mediated conditions for which compounds of the present invention are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.

The compounds of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described above.

The amine-free bases corresponding to the isolated salts can be generated by neutralization with a suitable base, such as aqueous sodium hydrogen carbonate, sodium carbonate, sodium hydroxide and potassium hydroxide, and extraction of the liberated amine-free base into an organic solvent, followed by evaporation. The amine-free base, isolated in this manner, can be further converted into another pharmaceutically acceptable salt by dissolution in an organic solvent, followed by addition of the appropriate acid and subsequent evaporation, precipitation or crystallization. The carboxylic free acids corresponding to the isolated salts can be generated by neutralization with a suitable acid, such as aqueous hydrochloric acid, sodium hydrogen sulfate, sodium dihydrogen phosphate, and extraction of the liberated carboxylic-free acid into an organic solvent, followed by evaporation. The carboxylic acid, isolated in this manner, can be further converted into another pharmaceutically acceptable salt by dissolution in an organic solvent, followed by addition of the appropriate base and subsequent evaporation, precipitation or crystallization.

An illustration of the preparation of compounds of the present invention is shown below. Unless otherwise indicated in the schemes, the variables have the same meaning as described above. The examples presented below are intended to illustrate particular embodiments of the invention. Suitable starting materials, building blocks and reagents employed in the synthesis as described below are commercially available from Sigma-Aldrich or Acros Organics, for example, or can be routinely prepared by procedures described in the literature, for example in "March's Advanced Organic Chemistry: Reactions, Mechanisms, and Structure", 5th Edition; John Wiley & Sons or T. Eicher, S. Hauptmann "The Chemistry of Heterocycles; Structures, Reactions, Synthesis and Application", 2nd edition, Wiley-VCH 2003; Fieser et al. "Fiesers' Reagents for organic Synthesis" John Wiley & Sons 2000.

### List of Abbreviations

- DMF: dimethylformamide
- NCS: *N*-chlorosuccinimide
- DCM: dichloromethane
- THF: tetrahydrofurane
- PE: petroleum ether
- DMSO: dimethylsulfoxide
- IBX: *o*-iodoxybenzoic acid
- DBU: 1,8-diazabicyclo[5.4.0]undec-7-ene
- p-TsOH: *p*-toluenesulfonic acid
- TEA: triethylamine
- MsCl: mesyl chloride
- TFA: trifruoroacetic acid
- DIAD: diisopropyl azodicarboxylate
- DAST: (dimethylamino)sulfur trifluoride
- TLC: thinlayer chromatography
- MeCN: acetonitrile
- m-CPBA: *m*-chloroperbenzoic acid
- SEM-Cl: 2-(trimethylsilyl)ethoxymethyl chloride
- TFAA: trifluoroacetic anhydride
- ACN: acetonitrile
- TMS: trimethylsilyl
- TEMPO: 2,2,6,6-tetramethylpiperidinyloxyl, free radical
- PCC: pyridinium perchromate
- HMPA: hexamethylphosphonamide
- Dba: dibenzylidineacetone
- Xantphos: 4,5-bis(diphenylphosphino)-9,9-dimethylxanthene
- EDCI: 1-(3-dimethylaminopropyl)-3-ethylcarbodiimide hydrochloride
- DMAP: 4-dimethylaminopyridine

### General Synthesis 1

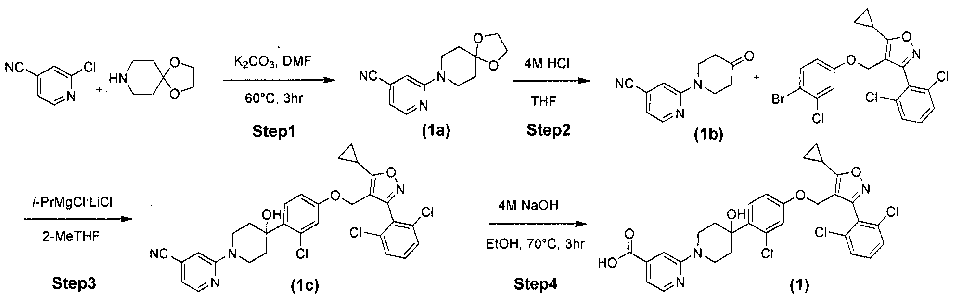

### Alternate Step1

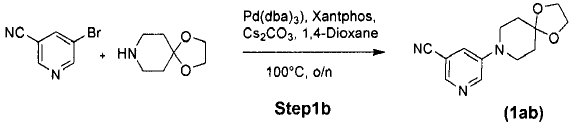

### Example 1c: 2-(4-(2-Chloro-4-((5-cyclopropyl-3-(2,6-dichlorophenyl)isoxazol-4-yl) methoxy)phenyl)-4-hydroxypiperidin-1-yl)isonicotinonitrile

### Step1: 2-(1,4-dioxa-8-azaspiro[4.5]decan-8-yl)isonicotinonitrile (1a)

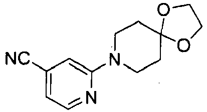

In a microwave 2-chloroisonicotinonitrile (300 mg, 2.17 mmol) was dissolved in DMF (3 ml), then treated with 1,4-dioxa-8-azaspiro[4.5]decane (0.42 ml, 3.25 mmol) and potassium carbonate (600 mg, 4.33 mmol). The vial was sealed and heated in a microwave reactor for 20 min at 110°C. After colling to room temperature water was added and the mixture was extracted twice with EtOAc, the organic layer was washed 4 times with brine, dried over Na₂SO₄, filtered and then concentrated. Purification by column chromatography (ISCO 25g 0-40% EtOAc/hexanes yielded the product (310 mg, 58% yield).

### Step2: 2-(4-oxopiperidin-1-yl)isonicotinonitrile (1b)

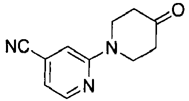

2-(1,4-dioxa-8-azaspiro[4.5]decan-8-yl)isonicotinonitrile (310 mg, 1.26 mmol) was dissolved in THF (4 ml), 4M HCI (4 ml) was added, and the reaction stirred at room temp overnight. Water was added then the pH was adjusted to 8 with 1N NaOH. EtOAc was added, the phases were separated. The aqueous phase was extracted once with EtOAc, the combined organics were washed with brine, dried over Na₂SO₄, filtered and concentrated (186 mg, 73% yield).

### Step 3, Example 1c: 2-(4-(2-chloro-4-((5-cyclopropyl-3-(2,6-dichlorophenyl)isoxazol-4-yl)methoxy)phenyl)-4-hydroxypiperidin-1-yl)isonicotinonitrile (1c)

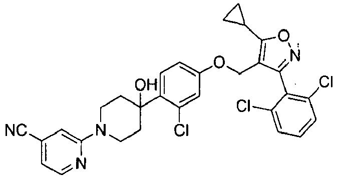

An oven-dried reaction vial containing 4-((4-bromo-3-chlorophenoxy)methyl)-5-cyclopropyl-3-(2,6-dichlorophenyl)isoxazole (200 mg, 0.42 mmol) was sealed, evacuated, and filled with N2 three times, then 2-MeTHF (3 ml) was added via syringe. 1.3M Isopropylmagnesium chloride lithium chloride complex in THF (0.39 ml) was then added dropwise via syringe at room temp and the mixture was stirred for 1hr at room temperature then heated to 50°C for 1hr. An additional 1.2eq i-PrMgCl was added and heating was continued at 50°C for 2hrs. The solution was cooled to room temperature then slowly added via syringe to a solution of 2-(4-oxopiperidin-1-yl)isonicotinonitrile (85 mg, 0.42 mmol) in THF (2 ml), and the mixture was allowed to stir overnight at room temperature. The mixture was quenched with H₂O and EtOAc then acidified with1N HCI. The phases were separated, the organics were washed with brine, dried over Na₂SO₄, and concentrated. Purification by chromatography (ISCO 25g GOLD silica 0-100% EtOAc/hexanes) gave the product. ¹H NMR (300 MHz, DMSO-d6) δ 8.27 (d, J = 5.0 Hz, 1H), 7.66 - 7.46 (m, 4H), 7.34 (s, 1H), 6.88 (dd, J = 5.0, 1.1 Hz, 1H), 6.80 - 6.71 (m, 2H), 5.27 (s, 1H), 4.86 (s, 2H), 4.24 (d, J = 12.5 Hz, 2H), 3.31 - 3.20 (m, 2H), 2.48 - 2.35 (m, 2H), 1.50 (d, J = 13.2 Hz, 2H), 1.26 - 1.03 (m, 5H).). MS (M+H): 595.05.

### Example 1: 2-(4-(2-chloro-4-((5-cyclopropyl-3-(2,6-dichlorophenyl)isoxazol-4-yl)methoxy)phenyl)-4-hydroxypiperidin-1-yl)isonicotinic acid (1)

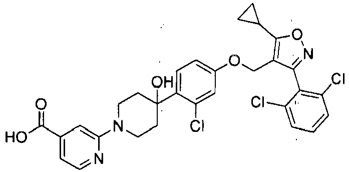

In a flask 2-(4-(2-chloro-4-((5-cyclopropyl-3-(2,6-dichlorophenyl)isoxazol-4-yl)methoxy)phenyl)-4-hydroxypiperidin-1-yl)isonicotinonitrile (40 mg, 0.07 mmol) was dissolved in ethanol (1.5 ml), treated with 4M Sodium hydroxide in water (0.67 mL) and stirred at room temperature for 1hr then heated to 80°C for 3hrs. The mixture was cooled and the EtOH was removed under vacuum. The remaining solution was cooled in ice bath, treated with water ∼2ml, and the pH was adjusted ∼4 with 1M HCl. The mixture was extracted with EtOAc, washed with brine, dried over Na₂SO₄, filtered and concentrated to give the product (29 mg, 70% yield). ¹H NMR (300 MHz, DMSO-d6) δ 13.39 (s, 1H), 8.23 (d, J = 5.1 Hz, 1H), 7.67 - 7.45 (m, 4H), 7.23 (d, J = 1.3 Hz, 1H), 6.97 (dd, J = 5.1, 1.1 Hz, 1H), 6.80 - 6.70 (m, 2H), 5.24 (s, 1H), 4.86 (s, 2H), 4.20 (d, J = 12.0 Hz, 2H), 3.31 - 3.20 (m, 2H), 2.47 - 2.35 (m, 2H), 1.51 (d, J = 13.1 Hz, 2H), 1.28 - 1.01 (m, 5H). MS (M+H): 614.05.

### General Synthesis 2

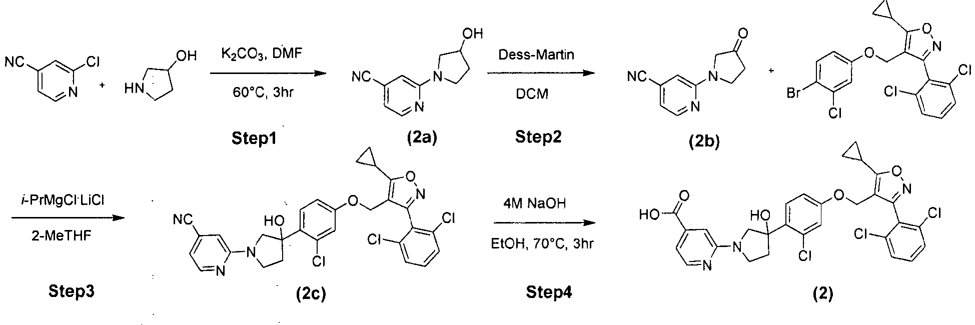

### Alternate Step1

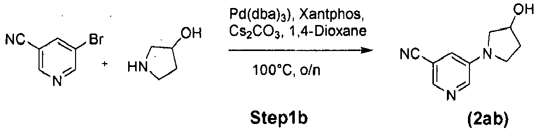

### Example 2: 2-(3-(2-Chloro-4-((5-cyclopropyl-3-(2,6-dichlorophenyl)isoxazol-4-yl)methoxy)phenyl)-3-hydroxypyrrolidin-1-yl)isonicotinic acid (2)

### Step1: 2-(3-hydroxypyrrolidin-1-yl)isonicotinonitrile (2a)

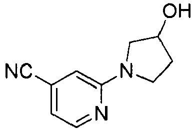

In a 100ml flask equipped with a magnetic stir bar and condenser was placed 2-chloroisonicotinonitrile (1500 mg, 10.8 mmol), DMF (15 ml), 3-pyrrolidinol (1.3 ml, 16.2 mmol) and potassium carbonate (2300 mg, 21.7 mmol). The reaction was heated to 60°C for 3 hours then partially concentrated to remove DMF, diluted with EtOAc and water, separated, extracted 1X with EtOAc, washed four times with brine, dried over sodium sulfate, filtered, then concentrated under reduced pressure (1770mg, 86% yield).

### Step2: 2-(3-oxopyrrolidin-1-yl)isonicotinonitrile (2b)

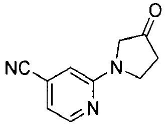

In a flask equipped with a magnetic stir bar a solution of 2-(3-hydroxypyrrolidin-1-yl) isonicotinonitrile (1770 mg, 9.35 mmol) in dichloromethane (90 ml) was treated with Dess-martin periodinane (4760 mg, 11.23 mmol) in 4 portions and the mixture was stirred overnight at room temperature. The reaction was quenched with a sodium thiosulfate solution, diluted with EtOAc and stirred for ∼15min then treated with water to give a clear solution. The layers were separated and the organics washed with 50% aqueous sodium bicarbonate solution then brine, dried over Na₂SO₄, filtered and concentrated. Purification by column chromatography (ISCO 40g silica, 0-100% EtOAc/hexanes) gave the product.

### Step3: 2-(3-(2-chloro-4-((5-cyclopropyl-3-(2,6-dichlorophenyl)isoxazol-4-yl)methoxy)phenyl)-3-hydroxypyrrolidin-1-yl)isonicotinonitrile (2c)

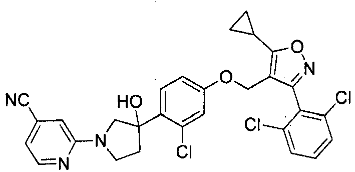

In a dry reaction vial equipped with a magnetic stir bar under nitrogen, 4-((4-bromo-3-chlorophenoxy)methyl)-5-cyclopropyl-3-(2,6-dichlorophenyl)isoxazole (430 mg, 0.74 mmol) was dissolved in 2-MeTHF (1.4 mL). To this was added dropwise 1.3M i-PrMgCI-LiCI complex in THF (1.5 mL, 1.9 mmol) and the mixture was stirred for 2.5hrs. The mixture was cooled in an ice/water bath and added 2-(3-oxopyrrolidin-1-yl)isonicotinonitrile as a suspension in 2-MeTHF (0.8mL). The mixture was stirred for 2hrs then quenched with water, diluted with EtOAc, acidified with 1N HCI then stirred for 15min. The layers were separated, the organics washed with water then brine, dried over Na₂SO₄, filtered, and concentrated. Purification using ISCO 40g GOLD silica 0-60% EtOAc/hexanes gave the product (490mg, 60%).

### Step 4, Example 2: racemic 2-(3-(2-chloro-4-((5-cyclopropyl-3-(2,6-dichlorophenyl)isoxazol-4-yl)methoxy)phenyl)-3-hydroxypyrrolidin-1-yl)isonicotinic acid (2)

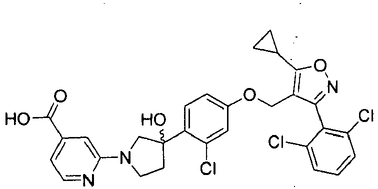

In 100ml flask a solution of 2-(3-(2-chloro-4-((5-cyclopropyl-3-(2,6-dichlorophenyl)isoxazol-4-yl)methoxy)phenyl)-3-hydroxypyrrolidin-1-yl)isonicotinonitrile (430 mg, 0.739 mmol) in ethanol (14 ml) was treated with 4M sodium hydroxide in water (7.4 ml) and stirred at 70°C for 2.5hrs. The mixture was cooled to room temperature and the EtOH was removed under vacuum. The remaining solution was cooled in ice bath, treated with water ∼2ml, and adjusted pH to ∼4 with 1M HCl. The mixture was extracted with EtOAc, washed with brine, dried over Na₂SO₄, filtered and concentrated to give the product (421mg, 95% yield). ¹H NMR (300 MHz, DMSO-d₆) δ 13.32 (s, 1H), 8.19 (dd, *J* = 5.1, 0.8 Hz, 1H), 7.66 - 7.47 (m, 4H), 6.93 (dd, *J* = 5.2, 1.3 Hz, 1H), 6.89 (d, *J* = 2.6 Hz, 1H), 6.84 (s, 1H), 6.77 (dd, *J* = 8.8, 2.6 Hz, 1H), 5.43 (s, 1H), 4.90 (s, 2H), 3.89 - 3.75 (m, 2H), 3.65 (d, *J* = 8.9 Hz, 1H), 3.58 - 3.45 (m, 1H), 2.61 (t, *J* = 10.5 Hz, 1H), 2.46 - 2.38 (m, 1H), 2.30 - 2.15 (m, 1H), 1.25 - 1.05 (m, 4H). MS (M+H): 600.05.

This material was then subjected to chiral resolution (SFC AD-H, 40%EtOH) to give single isomers: Examples 2a and 2b.

### Example 2a: 2-(3-(2-chloro-4-((5-cyclopropyl-3-(2,6-dichlorophenyl)isoxazol-4-yl)methoxy)phenyl)-3-hydroxypyrrolidin-1-yl)isonicotinic acid, enantiomer 1

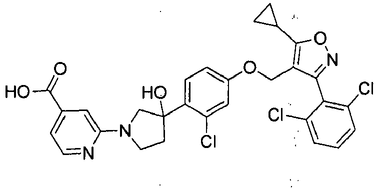

¹H NMR (300 MHz, DMSO-*d*₆) δ 13.30 (s, 1H), 8.20 (dd, *J* = 5.2, 0.7 Hz, 1H), 7.67 - 7.46 (m, 5H), 6.95 (dd, *J* = 5.1, 1.3 Hz, 1H), 6.90 (d, *J* = 2.6 Hz, 1H), 6.85 (s, 1H), 6.78 (dd, *J* = 8.8, 2.6 Hz, 1H), 5.44 (s, 1H), 4.92 (s, 2H), 3.90 - 3.74 (m, 2H), 3.71 - 3.65 (m, 1H), 3.62 - 3.47 (m, 1H), 2.64 (q, *J* = 10.2, 9.5 Hz, 1H), 2.50 - 2.38 (m, 1H), 2.24 (dd, *J* = 12.6, 6.0 Hz, 1H), 1.26 - 1.03 (m, 4H). MS (M+H): 600.12.

### Example 2b: 2-(3-(2-chloro-4-((5-cyclopropyl-3-(2,6-dichlorophenyl)isoxazol-4-yl)methoxy)phenyl)-3-hydroxypyrrolidin-1-yl)isonicotinic acid, enantiomer 2

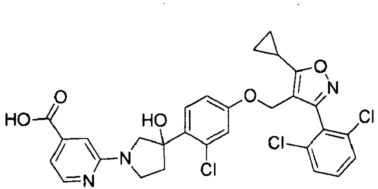

¹H NMR (300 MHz, DMSO-*d*₆) δ 13.34 (s, 1H), 8.19 (dd, *J* = 5.1, 0.7 Hz, 1H), 7.65 - 7.46 (m, 4H), 6.94 (dd, *J* = 5.1, 1.3 Hz, 1H), 6.90 (d, *J* = 2.6 Hz, 1H), 6.84 (d, *J* = 1.4 Hz, 1H), 6.78 (dd, *J* = 8.8, 2.7 Hz, 1H), 5.44 (s, 1H), 4.91 (s, 2H), 3.89 - 3.76 (m, 2H), 3.75 - 3.60 (m, 1H), 3.60 - 3.46 (m, 1H), 2.72 - 2.55 (m, 1H), 2.50 - 2.38 (m, 1H), 2.30 - 2.18 (m, 1H), 1.26 - 1.07 (m, 4H). MS (M+H): 600.07

### General synthesis 3

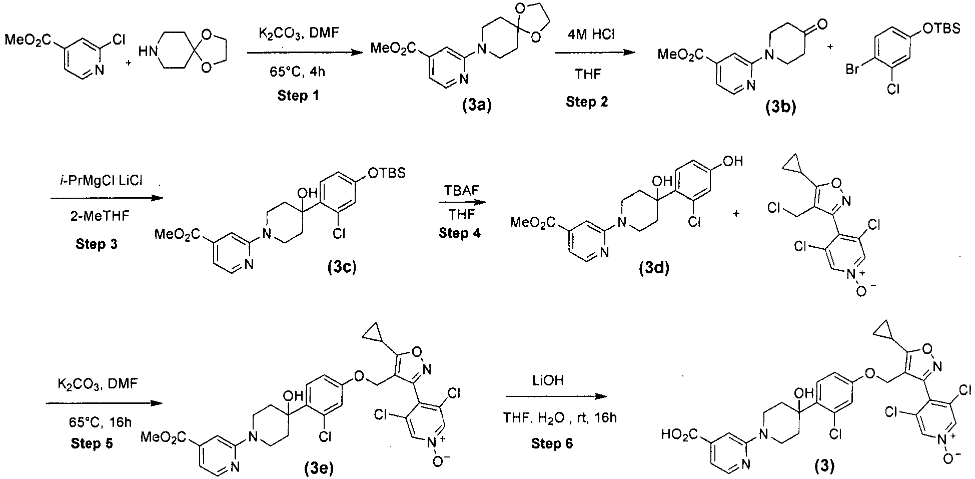

### Example 3: 4-(4-((4-(1-(4-carboxypyridin-2-yl)-4-hydroxypiperidin-4-yl)-3-chlorophenoxy)methyl)-5-cyclopropylisoxazol-3-yl)-3,5-dichloropyridine 1-oxide (3)

### Step 1: Methyl 2-(1,4-dioxa-8-azaspiro[4.5]decan-8-yl)isonicotinate (3a)

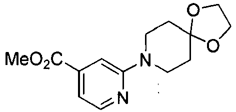

To a solution of methyl 2-chloropyridine-4-carboxylate (2.6 g, 15.2 mmol) in DMF (12.7 mL) was added 1,4-dioxa-8-azaspiro[4.5]decane (2.3 mL, 18.3 mmol), K₂CO₃ (4.2 g, 30.5 mmol). The mixture was stirred at 65 °C for 4 hours under N₂ atmosphere. The resulting solution was cooled to room temperature, H₂O was added, the aqueous was extracted with EtOAc, the combined organics were dried over Na₂SO₄ and concentrated. Purification by chromatography (ISCO (24g silica column) using a gradient of 100% hexanes - 1:1 hexance/EtOAc) gave compound **3a** (220mg, 5% yield).

### Step 2: Methyl 2-(4-oxopiperidin-1-yl)isonicotinate (3b)

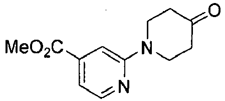

Following the procedure described in Example 1/Step 2, compound **3b** was obtained from Methyl 2-(1,4-dioxa-8-azaspiro[4.5]decan-8-yl)isonicotinate **(3a)** in quantitative yield.

### Step 3: Methyl 2-(4-(4-((tert-butyldimethylsilyl)oxy)-2-chlorophenyl)-4-hydroxypiperidin-1-yl)isonicotinate (3c)

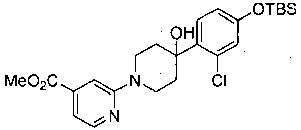

In an oven dried reaction vial flushed with N₂ was placed (4-bromo-3-chlorophenoxy)(tert-butyl)dimethylsilane (255 mg, 0.79 mmol) in 2-MeTHF (1 mL). 1.3M Isopropylmagnesium chloride lithium chloride complex in THF (0.92 mL, 1.2 mmol) was then added dropwise via syringe at room temperature, and the mixture was stirred for 3 h. A solution of methyl 2-(4-oxopiperidin-1-yl)isonicotinate **(3b)** in 2-MeTHF (1 ml) was slowly added and the mixture was allowed to stir for 30 min at room temperature. The reaction was quenched with H₂O, extracted with EtOAc, the organics were washed with brine, dried over Na₂SO₄, and concentrated. Purification by chromatography (ISCO (12g silica column) using a gradient of 100% hexanes - 7:3 hexance/EtOAc) gave compound **3c** (124mg, 30% yield).

### Step 4: Methyl 2-(4-(2-chloro-4-hydroxyphenyl)-4-hydroxypiperidin-1-yl)isonicotinate (3d)

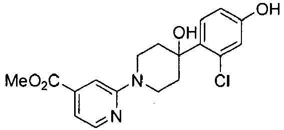

To a solution of methyl 2-(4-(4-((tert-butyldimethylsilyl)oxy)-2-chlorophenyl)-4-hydroxypiperidin-1-yl)isonicotinate **(3c)** (124 mg, 0.26 mmol) in 2-MeTHF (2 mL) was added 1M TBAF solution in THF (0.3 mL, 0.29 mmol) at room temperature and the mixture was stirred at room temperature for 30 min. The mixture was quenched with water and extracted with EtOAc. The organic phase was washed with brine (20 mL), dried over Na₂SO₄, and concentrated to give the title compound (94mg), which was used directly in next step without further purification.

### Step 5: 3,5-Dichloro-4-(4-((3-chloro-4-(4-hydroxy-1-(4-(methoxycarbonyl)pyridin-2-yl)piperidin-4-yl)phenoxy)methyl)-5-cyclopropylisoxazol-3-yl)pyridine 1-oxide (3e)

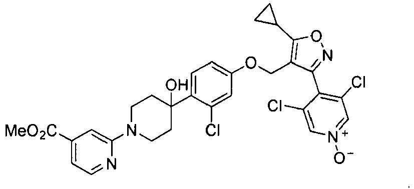

3,5-dichloro-4-(4-(chloromethyl)-5-cyclopropylisoxazol-3-yl)pyridine 1-oxide (100 mg, 0.31 mmol) (prepared as described in WO2011/020615), methyl 2-(4-(2-chloro-4-hydroxyphenyl)-4-hydroxypiperidin-1-yl)isonicotinate **(3d)** (125 mg, 0.34 mmol) and K₂CO₃ (87 mg, 0.63 mmol) were combined in anhydrous DMF (1.6 mL) at room temperature. The mixture was heated to 65 °C under nitrogen and stirred overnight. The resulting solution was cooled to room temperature, quenched with H₂O and extracted with EtOAc. The combined organic layers were washed with brine, dried over Na₂SO₄, filtered, and concentrated. Purification by chromatography (ISCO (4g silica column) using a gradient of 100% hexanes - 100% EtOAc) gave compound **3e** (202mg, 55% yield).

### Step 6, Example 3: 4-(4-((4-(1-(4-carboxypyridin-2-yl)-4-hydroxypiperidin-4-yl)-3-chlorophenoxy)methyl)-5-cyclopropylisoxazol-3-yl)-3,5-dichloropyridine 1-oxide (3)

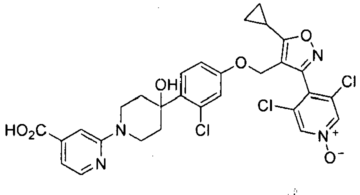

To a solution of 3,5-dichloro-4-(4-((3-chloro-4-(4-hydroxy-1-(4-(methoxycarbonyl)pyridin-2-yl)piperidin-4-yl)phenoxy)methyl)-5-cyclopropylisoxazol-3-yl)pyridine 1-oxide **(3e)** (111 mg, 0.18 mmol) in THF (1.4 mL) and H₂O (0.2 mL) was added LiOH·H₂O (15 mg, 0.36 mmol) at room temperature. After stirring for 16 h, H₂O and dichloromethane were added. 1N *aq.* HCI solution was added to acidify the mixture to pH = 3∼4, which was then extracted with dichloromethane. The combined organic layers were washed with brine, dried over anhydrous Na₂SO₄, filtered, and concentrated. The solid was triturated with dichloromethane and dried to give compound **3** (114 mg, 57% yield). ¹H NMR (300 MHz, d₆-DMSO) δ 13.34 (s, 1H), 8.72 (s, 2H), 8.24 (d, *J* = 5.1 Hz, 1H), 7.66 (d, *J* = 8.7 Hz, 1H), 7.24 (s, 1H), 6.98 (dd, *J* = 5.1, 1.1 Hz, 1H), 6.90 - 6.77 (m, 2H), 5.24 (s, 1H), 4.95 (s, 2H), 4.22 (br d, *J* = 12.3 Hz, 2H), 3.45-3.26 (m, 2H), 2.49 - 2.35 (m, 3H), 1.55 (br d, *J* = 13.1 Hz, 2H), 1.26 - 1.03 (m, 4H). MS (ESI+) *m*/*z* 633.0 (M + H).

### General synthesis 4

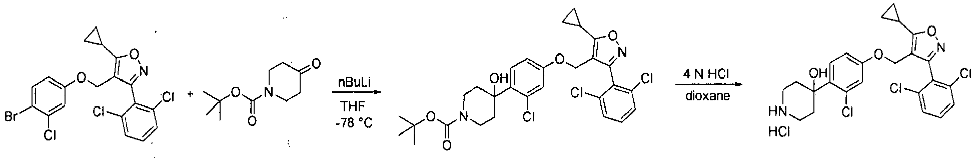

### Step 1: tert-butyl 4-(2-chloro-4-((5-cyclopropyl-3-(2,6-dichlorophenyl)isoxazol-4-yl)methoxy)phenyl)-4-hydroxypiperidine-1-carboxylate

To a round bottomed flask equipped with a magnetic stirring bar and a nitrogen tee, 4-((4-bromo-3-chlorophenoxy)methyl)-5-cyclopropyl-3-(2,6-dichlorophenyl)isoxazole (0.5 g, 1.06 mmol) and THF (19 mL) were added. The mixture was cooled to -78 °C in an acetone / liquid N₂ bath. *n*-BuLi (1.6M in hexanes, 0.86 mL, 1.37 mmol) was added dropwise, and the resulting mixture was stirred at this temperature for 30 minutes. Tert-butyl 4-oxopiperidine-1-carboxylate (210 mg, 1.06 mmol) in THF (5.3 mL) was added dropwise and the reaction was stirred for 30min. The reaction mixture was quenched with water (10 mL) and ethyl acetate (30 mL) and warmed to RT. More ethyl acetate (170 mL) was added and the mixture was washed with water (20 mL X 2), brine (20 mL), dried over Na₂SO₄, filtered and concentrated. The residue was passed through a silica gel column to afford the desired product (478 mg).

### Step 2: 4-(2-chloro-4-((5-cyclopropyl-3-(2,6-dichlorophenyl)isoxazol-4-yl)methoxy)phenyl)piperidin-4-ol hydrochloride

To a round bottomed flask equipped with a magnetic stirring bar, tert-butyl 4-(2-chloro-4-((5-cyclopropyl-3-(2,6-dichlorophenyl)isoxazol-4-yl)methoxy)phenyl)-4-hydroxypiperidine-1-carboxylate (558 mg, 0.94 mmol) and dichloromethane (56 mL) were added. Following the addition of HCI in dioxane (4 N, 9.4 mL, 38 mmol), the mixture was stirred at room temperature for 1.5 hrs. The volatiles were removed *in vacuo* to yield the product (406 mg).

### Example 4: racemic 2-(3-(2-chloro-4-((5-cyclopropyl-3-(2,6-dichlorophenyl)isoxazol-4-yl)methoxy)phenyl)-3-hydroxy-8-azabicyclo[3.2.1]octan-8-yl)isonicotinic acid

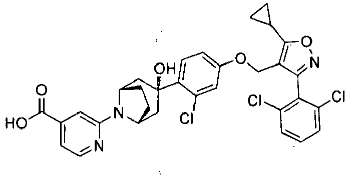

This compound was synthesized according to the procedure described for **Example 2,** substituting nortropine (2000 mg, 15.7 mmol) as a starting material.
¹H NMR (300 MHz, DMSO-d6) δ 13.31 (s, 1H), 8.22 (dd, J = 5.1, 0.7 Hz, 1H), 7.67 - 7.44 (m, 5H), 7.10 (t, J = 1.1 Hz, 1H), 6.91 (dd, J = 5.1, 1.3 Hz, 1H), 6.73 (dd, J = 8.9, 2.6 Hz, 1H), 6.57 (d, J = 2.6 Hz, 1H), 5.18 (s, 1H), 4.80 (s, 2H), 4.58 (s, 2H), 2.66 (dd, J = 14.2, 4.0 Hz, 2H), 2.46 - 2.31 (m, 3H), 1.94 (d, J = 5.4 Hz, 2H), 1.45 (d, J = 14.0 Hz, 2H), 1.20 - 0.98 (m, 4H). MS (M+H): 640.00.

### Example 5: racemic 2-(3-(2-chloro-4-((5-cyclopropyl-3-(2-(trifluoromethoxy) phenyl)isoxazol-4-yl)methoxy)phenyl)-3-hydroxy-8-azabicyclo[3.2.1]octan-8-yl) isonicotinic acid

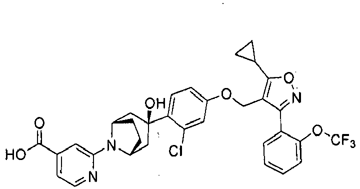

This compound was synthesized according to the procedure described for Example 4, using appropriate starting materials. ¹H NMR (300 MHz, DMSO-*d*₆) δ 13.31 (s, 1H), 8.22 (dd, *J* = 5.1, 0.7 Hz, 1H), 7.70 - 7.41 (m, 5H), 7.10 (t, *J* = 1.1 Hz, 1H), 6.91 (dd, *J* = 5.1, 1.3 Hz, 1H), 6.78 (dd, *J* = 8.9, 2.6 Hz, 1H), 6.64 (d, *J* = 2.6 Hz, 1H), 5.19 (s, 1H), 4.84 (s, 2H), 4.59 (s, 2H), 2.67 (dd, *J* = 14.1, 4.0 Hz, 2H), 2.47 - 2.23 (m, 3H), 1.98 - 1.91 (m, 2H), 1.47 (d, *J* = 14.1 Hz, 2H), 1.18 - 0.99 (m, 4H). (M+H): 656.10

### Example 6: racemic 6-(3-(2-chloro-4-((5-cyclopropyl-3-(2,6-dichlorophenyl)isoxazol-4-yl)methoxy)phenyl)-3-hydroxypyrrolidin-1-yl)nicotinic acid

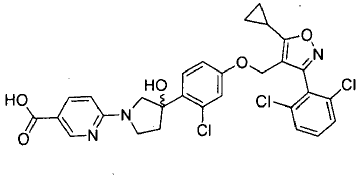

This compound was synthesized according to the procedure described for Example 2, substituting 2-bromo-5-cyanopyridine (1000 mg, 5.46 mmol) as a starting material.
¹H NMR (300 MHz, DMSO-*d*₆) δ 12.38 (s, 1H), 8.65 - 8.58 (m, 1H), 7.90 (dd, *J* = 8.9, 2.3 Hz, 1H), 7.67 - 7.46 (m, 4H), 6.91 (d, *J* = 2.6 Hz, 1H), 6.79 (dd, *J* = 8.8, 2.7 Hz, 1H), 6.50 (d, *J* = 8.9 Hz, 1H), 5.50 (s, 1H), 4.92 (s, 2H), 3.83 (d, *J* = 11.3 Hz, 1H), 3.75 (s, 1H), 3.61 - 3.54 (m, 1H), 2.64 (q, *J* = 10.7, 9.9 Hz, 1H), 2.50 - 2.38 (m, 1H), 2.24 (d, *J* = 9.7 Hz, 1H), 1.27 - 1.06 (m, 4H).

(M+H): 600.20. This material was then subjected to chiral resolution (Chiralpak AD-H; Heptane:IPA) to give single isomers: **Example 6a** and **Example 6b.**

### Example 6a: 6-(3-(2-chloro-4-((5-cyclopropyl-3-(2,6-dichlorophenyl)isoxazol-4-yl)methoxy)phenyl)-3-hydroxypyrrolidin-1-yl)nicotinic acid, enantiomer 1

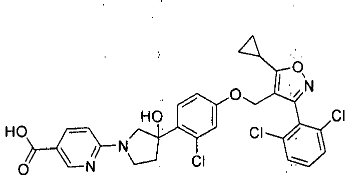

¹H NMR (300 MHz, DMSO-*d*₆) δ 12.41 (s, 1H), 8.61 (d, 1H), 7.90 (dd, *J* = 8.9, 2.3 Hz, 1H), 7.75 - 7.42 (m, 4H), 6.90 (d, *J* = 2.6 Hz, 1H), 6.79 (dd, *J* = 8.8, 2.6 Hz, 1H), 6.49 (d, *J* = 8.9 Hz, 1H), 5.50 (s, 1H), 4.92 (s, 2H), 3.83 (d, *J* = 11.3 Hz, 1H), 3.73 (s, 1H), 3.62 - 3.52 (m, 1H), 2.73 - 2.55 (m, 1H), 2.50 - 2.38 (m, 1H), 2.31 - 2.18 (m, 1H), 1.25 - 1.06 (m, 4H). (M+H): 600.17

### Example 6b: 6-(3-(2-chloro-4-((5-cyclopropyl-3-(2,6-dichlorophenyl)isoxazol-4-yl)methoxy)phenyl)-3-hydroxypyrrolidin-1-yl)nicotinic acid, enantiomer 2

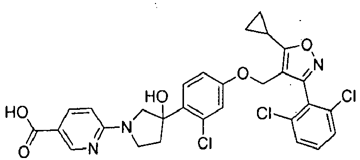

¹H NMR (300 MHz, DMSO-*d*₆) δ 12.38 (s, 1H), 8.61 (d, *J* = 2.3 Hz, 1H), 7.90 (dd, *J* = 8.9, 2.3 Hz, 1H), 7.67 - 7.48 (m, 4H), 6.91 (d, *J* = 2.6 Hz, 1H), 6.79 (dd, *J* = 8.8, 2.6 Hz, 1H), 6.49 (d, *J* = 8.9 Hz, 1H), 5.50 (s, 1H), 4.92 (s, 2H), 3.88 - 3.69 (m, 1H), 3.57 (d, *J* = 9.2 Hz, 1H), 2.64 (q, *J* = 10.3, 9.8 Hz, 1H), 2.50 - 2.38 (m, 1H), 2.26 (s, 1H), 1.25 - 1.08 (m, 4H). (M+H): 600.18.

### Example 7: racemic 5-(3-(2-chloro-4-((5-cyclopropyl-3-(2,6-dichlorophenyl)isoxazol-4-yl)methoxy)phenyl)-3-hydroxypyrrolidin-1-yl)nicotinic acid

### Step1b: 5-(3-hydroxypyrrolidin-1-yl)nicotinonitrile (2ab)

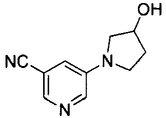

In a dry 3-neck flask with a condenser was placed 5-bromo-3-cyanopyridine (560 mg, 3.06 mmol), cesium carbonate (1994.03 mg, 6.12 mmol), Pd(dba)₃) (123.82 mg, 0.15 mmol), and Xantphos (141.65 mg, 0.24 mmol) then the flask was evacuated and filled with N₂ three times. A solution of 3-pyrrolidinol (0.32 ml, 3.98 mmol) in 1,4-dioxane (6 ml) was added via syringe and the mixture was heated at 100°C (oil bath) overnight. The mixture was cooled, treated with water and EtOAc, filtered through a pad of celite, and separated. The aqueous layer was washed with brine, the organics were dried over Na₂SO₄, filtered, and concentrated. Purification by chromatography (ISCO 40g silica 0-100% EtOAc/hexanes) gave the product (310 mg, 54%).

### Step 2: 5-(3-(2-chloro-4-((5-cyclopropyl-3-(2,6-dichlorophenyl)isoxazol-4-yl)methoxy)phenyl)-3-hydroxypyrrolidin-1-yl)nicotinic acid hydroxypyrrolidin-1-yl)nicotinic acid (7)

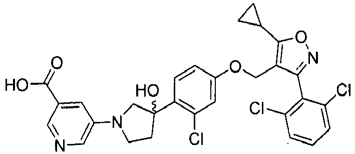

This compound was synthesized according to the procedure described for Example 2, using alternate Step1b to give material **2ab.** ¹H NMR (300 MHz, DMSO-*d*₆) δ 13.19 (s, 1H), 8.33 (d, *J* = 1.7 Hz, 1H), 8.12 (d, *J* = 2.9 Hz, 1H), 7.67 - 7.46 (m, 4H), 7.27 (dd, *J* = 2.9, 1.7 Hz, 1H), 6.91 (d, *J* = 2.6 Hz, 1H), 6.79 (dd, *J* = 8.8, 2.7 Hz, 1H), 5.50 (s, 1H), 4.92 (s, 2H), 3.84 (d, *J* = 10.5 Hz, 1H), 3.66 - 3.41 (m, 3H), 2.71 - 2.53 (m, 1H), 2.51 - 2.38 (m, 1H), 2.31 - 2.19 (m, 1H), 1.26 - 1.06 (m, 4H). (M+H): 600.21. This material was then subjected to chiral resolution (Chiralpak AD-H; Heptane:IPA) to give single isomers: **Example 7a** and **Example 7b.**

### Example 7a: 5-(3-(2-chloro-4-((5-cyclopropyl-3-(2,6-dichlorophenyl)isoxazol-4-yl)methoxy)phenyl)-3-hydroxypyrrolidin-1-yl)nicotinic acid, enantiomer 1

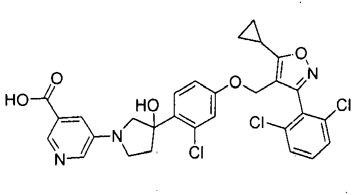

¹H NMR (300 MHz, DMSO-*d*₆) δ 13.14 (s, 1H), 8.31 (d, *J* = 1.5 Hz, 1H), 8.10 (d, *J* = 2.8 Hz, 1H), 7.70 - 7.45 (m, 4H), 7.24 (s, 1H), 6.88 (d, *J* = 2.5 Hz, 1H), 6.76 (dd, *J* = 8.6, 2.7 Hz, 1H), 5.47 (s, 1H), 4.89 (s, 2H), 3.81 (d, *J* = 10.5 Hz, 1H), 3.63 - 3.38 (m, 3H), 2.69 - 2.55 (m, 1H), 2.45 - 2.37 (m, 1H), 2.29 - 2.16 (m, 1H), 1.43 - 0.96 (m, 4H). (M+H): 600.22.

### Example 7b: 5-(3-(2-chloro-4-((5-cyclopropyl-3-(2,6-dichlorophenyl)isoxazol-4-yl)methoxy)phenyl)-3-hydroxypyrrolidin-1-yl)nicotinic acid, enantiomer 2

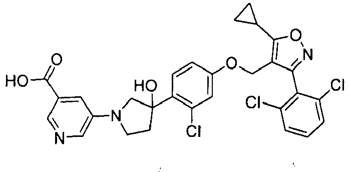

¹H NMR (300 MHz, DMSO-*d*₆) δ 13.14 (s, 1H), 8.31 (d, *J* = 1.5 Hz, 1H), 8.10 (d, *J* = 2.8 Hz, 1H), 7.64 - 7.45 (m, 4H), 7.28 - 7.20 (m, 1H), 6.88 (d, *J* = 2.6 Hz, 1H), 6.76 (dd, *J* = 8.8, 2.6 Hz, 1H), 5.47 (s, 1H), 4.89 (s, 2H), 3.81 (d, *J* = 10.5 Hz, 1H), 3.63 - 3.38 (m, 3H), 2.68 - 2.55 (m, 1H), 2.50 - 2.35 (m, 1H), 2.29 - 2.16 (m, 1H), 1.23 - 0.96 (m, 4H). (M+H): 600.22.

### Example 8: racemic 5-(3-(2-chloro-4-((5-cyclopropyl-3-(2-(difluoromethoxy)phenyl) isoxazol-4-yl)methoxy)phenyl)-3-hydroxypyrrolidin-1-yl)nicotinic acid

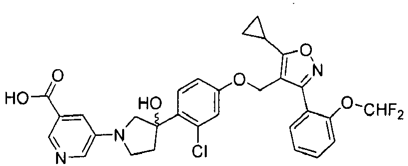

This compound was synthesized according to the procedure described for Example 12 using appropriate starting materials. ¹H NMR (300 MHz, DMSO-*d*₆) δ 13.14 (s, 1H), 8.32 (d, *J* = 1.7 Hz, 1H), 8.11 (d, *J* = 2.9 Hz, 1H), 7.64 - 7.22 (m, 6H), 7.20 (t, *J* = 76.5, 73.5 Hz, 1H), 6.92 (d, *J* = 2.6 Hz, 1H), 6.81 (dd, *J* = 8.8, 2.6 Hz, 1H), 5.49 (s, 1H), 4.95 (s, 2H), 3.83 (d, *J* = 10.5 Hz, 1H), 3.69 - 3.40 (m, 3H), 2.78 - 2.53 (m, 1H), 2.45 - 2.31 (m, 1H), 2.31 - 2.16 (m, 1H), 1.26 - 0.95 (m, 4H). (M+H): 598.31. This material was then subjected to chiral resolution (SFC AD-H, 30% MeOH/Ammonia) to give single isomers: **Example 8a** and **Example 8b.**

### Example 8a: 5-(3-(2-chloro-4-((5-cyclopropyl-3-(2-(difluoromethoxy)phenyl)isoxazol-4-yl)methoxy)phenyl)-3-hydroxypyrrolidin-1-yl)nicotinic acid, enantiomer 1

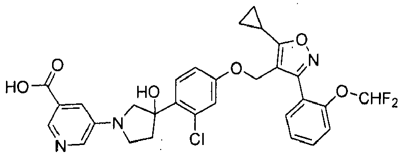

¹H NMR (300 MHz, DMSO-*d*₆) δ 8.30 (s, 1H), 8.05 (s, 1H), 7.69 - 7.51 (m, 2H), 7.52 - 7.41 (m, 1H), 7.40 - 7.22 (m, 3H), 7.20 (t, *J* = 73.5 Hz, 1H), 6.92 (d, *J* = 2.6 Hz, 1H), 6.80 (dd, *J* = 8.8, 2.6 Hz, 1H), 5.48 (s, 1H), 4.95 (s, 2H), 3.82 (d, *J* = 10.4 Hz, 1H), 3.64 - 3.42 (m, 3H), 2.70 - 2.56 (m, 1H), 2.46 - 2.31 (m, 1H), 2.28 - 2.17 (m, 1H), 1.41 - 0.93 (m, 4H). (M+H): 598.33.

### Example 8b: 5-(3-(2-chloro-4-((5-cyclopropyl-3-(2-(difluoromethoxy)phenyl)isoxazol-4-yl)methoxy)phenyl)-3-hydroxypyrrolidin-1-yl)nicotinic acid, enantiomer 2

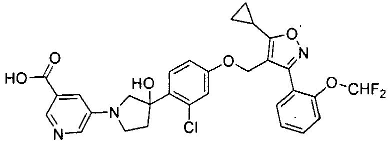

¹H NMR (300 MHz, DMSO-*d*₆) δ 8.31 (s, 1H), 8.07 (s, 1H), 7.63 - 7.39 (m, 3H), 7.39 - 7.28 (m, 2H), 7.28 - 7.22 (m, 1H), 7.20 (t, *J* = 73.2 Hz, 1H), 6.92 (d, *J* = 2.6 Hz, 1H), 6.80 (dd, *J* = 8.8, 2.7 Hz, 1H), 5.49 (s, 1H), 4.95 (s, 2H), 3.82 (d, *J* = 10.4 Hz, 1H), 3.64 - 3.42 (m, 3H), 2.70 - 2.56 (m, 1H), 2.46 - 2.31 (m, 1H), 2.31 - 2.07 (m, 1H), 1.24 - 1.00 (m, 4H). (M+H): 598.33.

### Example 9: racemic 6-(3-(2-chloro-4-((5-cyclopropyl-3-(2-(difluoromethoxy)phenyl) isoxazol-4-yl)methoxy)phenyl)-3-hydroxypyrrolidin-1-yl)nicotinic acid

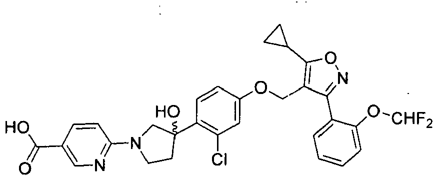

This compound was synthesized according to the procedure described for Example 2 using appropriate starting materials. ¹H NMR (300 MHz, DMSO-*d*₆) δ 12.35 (s, 1H), 8.60 (dd, *J* = 2.4, 0.7 Hz, 1H), 7.89 (dd, *J* = 8.9, 2.3 Hz, 1H), 7.63 - 7.41 (m, 3H), 7.39 - 7.27 (m, 2H), 7.20 (t, *J* = 73.2 Hz, 0H), 6.92 (d, *J* = 2.6 Hz, 1H), 6.80 (dd, *J* = 8.8, 2.6 Hz, 1H), 6.48 (d, *J* = 9.0 Hz, 1H), 5.49 (s, 1H), 4.94 (s, 2H), 3.82 (d, *J* = 11.4 Hz, 1H), 3.79 - 3.36 (m, 2H), 2.68 - 2.58 (m, 1H), 2.46 - 2.30 (m, 1H), 2.24 (t, *J* = 1.8 Hz, 1H), 2.32 - 2.15 (m, 1H), 1.24 - 0.97 (m, 4H). (M+H): 598.33. This material was then subjected to chiral resolution (Chiralpak AD-H; Heptane:IPA) to give single isomers: **Example 9a** and **Example 9b.**

### Example 9a: 6-(3-(2-chloro-4-((5-cyclopropyl-3-(2-(difluoromethoxy)phenyl)isoxazol-4-yl)methoxy)phenyl)-3-hydroxypyrrolidin-1-yl)nicotinic acid, enantiomer 1

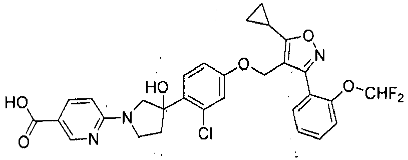

¹H NMR (300 MHz, DMSO-*d*₆) δ 12.32 (s, 1H, w), 8.59 (d, 1H), 7.88 (dd, *J* = 8.9, 2.3 Hz, 1H), 7.63 - 7.41 (m, 3H), 7.39 - 7.27 (m, 2H), 7.20 (t, *J* = 73.5 Hz, 1H), 6.92 (d, *J* = 2.7 Hz, 1H), 6.80 (dd, *J* = 8.8, 2.7 Hz, 1H), 6.47 (d, *J* = 8.9 Hz, 1H), 5.49 (s, 1H), 4.94 (s, 2H), 3.82 (d, *J* = 11.1 Hz, 1H), 3.75 - 3.68 (m, 1H), 3.60 - 3.51 (m, 1H), 2.68 - 2.51 (m, 1H), 2.49 - 2.30 (m, 1H), 2.31 - 2.16 (m, 1H), 1.24 - 1.00 (m, 4H). (M+H): 598.37.

### Example 9b: 6-(3-(2-chloro-4-((5-cyclopropyl-3-(2-(difluoromethoxy)phenyl)isoxazol-4-yl)methoxy)phenyl)-3-hydroxypyrrolidin-1-yl)nicotinic acid, enantiomer 2

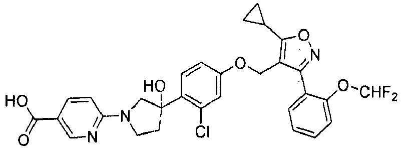

¹H NMR (300 MHz, DMSO-*d*₆) δ 12.33 (s, 1H, w), 8.59 (d, *J* = 2.3 Hz, 1H), 7.88 (dd, *J* = 8.9, 2.4 Hz, 1H), 7.63 - 7.41 (m, 3H), 7.39 - 7.27 (m, 2H), 7.20 (t, *J* = 73.5 Hz, 1H), 6.92 (d, *J* = 2.6 Hz, 1H), 6.80 (dd, *J* = 8.8, 2.7 Hz, 1H), 6.47 (d, *J* = 8.9 Hz, 1H), 5.49 (s, 1H), 4.94 (s, 2H), 3.82 (d, *J* = 11.0 Hz, 1H), 3.74 - 3.68 (m, 1H), 3.61 - 3.51 (m, 1H), 2.73 - 2.55 (m, 1H), 2.46 - 2.30 (m, 1H), 2.32 - 2.13 (m, 1H), 1.24 - 1.00 (m, 4H). (M+H): 597.96

### Example 10: 2-(4-(2-chloro-4-((5-cyclopropyl-3-(2,6-dichlorophenyl)isoxazol-4-yl) methoxy)phenyl)-4-hydroxypiperidin-1-yl)-6-(trifluoromethyl)isonicotinic acid

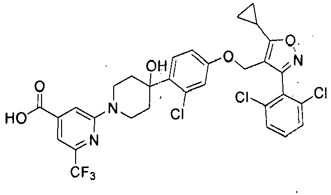

This compound was synthesized according to the procedure described for Example 1 using 2-chloro-6-(trifluoromethyl)isonicotinonitrile (120 mg, 0.58 mmol) as a starting material. ¹H NMR (300 MHz, DMSO-*d*₆) δ 13.81 (s, 1H, w), 7.67 - 7.45 (m, 5H), 7.25 (s, 1H), 6.81 - 6.72 (m, 2H), 5.29 (s, 1H), 4.86 (s, 2H), 4.26 (d, *J* = 13.0 Hz, 2H), 3.54 - 3.32 (m, 2H), 2.54 - 2.34 (m, 2H), 1.57 (d, *J* = 13.2 Hz, 2H), 1.25 - 1.03 (m, 5H). MS (M+H): 682.03.

### Example 11: 2-(4-(2-chloro-4-((5-cyclopropyl-3-(2-(trifluoromethoxy)phenyl)isoxazol-4-yl)methoxy)phenyl)-4-hydroxypiperidin-1-yl)-6-(trifluoromethyl)isonicotinic acid

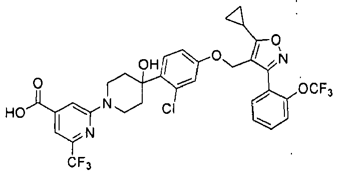

This compound was synthesized according to the procedure described for Example 1 using 2-chloro-6-(trifluoromethyl)isonicotinonitrile as a starting material. 1H NMR (300 MHz, DMSO-d6) δ 7.70 - 7.43 (m, 6H), 7.26 (d, J = 0.7 Hz, 1H), 6.87 - 6.75 (m, 2H), 5.28 (s, 1H), 4.90 (s, 2H), 4.23 (d, J = 13.0 Hz, 2H), 3.47 - 3.30 (m, 2H), 2.45 - 2.30 (m, 2H), 1.56 (d, J = 13.2 Hz, 2H), 1.25 - 1.01 (m, 5H). MS (M+H): 698.09.

### Example 12: 3-(4-(2-chloro-4-((5-cyclopropyl-3-(2-(trifluoromethoxy)phenyl)isoxazol-4-yl)methoxy)phenyl)-4-hydroxypiperidin-1-yl)-5-fluorobenzoic acid

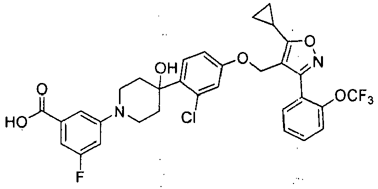

This compound was synthesized according to the procedure described for Example 1 using 3,5-difluorobenzonitrile as a starting material. ¹H NMR (300 MHz, DMSO-d6) δ 13.07 (s, 1H), 7.70 - 7.44 (m, 5H), 7.30 (dd, J = 2.4, 1.3 Hz, 1H), 7.03 (dt, J = 12.7, 2.4 Hz, 1H), 6.93 (ddd, J = 8.8, 2.3, 1.1 Hz, 1H), 6.88 - 6.76 (m, 2H), 5.20 (s, 1H), 4.90 (s, 2H), 3.68 (d, J = 12.6 Hz, 2H), 3.28 - 3.15 (m, 2H), 2.59 - 2.48 (m, 1H), 2.38 (tt, J = 8.1, 5.2 Hz, 1H), 1.56 (d, J = 13.1 Hz, 2H), 1.20 - 1.01 (m, 4H). MS (M+H): 647.16.

### Example 13: 3-(4-(2-chloro-4-((5-cyclopropyl-3-(2,6-dichlorophenyl)isoxazol-4-yl)methoxy)phenyl)-4-hydroxypiperidin-1-yl)-5-fluorobenzoic acids

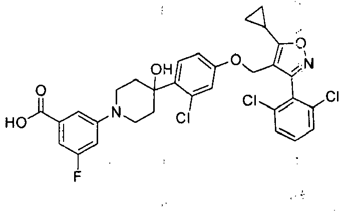

This compound was synthesized according to the procedure described for Example 1 using 3,5-difluorobenzonitrile as a starting material. ¹H NMR (300 MHz, DMSO-d6) δ 13.07 (s, 1H), 7.66 - 7.44 (m, 5H), 7.30 (dd, J = 2.4, 1.3 Hz, 1H), 7.02 (dt, J = 12.6, 2.4 Hz, 1H), 6.93 (ddd, J = 8.9, 2.3, 1.2 Hz, 1H), 6.81 - 6.70 (m, 1H), 5.19 (s, 1H), 4.87 (s, 2H), 3.67 (d, J = 12.0 Hz, 2H), 3.22 (t, J = 11.9 Hz, 2H), 2.50 (d, J = 6.1 Hz, 1H), 2.46 - 2.34 (m, 2H), 1.55 (d, J = 13.1 Hz, 2H), 1.27 - 1.03 (m, 5H). MS (M+H): 631.06

### Example 14: 5-(4-(2-chloro-4-((5-cyclopropyl-3-(2,6-dichlorophenyl)isoxazol-4-yl)methoxy)phenyl)-4-hydroxypiperidin-1-yl)nicotinic acid

### Step 1ab: 5-(1,4-dioxa-8-azaspiro[4.5]decan-8-yl)nicotinonitrile (1ab)

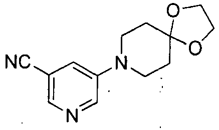

In a dry vial with a magnetic stir bar and septum cap was placed 5-bromo-3-cyanopyridine (500 mg, 2.73 mmol), cesium carbonate (1780.38 mg, 5.46 mmol), Pd(dba)3) (110.56 mg, 0.14 mmol), and Xantphos (126.47 mg, 0.22 mmol) and the vial was sealed and evacuated and filled with N₂ three times. A solution of 1,4-dioxa-8-azaspiro[4.5]decane (0.46 ml, 3.55 mmol) in 1,4-dioxane (5 ml) was added via syringe and the mixture was heated at 110°C (oil bath) overnight. The mixture was cooled then treated with water and EtOAc, filtered through celite, and separated. The organic phase was washed with brine, dried over Na₂SO₄, filtered and concentrated. Purification by chromatography (ISCO 40g silica, 0-100% EtOAc/hexanes) gave the product (605 mg, 92%).

### Step 2: 5-(4-(2-chloro-4-((5-cyclopropyl-3-(2,6-dichlorophenyl)isoxazol-4-yl)methoxy)phenyl)-4-hydroxypiperidin-1-yl)nicotinic acid

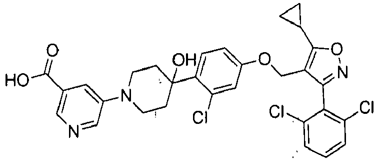

This compound was synthesized according to the procedure described for Example 1, using alternate **Step 1ab** to give material **1ab**. ¹H NMR (300 MHz, DMSO-d6) δ 12.41 (s, 1H), 8.62 (d, J = 2.4 Hz, 1H), 7.90 (dd, J = 9.0, 2.4 Hz, 1H), 7.68 - 7.46 (m, 4H), 6.89 (d, J = 9.1 Hz, 1H), 6.81 - 6.72 (m, 2H), 5.29 (s, 1H), 4.87 (s, 2H), 4.34 (d, J = 12.7 Hz, 2H), 3.40 - 3.26 (m, 2H), 2.48 - 2.35 (m, 3H), 1.54 (d, J = 13.2 Hz, 2H), 1.26 - 1.04 (m, 4H). MS (M+H): 614.12.

### Example 15: 4-(4-(2-chloro-4-((5-cyclopropyl-3-(2,6-dichlorophenyl)isoxazol-4-yl)methoxy)phenyl)-4-hydroxypiperidin-1-yl)benzoic acid

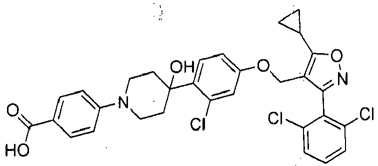

This compound was synthesized according to the procedure described for Example 14 using 4-iodobenzonitrile (100 mg, 0.44 mmol) as a starting material. ¹H NMR (300 MHz, DMSO-d6) δ 12.19 (s, 1H), 7.75 (d, J = 8.9 Hz, 2H), 7.67 - 7.47 (m, 4H), 6.97 (d, J = 9.1 Hz, 2H), 6.78 (s, 1H), 5.22 (s, 1H), 4.88 (s, 2H), 3.79 (d, J = 13.0 Hz, 2H), 3.31 - 3.20 (m, 3H), 2.55 (s, 0H), 2.49 - 2.35 (m, 1H); 1.54 (d, J = 13.1 Hz, 2H), 1.26 - 1.04 (m, 4H). MS (M+H): 613.32.

### Example 16: 6-(4-(2-chloro-4-((5-cyclopropyl-3-(2,6-dichlorophenyl)isoxazol-4-yl)methoxy)phenyl)-4-hydroxypiperidin-1-yl)nicotinic acid

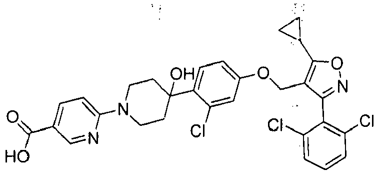

This compound was synthesized according to the procedure described for Example 2 using 6-bromonicotinonitrile (600 mg, 3.28 mmol) as a starting material. ¹H NMR (300 MHz, DMSO-d6) δ 13.27 (s, 1H), 8.52 (d, J = 2.9 Hz, 1H), 8.42 (d, J = 1.6 Hz, 1H), 7.73 - 7.47 (m, 6H), 6.82 - 6.71 (m, 2H), 5.21 (s, 1H), 4.88 (s, 2H), 3.72 (d, J = 12.5 Hz, 2H), 3.27 - 3.16 (m, 3H), 2.63 - 2.51 (m, 1H), 2.49 - 2.36 (m, 1H), 1.57 (d, J = 13.1 Hz, 2H), 1.27 - 1.04 (m, 4H). MS (M+H): 614.15.

### Example 17: 2-(4-(2-chloro-4-((5-cyclopropyl-3-(2,6-dichlorophenyl)isoxazol-4-yl)methoxy)phenyl)-4-hydroxypiperidin-1-yl)isonicotinamide

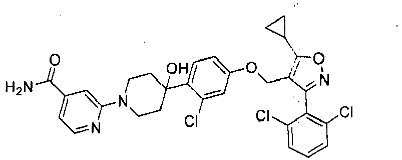

To a solution of Example 1 (12 mg, 0.02 mmol) in DMF (0.2 ml) was added 0.5M ammonia in dioxane (0.2 ml, 0.098 mmol) followed by BOP (17mg, 0.39mmol) and DIEA (0.01 ml, 0.06 mmol). The mixture was stirred overnight at room temperature, treated with EtOAc and water, separated, and extracted 1X with EtOAc. The combined organic phases were washed with brine, dried over Na₂SO₄, filtered and concentrated. Purification by chromatography (ISCO C18 reverse phase, 0-70% water/acetonitrile/0.1%TFA) gave the desired product (5.5mg, 46% yield). 1H NMR (300 MHz, DMSO-d6) δ 8.34 (d, J = 5.1 Hz, 1H), 8.23 (s, 1H), 7.85 - 7.63 (m, 5H), 7.39 (s, 1H), 7.11 (d, J = 5.3 Hz, 1H), 6.97 - 6.88 (m, 2H), 5.38 (s, 1H), 5.04 (s, 2H), 4.39 (d, J = 12.6 Hz, 2H), 3.53 - 3.36 (m, 3H), 2.65 - 2.45 (m, 2H), 1.69 (d, J = 13.2 Hz, 2H), 1.37 - 1.21 (m, 4H). MS (M+H): 613.56.

### Example 18: racemic 2-(3-(2-chloro-4-((5-cyclopropyl-3-(2,6-dichlorophenyl)isoxazol-4-yl)methoxy)phenyl)-3-hydroxypyrrolidin-1-yl)isonicotinamide

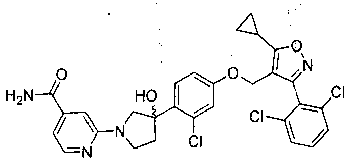

To a solution of Example 2 (40 mg, 0.067 mmol) in DMF (0.6 ml) was added 0.5M Ammonia in dioxane (0.666 ml, 0.333 mmol) followed by BOP (59 mg, 0.133 mmol) and DIEA (0.012 ml, 0.067 mmol). The mixture was stirred overnight at room temperature, treated with EtOAc and water, separated, and extracted 1X with EtOAc. The combined organic phases were washed with brine, dried over Na2SO4, filtered and concentrated. Purification by chromatography (ISCO C18 reverse phase, 0-70% water/acetonitrite/0.1%TFA) gave the product (25 mg, 62% yield). ¹H NMR (300 MHz, DMSO-d6) δ 8.31 (d, J = 5.2 Hz, 1H), 8.22 (s, 1H), 7.84 - 7.65 (m, 5H), 7.12 - 7.03 (m, 2H), 7.03 - 6.90 (m, 2H), 5.61 (s, 1H), 5.08 (s, 2H), 3.97 (d, J = 11.2 Hz, 2H), 3.91 - 3.78 (m, 1H), 3.79 - 3.63 (m, 1H), 2.81 (q, J = 10.7, 9.5 Hz, 1H), 2.64 - 2.50 (m, 1H), 2.39 (dd, J = 12.7, 6.5 Hz, 1H), 1.44 - 1.23 (m, 4H). MS (M+H): 599.19.

### Example 19: racemic 2-(2-(3-(2-chloro-4-((5-cyclopropyl-3-(2,6-dichlorophenyl)isoxazol-4-yl)methoxy)phenyl)-3-hydroxypyrrolidin-1-yl)isonicotinamido)ethane-1-sulfonic acid

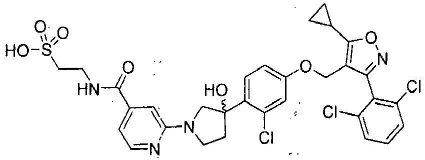

This compound was synthesized according to the procedure described for Example 18 using taurine (12.5 mg, 0.1 mmol) as a starting material. ¹H NMR (300 MHz, DMSO-d6) δ 9.03 (s, 1H), 8.28 (d, J = 6.2 Hz, 1H), 7.84 - 7.65 (m, 4H), 7.39 (s, 1H), 7.21 (d, J = 6.2 Hz, 1H), 7.10 (d, J = 2.6 Hz, 1H), 6.98 (dd, J = 8.9, 2.6 Hz, 1H), 5.86 (s, 1H), 5.10 (s, 2H), 4.16 - 4.09 (m, 2H), 4.06 - 3.91 (m, 1H), 3.96 - 3.74 (m, 2H), 3.66 - 3.36 (m, 2H), 2.90 - 2.76 (m, 3H), 2.57 - 2.50 (m, 1H), 1.55 - 1.08 (m, 5H).). MS (M+H): 707.20.

### Example 20: racemic ethyl (2-(3-(2-chloro-4-((5-cyclopropyl-3-(2,6-dichlorophenyl) isoxazol-4-yl)methoxy)phenyl)-3-hydroxypyrrolidin-1-yl)isonicotinoyl)glycinate

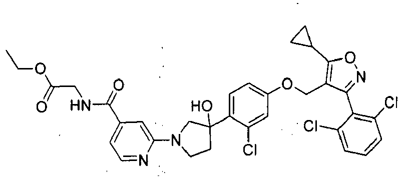

To a solution of Example 2 (40 mg, 0.07 mmol) in dichloromethane (0.5 ml) was added glycine ethylester HCI (10.22 mg, 0.07 mmol), EDCI (11.37 mg, 0.07 mmol), HOBt (9.89 mg, 0.07 mmol) and DIEA (0.02 ml, 0.13 mmol) and the mixture was stirred overnight at room temperature, treated with EtOAc and water, separated, and extracted once with EtOAc. The combined organic phases were washed with brine, dried over Na₂SO₄, filtered and concentrated. Purification by chromatography (ISCO 12g GOLD silica, 0-100% EtOAc/hexanes) gave the product (46 mg). ¹H NMR (300 MHz, DMSO-d6) δ 9.21 (t, J = 5.9 Hz, 1H), 8.36 (d, J = 5.2 Hz, 1H), 7.85 - 7.66 (m/4H), 7.13 - 7.04 (m, 2H), 7.04 - 6.92 (m, 2H), 5.64 (s, 1H), 5.10 (s, 2H), 4.29 (q, J = 7.1 Hz, 2H), 4.16 (d, J = 5.8 Hz, 2H), 4.02 (s, 2H), 3.90 - 3.84 (m, 1H), 3.81 - 3.68 (m, 1H), 2.82 (t, J = 10.4 Hz, 1H), 2.62 (dd, J = 8.0, 5.1 Hz, 1H), 2.41 (dd, J = 12.3, 6.2 Hz, 1H), 1.51 - 1.23 (m, 7H). MS (M+H): 685.33.

### Example 21: racemic (2-(3-(2-chloro-4-((5-cyclopropyl-3-(2,6-dichlorophenyl)isoxazol-4-yl)methoxy)phenyl)-3-hydroxypyrrolidin-1-yl)isonicotinoyl)glycine

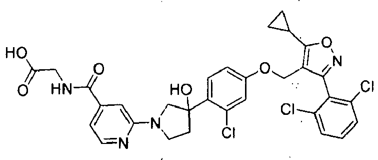

To a vial containing Example 20 (37 mg, 0.05 mmol) and THF (0.3 ml) was added 1M lithium hydroxide (0.16 ml, 0.16 mmol) and the mixture was stirred for 4 hrs at room temperature. EtOAc and water were added and the pH was adjusted to ∼6 with 1M HCl. The phases were separated, the combined organics washed with brine, dried over Na₂SO₄, filtered and concentrated and dried under vacuum (29 mg, 82%). 1H NMR (300 MHz, DMSO-d6) δ 12.65 (s, 1H, w), 8.89 (t, J = 5.9 Hz, 1H), 8.15 (d, J = 5.2 Hz, 1H), 7.66 - 7.47 (m, 5H), 6.93 - 6.86 (m, 2H), 6.82 (s, 1H), 6.78 (dd, J = 8.8, 2.7 Hz, 1H), 5.45 (s, 1H), 4.91 (s, 2H), 3.89 (d, J = 5.7 Hz, 2H), 3.81 (d, J = 10.9 Hz, 2H), 3.72 - 3.65 (m, 1H), 3.62 - 3.47 (m, 1H), 2.70 - 2.56 (m, 1H), 2.49 - 2.37 (m, 1H), 2.26 - 2.15 (m, 1H), 1.20 - 1.05 (m, 4H). MS (M+H): 657.25.

### Examples 22f and 22g: 3-(3-(2-chloro-4-((5-cyclopropyl-3-(2,6-dichlorophenyl)isoxazol-4-yl)methoxy)phenyl)-3-hydroxycyclopentyl)benzoic acid

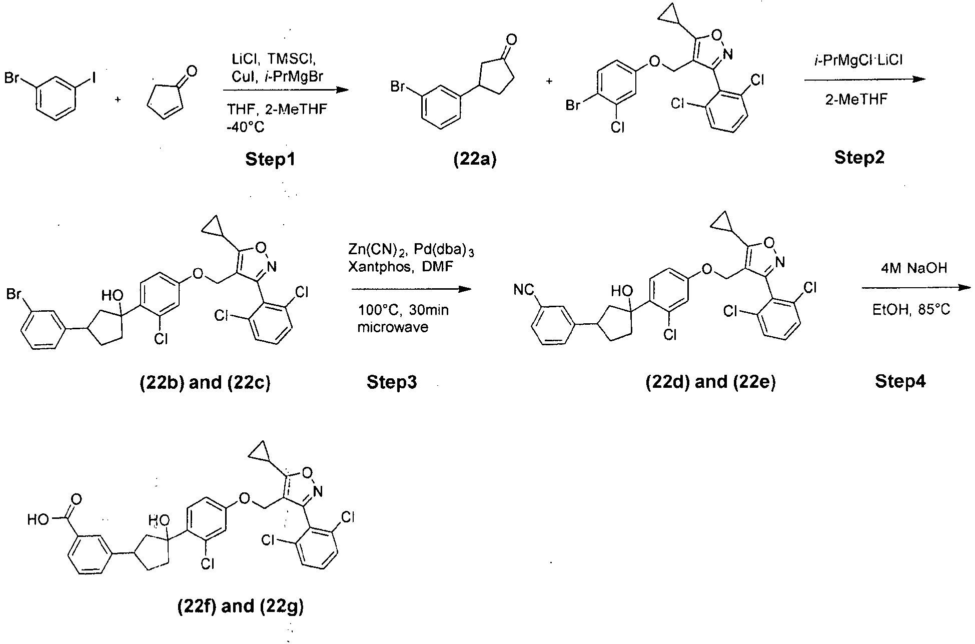

### Step1: 3-(3-bromophenyl)cyclopentan-1-one (22a)

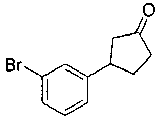

To a dry 3-neck flask under N₂ was added a solution of 1-bromo-3-iodobenzene (0.44 ml, 2.56 mmol) in THF (4 mL) then the mixture was cooled to -40°C in a dry ice/acetonitrile bath. 2.9M Isopropylmagnesium Bromide in 2-MeTHF (1.01 ml) was then added dropwise and the resulting mixture stirred at -40°C for 1hr. A separate flask was charged with lithium chloride (21 mg, 0.49 mmol) and placed under vacuum then flamed with a torch. Upon cooling to room temperature, copper(I) iodide (46 mg, 0.24 mmol) was added and the flask evacuated and filled with N₂ three times. THF (6 ml), Chlorotrimethylsilane (0.31 ml, 2.44 mmol), and cyclopent-2-enone (0.2 ml, 2.44 mmol) were then added in order via syringe whereupon a clear yellow solution formed. The resulting solution was then added to the first flask dropwise via syringe and the reaction was stirred for an additional hour at -40°C then quenched with aq NH₄Cl, diluted with ether, then acidified to pH 1 with 1N HCI and stirred for 0.5hr while warming to room temperature. The phases were separated, the organic layer washed with brine, dried over Na₂SO₄, filtered and concentrated. The residue was purified by flash chromatography (ISCO, 40g GOLD, EtOAc/hexanes) to give the product.

### Step2: 3-(3-bromophenyl)-1-(2-chloro-4-((5-cyclopropyl-3-(2,6-dichlorophenyl)isoxazol-4-yl))methoxy)phenyl)cyclopentan-1-ol (22b), (22c)

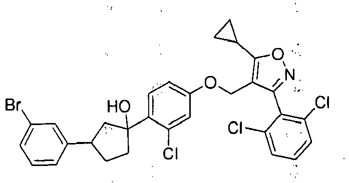

This material was synthesized following the general procedure described in in General Synthesis 2 Step3. Purification by column chromatography (ISCO 24g GOLD silca 0-100% EtOAc/hexanes) gave two isomers: **(22b)** and **(22c).**

### Step3: 3-(3-(2-chloro-4-((5-cyclopropyl-3-(2,6-dichlorophenyl)isoxazol-4-yl)methoxy)phenyl)-3-hydroxycyclopentyl)benzonitrile (22d)

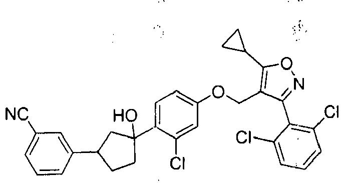

In a microwave vial were placed **(22b)** (160 mg, 0.25 mmol), zinc cyanide (36 mg, 0.3 mmol), Pd₂(DBA)₃ (23 mg, 0.03 mmol), and Xantphos (15 mg, 0.03 mmol). The vial was sealed then evacuated and filled with N₂ three times, then DMF (10 ml) was added. The reaction mixture was irradiated in a microwave reactor for 30min @100°C. The mixture was cooled to room temperature, concentrated under reduced pressure to remove the majority of DMF, then diluted with EtOAc and water, and filtered through celite. The phases were separated, the organic layer was washed three times with brine, dried over Na2SO4, filtered and concentrated. Purification by chromatography (ISCO 12g GOLD silica, 0-70% EtOAc/hexanes) gave the product **(22d)** (66mg, 45% yield).

### Step4, Example 22f: 3-(3-(2-chloro-4-((5-cyclopropyl-3-(2,6-dichlorophenyl)isoxazol-4-yl)methoxy)phenyl)-3-hydroxycyclopentyl)benzoic acid (22f)

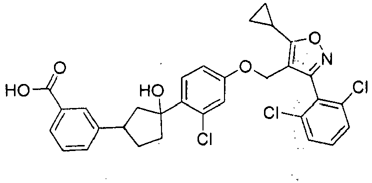

This compound was synthesized according to the procedure as described in General Synthesis 2 Step4 using **(22d)** (55mg, 0.095mmol) as a starting material. ¹H NMR (300 MHz, DMSO-*d*₆) δ 12.88 (s, 1H), 7.92 (t, *J* = 1.8 Hz, 1H), 7.74 (dt, *J* = 7.6, 1.4 Hz, 1H), 7.65 - 7.47 (m, 5H), 7.40 (t, *J* = 7.7 Hz, 1H), 6.85 (d, *J* = 2.6 Hz, 1H), 6.74 (dd, *J* = 8.8, 2.7 Hz, 1H), 5.15 (s, 1H), 4.88 (s, 2H), 3.52 - 3.38 (m, 1H), 2.83 - 2.71 (m, 1H), 2.49 - 2.30 (m, 1H), 2.14 - 1.75 (m, 3H), 1.36 - 1.04 (m, 4H), 0.89 - 0.75 (m, 2H). MS (M+H): 580.14.

### Example 22g, 3-(3-(2-chloro-4-((5-cyclopropyl-3-(2,6-dichlorophenyl)isoxazol-4-yl)methoxy)phenyl)-3-hydroxycyclopentyl)benzoic acid (22g)

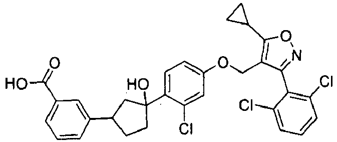

This compound was synthesized according to the procedure shown for Example 22f using **(22c)** as a starting material. ¹H NMR (300 MHz, DMSO-*d*₆) δ 12.90 (s, 1H), 7.86 (t, 1H), 7.76 (dt, *J* = 7.6, 1.4 Hz, 1H), 7.67 - 7.48 (m, 5H), 7.41 (t, *J* = 7.6 Hz, 2H), 6.85 (d, *J* = 2.6 Hz, 1H), 6.74 (dd, *J* = 8.8, 2.6 Hz, 1H), 5.13 (s, 1H), 4.88 (s, 2H), 3.63 - 3.56 (m, 1H), 2.47 - 2.36 (m, 2H), 2.29 (dd, *J* = 8.3, 4.3 Hz, 1H), 2.12 - 1.98 (m, 2H), 1.93 - 1.77 (m, 1H), 1.27 - 1.02 (m, 5H). MS (M+H): 580.21.

### Example 23: 2-(4-(2-chloro-4-((5-cyclopropyl-3-(2,6-dichlorophenyl)isoxazol-4-yl)methoxy)phenyl)-4-hydroxypiperidin-1-yl)thiazole-5-carboxylic acid

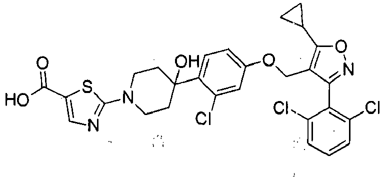

Example 23 was prepared according to the procedure described in general procedure 2 to provide the title compound (105 mg, 86% yield) ¹H NMR (300 MHz, DMSO- *d*₆) δ 13.31 (s, 1H), 8.32 (s, 1H), 7.32 - 7.65 (m, 5H), 7.10 (t, J = 1.1 Hz, 1H), 6.91 (dd, J = 5, 1.2 Hz, 1H), 6.73 (dd, J = 8.9, 2.6 Hz, 1H), 6.57 (d, J = 2.6 Hz, 1H), 5.22 (s, 1H), 4.80 (s, 2H), 4.58 (s, 2H), 2.66 (dd, J = 14.2, 4.0 Hz, 2H), 2.46 - 2.32 (m, 3H), 1.94 (d, J = 5.4 Hz, 2H), 1.45 (d, J = 14.0 Hz, 2H), 1.20 - 0.98 (m, 4H); (M+H): 620.2.

### Example 24: 2-(4-(4-((5-cyclopropyl-3-(2,6-dichlorophenyl)isoxazol-4-yl)methoxy)-2-methylphenyl)-4-hydroxygiperidin-1-yl)isonicotinic acid

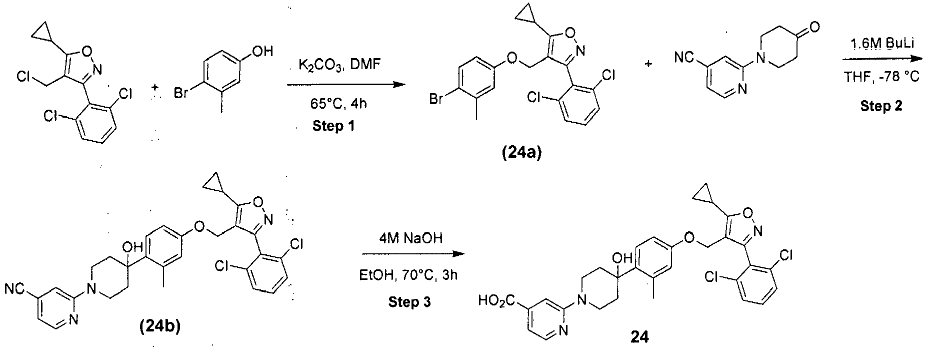

### Step1: 4-((4-bromo-3-methylphenoxy)methyl)-5-cyclopropyl-3-(2,6-dichlorophenyl)isoxazole (24a)

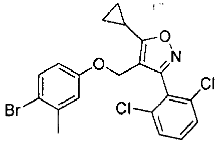

Following the procedure described in Example 3/Step 5, compound **24a** was obtained by combining 4-(chloromethyl)-5-cyclopropyl-3-(2,6-dichlorophenyl)isoxazole (prepared as described in WO2013/007387) and 4-bromo-3-methylphenol in 91% yield.

### Step2: 2-(4-(4-((5-cyclopropyl-3-(2,6-dichlorophenyl)isoxazol-4-yl)methoxy)-2-methylphenyl)-4-hydroxypiperidin-1-yl)isonicotinonitrile (24b)

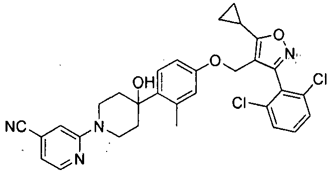

n-BuLi (1.6M in hexanes, 0.18 mL, 0.29 mmol) was added slowly to a chilled (-78 °C) solution of 4-((4-bromo-3-methylphenoxy)methyl)-5-cyclopropyl-3-(2,6-dichlorophenyl)isoxazole (**24a**) (100 mg, 0.22 mmol) in THF (4 mL). After 10min, a solution of 2-(4-oxopiperidin-1-yl)isonicotinonitrile (44 mg, 0.22 mmol) in THF (1.1 mL) was added. After a further 20 min the reaction mixture was quenched with H₂O, extracted with EtOAc, and the combined organics were washed with brine, dried over Na₂SO₄ and concentrated. The residue was purified by chromatography (ISCO (4g silica column) using a gradient of 100% hexanes - 2:1 hexance/EtOAc) to give compound **24b** (130mg, 43% yield).

### Step3, Example 24: 2-(4-(4-((5-cyclopropyl-3-(2,6-dichlorophenyl)isoxazol-4-yl)methoxy)-2-methylphenyl)-4-hydroxypiperidin-1-yl)isonicotinic acid (24)

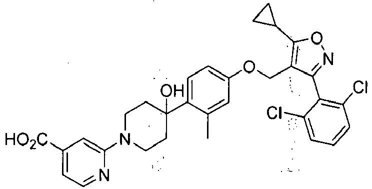

Following the procedure described in Example 1/Step 4, Example 24 was obtained from 2-(4-(4-((5-cyclopropyl-3-(2,6-dichlorophenyl)isoxazol-4-yl)methoxy)-2-methylphenyl)-4-hydroxypiperidin-1-yl)isonicotinonitrile (**24b**) in 81% yield. ¹H NMR (300 MHz, DMSO-*d*₆) δ 8.37 (d, *J* = 5.3 Hz, 1H), 7.83 - 7.64 (m, 3H), 7.47 (s, 1H), 7.39 - 7.29 (m, 1H), 7.17 (d, *J* = 5.4 Hz, 1H), 6.71 (dd, J = 4.6, 2.1 Hz, 2H), 4.97 (s, 2H), 4.33 (d, *J* = 12.7 Hz, 2H), 3.67 - 3.52 (m, 2H), 2.60 (s, 3H), 2.59 - 2.40 (m, 1H), 2.05 (s, 4H), 1.3601.24 (m, 4H). MS (ESI+) *m*/*z* 596.1 (M + H).

### Example 25: 2-(4-(2-chloro-4-((5-cyclopropyl-3-(3,5-dichloropyridin-4-yl)isoxazol-4-yl)methoxy)phenyl)-4-hydroxypiperidin-1-yl)isonicotinic acid

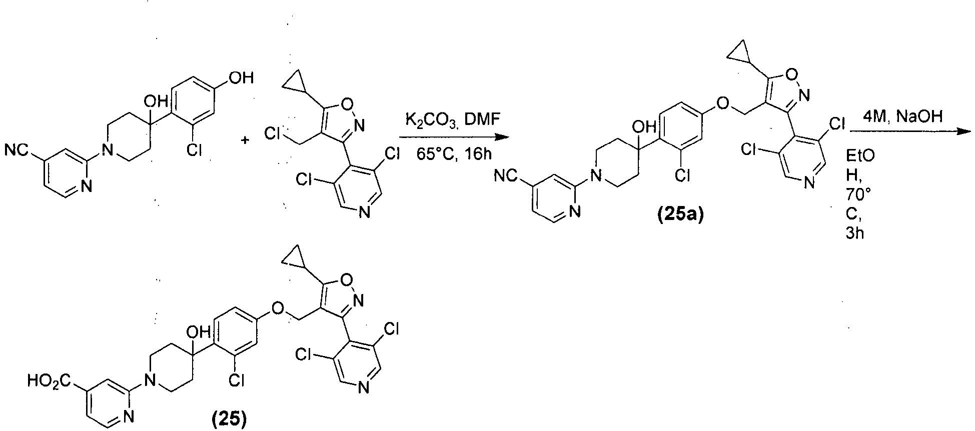

### Step1: 2-(4-(2-chloro-4-((5-cyclopropyl-3-(3,5-dichloropyridin-4-yl)isoxazol-4-yl)methoxy) phenyl)-4-hydroxypiperidin-1-yl)isonicotinonitrile (25a)

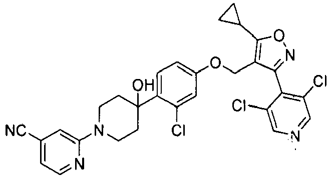

Following the procedure described in Example 3/Step 5, compound **25a** was obtained by combining 4-(chloromethyl)-5-cyclopropyl-3-(3,5-dichloropyridin-4-yl)isoxazole (prepared as described in WO2011/020615) and 2-(4-(2-chloro-4-hydroxyphenyl)-4-hydroxypiperidin-1-yl)isonicotinonitrile in 74% yield.

### Step2, Example 25: 2-(4-(2-chloro-4-((5-cyclopropyl-3-(3,5-dichloropyridin-4-yl)isoxazol-4-yl)methoxy)phenyl)-4-hydroxypiperidin-1-yl)isonicotinic acid (25)

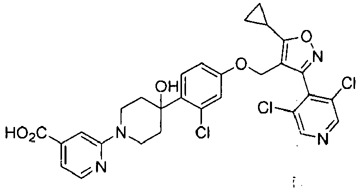

Following the procedure described in Example 1/Step 4, Example 25 was obtained from 2-(4-(4-((5-cyclopropyl-3-(2,6-dichlorophenyl)isoxazol-4-yl)methoxy)-2-methylphenyl)-4-hydroxypiperidin-1-yl)isonicotinonitrile in 81% yield. ¹H NMR (300 MHz, DMSO-*d*₆) δ 13.36 (s, 1H), 8.80 (s, 1H), 8.24 (d, J = 5.1 Hz, 1H), 7.64 (d, J = 8.6 Hz, 1H), 7.24 (s, 1H), 6.98 (dd, J = 5.0, 1.0 Hz, 1H), 6.81 - 6.69 (m, 2H), 5.25 (s, 1H), 4.96 (s, 2H), 4.22 (d, J = 12.7 Hz, 2H), 3.37-3.21 (m, 2H), 2.52-2.40 (m, 2H), 1.54 (d, J = 13.1 Hz, 2H), 1.22-109 (m, 4H). MS (ESI+) m/z 616.9 (M + H).

### Example 26: 2-(4-(2-chloro-4-((5-cyclopropyl-3-(2,6-dichlorophenyl)isoxazol-4-yl)methoxy) phenyl)-4-hydroxypiperidin-1-yl)nicotinic acid

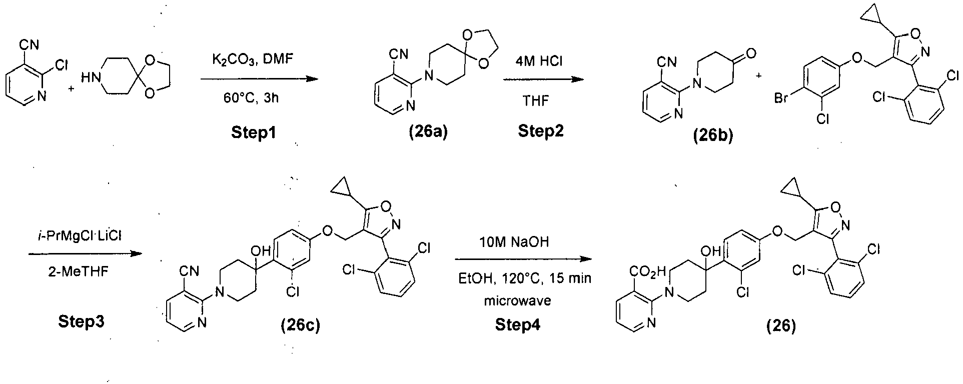

### Step1: 2-(1,4-dioxa-8-azaspiro[4.5]decan-8-yl)nicotinonitrile (26a)

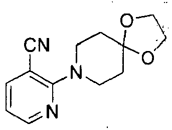

Following the procedure described in Example 1/Step 1, compound **26a** was obtained by combining 2-chloronicotinonitrile with 1,4-dioxa-8-azaspiro[4.5]decane in 94% yield.

### Step2: 2-(4-oxopiperidin-1-yl)nicotinonitrile (26b)

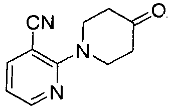

Following the procedure described in Example 1/Step 2, compound **26b** was obtained from 2-(1,4-dioxa-8-azaspiro[4.5]decan-8-yl)nicotinonitrile in 85% yield.

### Step3: 2-(4-(2-chloro-4-((5-cyclopropyl-3-(2,6-dichlorophenyl)isoxazol-4-yl)methoxy)phenyl)-4-hydroxypiperidin-1yl)nicotinonitrile (26c)

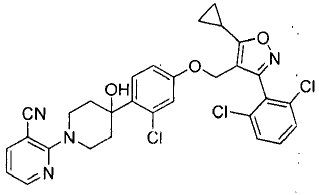

Following the procedure described in Example 1/Step 3, compound **26c** was obtained from 2-(4-oxopiperidin-1-yl)nicotinonitrile (**26b**) in 61% yield.

### Step 4. Example 26: 2-(4-(2-chloro-4-((5-cyclopropyl-3-(2,6-dichlorophenyl)isoxazol-4-yl)methoxy)phenyl)-4-hydroxypiperidin-1-yl)nicotinic acid (26)

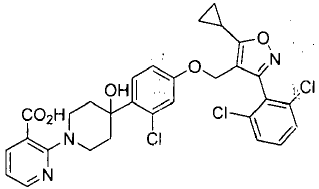

2-(4-(2-chloro-4-((5-cyclopropyl-3-(2,6-dichlorophenyl)isoxazol-4-yl)methoxy)phenyl)-4-hydroxypiperidin-1-yl)nicotinonitrile (100 mg, 0.17 mmol), 10M sodium hydroxide in water (0.29 ml) and ethanol (0.8 mL) were combined in a microwave vial and heated at 120 °C for 15 min in a microwave reactor. The mixture was cooled and the pH was adjusted to 5 with 1M aqueous HCI solution. The resulting mixture was filtered, and the insoluble solid was rinsed with water and Et₂O, and dried *in vacuo* to give Example 26 (23 mg, 22%). ¹H NMR (300 MHz, DMSO-*d*₆) δ 8.24 (dd, J = 4.8, 2.0 Hz, 1H), 7.98 (s, 1H), 7.77 (dd, J = 7.4, 2.0 Hz, 1H), 7.68 - 7.48 (m, 6H), 6.94 - 6.72 (m, 3H), 5.14 (s, 1H), 4.89 (s, 3H), 3.57 (d, J = 12.6 Hz, 2H), 3.37 - 3.20 (m, 1H), 2.69 - 2.55 (m, 2H), 2.50-2.40 (m, 1H), 1.53 (d, J = 12.9 Hz, 2H), 1.27 - 1.00 (m, 4H). MS (ESI+) m/z 615.2 (M + H).

### Example 27: 2-(4-(2-chloro-4-((4-cyclopropyl-1-(2-(trifluoromethoxy)phenyl)-1H-1,2,3-triazol-5-yl)methoxy)phenyl)-4-hydroxypiperidin-1-yl)isonicotinic acid

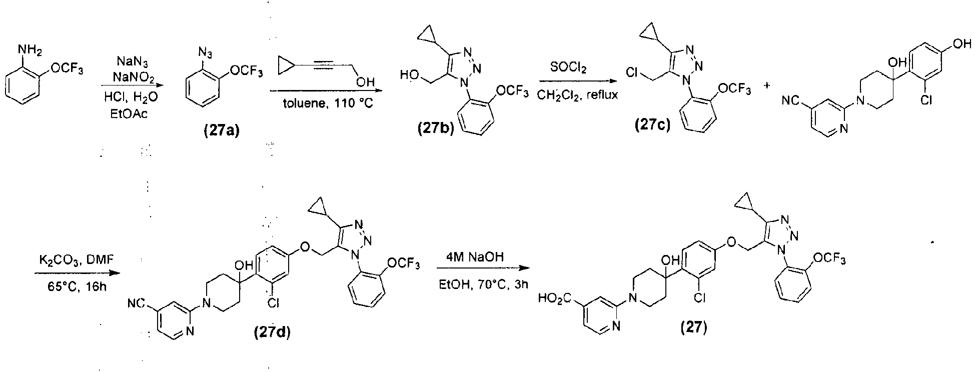

### Step1: 1-azido-2-(trifluoromethoxy)benzene (27a)

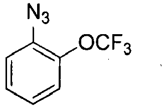

Concentrated HCl (7.9 mL) was added to a solution of 2-(trifluoromethoxy)aniline (1.1 mL, 7.9 mmol) in EtOAc (26 mL) at 0 °C. After 10min, a solution of sodium nitrite (1.6 g, 23.7 mmol) dissolved in water (4.6 mL) was added slowly over 5 min. The mixture was stirred for 30min, and then treated with a solution of sodium azide (1.5 g, 23.7 mmol) in water (5.3 mL) slowly over 5min. The reaction was stirred for 1h then treated with phosphate buffer saline (pH 7.4, 50 mL). The mixture was extracted with EtOAc, the organics were washed with brine, dried over Na₂SO₄ and concentrated to give compound **27a.**

### Step2: (4-cyclopropyl-1-(2-(trifluoromethoxy)phenyl)-1H-1,2,3-triazol-5-yl)methanol (27b)

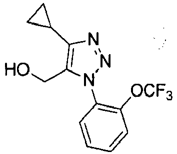

A solution of 1-azido-2-(trifluoromethoxy)benzene (**27a**, 2.0 g, 9.8 mmol) and 3-cyclopropylprop-2-yn-1-ol (1.2 mL, 9.8 mmol) in toluene (20 mL) was stirred at 110 °C under an atmosphere of nitrogen. After 16h, the solution was cooled to room temperature, and both water and EtOAc were added. The mixture was partitioned and the organics were extracted with EtOAc, washed with brine, dried over Na₂SO₄ and concentrated. The residue was purified by chromatography (ISCO, 24g silica column, using a gradient of 100% hexanes - 100% EtOAc) to give compound **27b** (230 mg, 8% yield) as the less polar isomer. nOe correlations from H1 and H2 to H3 confirmed the structure of compound **27b.**

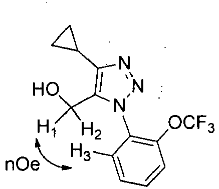

### Step4: 5-(chloromethyl)-4-cyclopropyl-1-(2-(trifluoromethoxy)phenyl)-1H-1,2,3-triazole (27c)

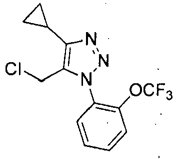

To a solution of (4-cyclopropyl-1-(2-(trifluoromethoxy)phenyl)-1H-1,2,3-triazol-5-yl)methanol (**27b**) (52 mg, 0.17 mmol) in dichloromethane (1 mL) was added thionyl chloride (0.038 µL, 0.52 mmol) at room temperature. The mixture was heated to reflux for 15 min and cooled to room temperature then concentrated *in vacuo.* Additional dichloromethane (5 mL) was added and the mixture was concentrated again. This process was repeated a third time to remove excess thionyl chloride. The crude residue was used in the next step without any further purification.

### Step5: 2-(4-(2-chloro-4-((4-cyclopropyl-1-(2-(trifluoromethoxy)phenyl)-1H-1,2,3-triazol-5-yl)methoxy)phenyl)-4-hydroxypiperidin-1-yl)isonicotinonitrile (27d)

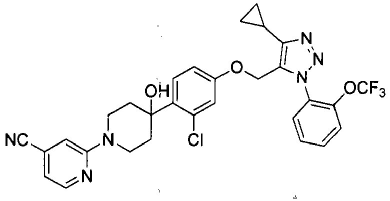

Following the procedure described in Example 3/Step 5, compound **27d** was obtained by combining 2-(4-(2-chloro-4-((4-cyclopropyl-1-(2-(trifluoromethoxy)phenyl)-1H-1,2,3-triazol-5-yl) methoxy)phenyl)-4-hydroxypiperidin-1-yl)isonicotinonitrile and 2-(4-(2-chloro-4-hydroxyphenyl)-4-hydroxypiperidin-1-yl)isonicotinonitrile in 75% yield.

### Step 6, Example 27: 2-(4-(2-chloro-4-((4-cyclopropyl-1-(2-(trifluoromethoxy)phenyl)-1H-1,2,3-triazol-5-yl)methoxy)phenyl)-4-hydroxypiperidin-1-yl)isonicotinic acid (27)

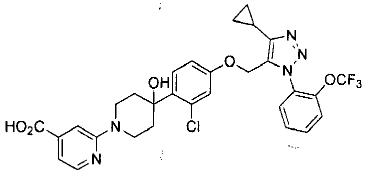

Following the procedure described in Example 1/Step 4, Example 27 was obtained from 2-(4-(2-chloro-4-((4-cyclopropyl-1-(2-(trifluoromethoxy)phenyl)-1H-1,2,3-triazol-5-yl)methoxy)phenyl)-4-hydroxypiperidin-1-yl)isonicotinonitrile (**27d**) in 62% yield. ¹H NMR (300 MHz, DMSO-*d*₆) δ 13.35 (s, 1H), 8.22 (d, J = 5.0 Hz, 1H), 7.77 - 7.51 (m, 5H), 7.23 (s, 1H), 6.97 (d, J = 5.0 Hz, 1H), 6.86 - 6.74 (m, 2H), 5.24 (s, 1H), 5.12 (s, 2H), 4.20 (d, J = 12.7 Hz, 2H), 3.24 (s, 1H), 2.42 (d, J = 9.9 Hz, 2H), 1.97 (s, 1H), 1.52 (d, J = 13.2 Hz, 2H), 1.16 (t, J = 7.1 Hz, 1H), 1.05 - 0.85 (m, 4H). MS (ESI+) m/z 616.9 (M + H).

### Example 28: 2-(4-(2-chloro-4-((5-cyclopropyl-3-(2,6-dichlorophenyl)isoxazol-4-yl)methoxy)phenyl)-4-hydroxypiperidin-1-yl)benzo[d]thiazole-6-carboxylic acid

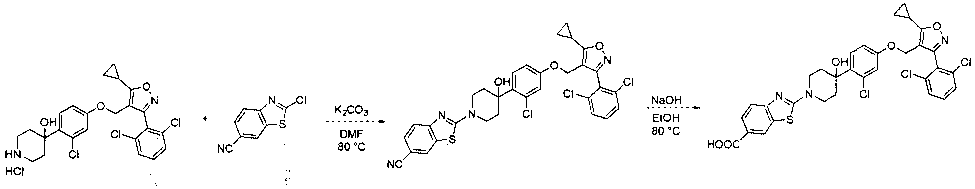

### Step 1: 2-(4-(2-chloro-4-((5-cyclopropyl-3-(2,6-dichlorophenyl)isoxazol-4-yl)methoxy)phenyl)-4-hydroxypiperidin-1-yl)benzo[d]thiazole-6-carbonitrile

To a sealed tube equipped with a magnetic stirring bar, 4-(2-chloro-4-((5-cyclopropyl-3-(2,6-dichlorophenyl)isoxazol-4-yl)methoxy)phenyl)piperidin-4-ol hydrochloride (General synthesis 4, step 2) (100 mg, 0.19 mmol), 2-chlorobenzo[d]thiazole-6-carbonitrile (54.1 mg, 0.23 mmol), potassium carbonate (234 mg, 3.8 mmol) and DMF (2 mL) were added. The tube was sealed and the mixture was heated at 80 °C for 40 minutes. Water (20 mL) was added and the resulting mixture was extracted with EtOAc (50 mL X 3), the combined organic phases were washed with brine (20 mL), dried over Na₂SO₄, filtered, and concentrated *in vacuo.* Silica gel column chromatography gave the desired product (37 mg, 30% yield).

### 2-(4-(2-chloro-4-((5-cyclopropyl-3-(2,6-dichlorophenyl)isoxazol-4-yl)methoxy)phenyl)-4-hydroxypiperidin-1-yl)benzo[d]thiazole-6-carboxylic acid

To a sealed tube equipped with a magnetic stirring bar, 2-(4-(2-chloro-4-((5-cyclopropyl-3-(2,6-dichlorophenyl)isoxazol-4-yl)methoxy)phenyl)-4-hydroxypiperidin-1-yl)benzo[d]thiazole-6-carbonitrile (37 mg, 0.06 mmol), EtOH (0.6 mL) and 30 % NaOH (0.36 mL, 2.73 mmol) were added. The tube was sealed and the mixture was heated at 80 °C overnight. After adjusting pH to ∼ 4 with 4 N HCI, ethyl acetate (200 mL) was added. The mixture was washed with water (10 mL X 2), brine (20 mL), dried over Na₂SO₄, filtered, and concentrated *in vacuo.* Silica gel column chromatography gave the desired product (7.1 mg, 19% yield). ¹H NMR (400 MHz, DMSO-*d*₆) *δ* 8.36 (d, *J* = 1.6 Hz, 1 H), 7.96 (d, *J* = 11.2 Hz, 1 H), 7.85 (dd, *J* = 11.6 z, *J* = 2.6 Hz, 1 H), 7.64 (m, 1 H), 7.46 (m, 1 H), 7.23 (m, 1 H), 6.95 (dd, *J* = 10.4 Hz, *J* = 2.2 Hz, 1 H), 6.86 (m, 1 H), 6.72 (m, 1 H), 4.89 (m, 2 H), 4.02 (dm, *J* = 13.2 Hz, 2 H), 2.43 (m, 1 H), 1.70 (m, 2 H), 1.09-1.23 (m, 6 H) ppm; MS *m*/*z* 670.19 [M + H]⁺.

### Example 29: 2-(4-(2-chloro-4-((5-cyclopropyl-3-(2,6-dichlorophenyl)isoxazol-4-yl)methoxy)phenyl)-4-hydroxypiperidin-1-yl)-6-methoxyisonicotinic acid

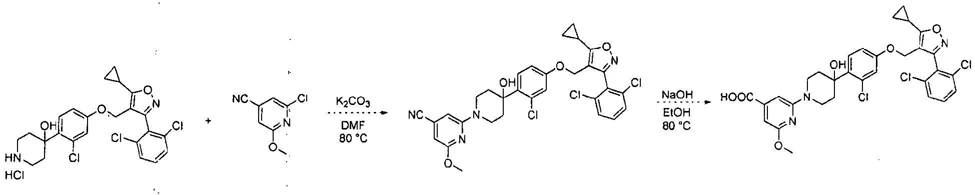

### Step 1: 2-(4-(2-chloro-4-((5-cyclopropyl-3-(2,6-dichlorophenyl)isoxazol-4-yl)methoxy)phenyl)-4-hydroxypiperidin-1-yl)-6-methoxyisonicotinonitrile

To a sealed tube equipped with a magnetic stirring bar, 4-(2-chloro-4-((5-cyclopropyl-3-(2,6-dichlorophenyl)isoxazol-4-yl)methoxy)phenyl)piperidin-4-ol hydrochloride (General synthesis 4 step 2) (95 mg, 0.18 mmol), 2-chloro-6-methoxyisonicotinonitrile (36.2 mg, 0.22 mmol), potassium carbonate (222 mg, 3.6 mmol) and DMF (2 mL) were added. The tube was sealed and the mixture was heated at 80 °C overnight. Water (20 mL) was added and the resulting mixture was extracted with EtOAc (50 mL X 3), the combined organic phases were washed with brine (20 mL), dried over Na₂SO₄, filtered, and concentrated *in vacuo.* Silica gel column chromatography gave the desired product (97 mg, 86% yield).

### Step 2: 2-(4-(2-chloro-4-((5-cyclopropyl-3-(2,6-dichlorophenyl)isoxazol-4-yl)methoxy)phenyl)-4-hydroxypiperidin-1-yl)-6-methoxyisonicotinic acid (Example 29)

To a sealed tube equipped with a magnetic stirring bar, 2-(4-(2-chloro-4-((5-cyclopropyl-3-(2,6-dichlorophenyl)isoxazol-4-yl)methoxy)phenyl)-4-hydroxypiperidin-1-yl)-6-methoxyisonicotinonitrile (90 mg, 0.14 mmol), EtOH (2.0 mL), 30 % NaOH (0.92 mL, 6.9 mmol) were added. the tube was sealed and the mixture was heated at 80 °C for 6 hours. After adjusting pH to ∼ 4 with 4 N HCl, ethyl acetate (200 mL) was added. The mixture was washed with water (10 mL X 2), brine (20 mL), dried over Na₂SO₄, filtered, and concentrated *in vacuo.* Silica gel column chromatography gave the desired product (4.6 mg, 5% yield). ¹H NMR (400 MHz, DMSO-*d*₆) *δ* 7.96 (dm, *J* = 11.2 Hz, 1 H), 7.61 (m, 2 H), 7.22 (m, 1 H), 6.94 (m, 1 H), 6.69-6.84 (m, 2 H), 6.37 (s, 1 H), 5.76 (s, 1 H), 4.88 (m, 2 H), 4.21 (m, 2 H), 3.81 (s, 3 H), 2.44 (m, 1 H), 1.56 (m, 2 H), 1.12-1.23 (m, 6 H) ppm; MS *m*/*z* 644.13 [M + H]⁺.

### Example 30: 1-(4-(1H-tetrazol-5-yl)pyridin-2-yl)-4-(2-chloro-4-((5-cyclopropyl-3-(2,6-dichlorophenyl)isoxazol-4-yl)methoxy)phenyl)piperidin-4-ol

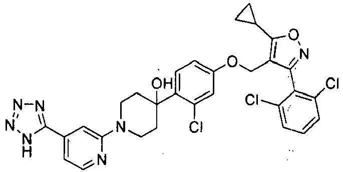

To a sealed tube equipped with a magnetic stirring bar, 2-(4-(2-chloro-4-((5-cyclopropyl-3-(2,6-dichlorophenyl)isoxazol-4-yl)methoxy)phenyl)-4-hydroxypiperidin-1-yl)isonicotinonitrile (Example 1c, 40 mg, 0.07 mmol), sodium azide (56.7 mg, 0.87 mmol), triethylamine hydrochloride (277 mg, 2.01 mmol) and toluene (2 mL) were added. The tube was sealed and heated at 115 °C for 8 hours. Some precipitation appeared. The precipitate was filtered and washed with ethyl acetate (5 mL X 4) and the collected soluble phases were treated with more ethyl acetate (180 mL), dried over Na₂SO₄, filtered, and concentrated *in vacuo.* Silica gel column chromatography gave the desired product (3.8 mg, 9 % yield). ¹H NMR (400 MHz, DMSO-*d*₆) *δ* 8.21 (d, *J* = 6.4 Hz, 1 H), 7.97 (d, *J* = 12.4 Hz, 1 H), 7.63 (m, 1 H), 7.60 (m, 1 H), 7.43 (m, 1 H), 7.19 (m, 2 H), 6.91 (d, *J* = 9.6 Hz, 1 H), 6.85 (m, 1 H), 6.69 (m, 1 H), 5.57 (s, 1 H), 4.79-4.90 (m, 2 H), 4.25 (dm, *J* = 12.1 Hz, 2 H), 2.42 (m, 1 H), 1.57 (m, 2 H), 1.11-1.24 (m, 6 H) ppm; MS *m*/*z* 637.91 [M + H]⁺.

### Example 31: 2-(4-(2-chloro-4-((4-cyclopropyl-1-(2,6-dichlorophenyl)-1H-pyrazol-5-yl)methoxy)phenyl)-4-hydroxypiperidin-1-yl)isonicotinic acid

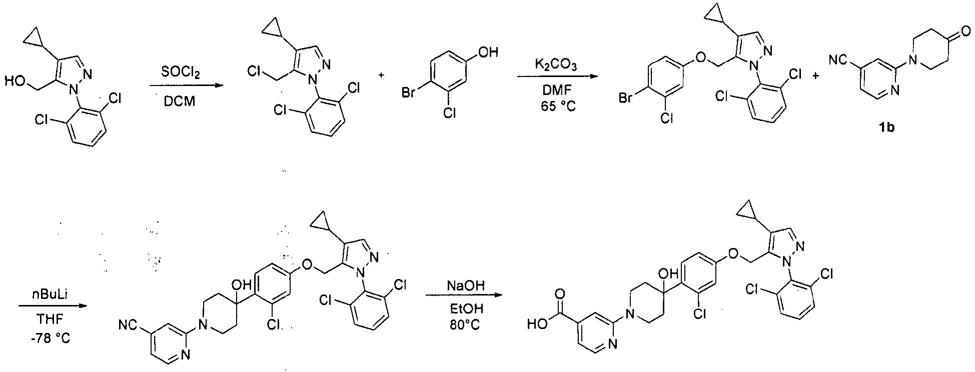

### Step 1: 5-(chloromethyl)-4-cyclopropyl-1-(2,6-dichlorophenyl)-1H-pyrazole

To a round-bottomed flask equipped with a magnetic stirring bar, (4-cyclopropyl-1-(2,6-dichlorophenyl)-1H-pyrazol-5-yl)methanol (200 mg, 0.71 mmol), DCM (3.5 mL) were added. Following the addition of thionyl chloride (588 mg, 4.94 mmol), the mixture was stirred at room temperature overnight. The mixture was coventrated *in vacuo* and to give the crude product (212.9 mg) which was used in the next step directly.

### Step 2: 5-((4-bromo-3-chlorophenoxy)methyl)-4-cyclopropyl-1-(2,6-dichlorophenyl)-1H-pyrazole

To a round-bottomed flask equipped with a magnetic stirring bar, 5-(chloromethyl)-4-cyclopropyl-1-(2,6-dichlorophenyl)-1H-pyrazole (212.9 mg, 0.71 mmol), 4-bromo-3-chlorophenol (189.8 mg, 0.92 mmol), potassium carbonate (617.3 mg, 4.47 mmol), NaI (183.6, 1.23 mmol), and DMF (4.0 mL) were added. The mixture was heated at 60 °C for 3.5 hours. Water (20 mL) was added and the resulting mixture was extracted with EtOAc (50 mL X 3), the combined organic phases were washed with brine (20 mL), dried over Na₂SO₄, filtered, and concentrated *in vacuo.* Silica gel column chromatography gave the desired product (388 mg).

### Step 3: 2-(4-(2-chloro-4-((4-cyclopropyl-1-(2,6-dichlorophenyl)-1H-pyrazol-5-yl)methoxy)phenyl)-4-hydroxypiperidin-1-yl)isonicotinonitrile

To a round bottomed flask equipped with a magnetic stirring bar and a nitrogen tee, 5-((4-bromo-3-chlorophenoxy)methyl)-4-cyclopropyl-1-(2,6-dichlorophenyl)-1H-pyrazole (100 mg, 0.21 mmol) and THF (3.9 mL) were added. The mixture was cooled to -78 °C in an acetone / liquid N₂ bath. *n*-BuLi (1.6M in hexanes, 0.17 mL, 0.28 mmol) was added dropwise, and the resulting mixture was stirred at this temperature for 30 minutes. A solution of 2-(4-oxopiperidin-1-yl)isonicotinonitrile (**1b,** 42.6 mg, 0.21 mmol) in THF (1.0 mL) was added dropwise. 30 minutes later, the reaction mixture was quenched with water (10 mL) and ethyl acetate (30 mL) and warmed to RT. More ethyl acetate (170 mL) was added and the mixture was washed with water (20 mL X 2), brine (20 mL), dried over Na₂SO₄, filtered and concentarted *in vacuo.* The residue was passed through a silica gel column to afford the desired product (14 mg, 11% yield).

### Step 4, Example 31: 2-(4-(2-chloro-4-((4-cyclopropyl-1-(2,6-dichlorophenyl)-1H-pyrazol-5-yl)methoxy)phenyl)-4-hydroxypiperidin-1-yl)isonicotinic acid

To a sealed tube equipped with a magnetic stirring bar 2-(4-(2-chloro-4-((4-cyclopropyl-1-(2,6-dichlorophenyl)-1H-pyrazol-5-yl)methoxy)phenyl)-4-hydroxypiperidin-1-yl)isonicotinonitrile (14 mg, 0.02 mmol), EtOH (0.6 mL), and 30 % NaOH (0.15 mL, 1.13 mmol) were added. The tube was sealed and the mixture was heated at 80 °C for 1 hour. After adjusting pH of the mixture to ∼ 4 with 4 N HCl, some precipitation appeared. The precipitation was filtered and washed with cold water (1 mL X 3) and dried under vacuum to afford the desired product (2.4 mg, 17% yield). ¹H NMR (400 MHz, DMSO-*d*₆) *δ* 13.4 (broad s, 1 H), 8.24 (d, *J* = 6.0 Hz, 1 H), 7.95 (d, *J* = 11.6 Hz, 1 H), 7.65 (d, *J* = 11.6 Hz, 1 H), 7.42 (s, 1 H), 7.26 (s, 1 H), 7.22 (m, 1 H), 6.96 (m, 2 H), 6.86 (s, 1 H), 6.71 (d, *J* = 11.2 Hz, 1 H), 5.61 (s, 1 H), 5.00 (m, 1 H), 4.85 (m, 1 H), 4.23 (d, *J* = 12.4 Hz, 2 H), 3.16 (s, 2 H), 1.86 (m, 1 H), 1.52 (m, 2 H), 1.22 (s, 1 H), 0.91 (m, 2 H), 0.63 (m, 2 H) ppm; MS *m*/*z* 613.3 [M + H]⁺.

### Example32: racemic 2-(3-(2-chloro-4-((5-cyclopropyl-3-(2,6-dichlorophenyl)isoxazol-4-yl)methoxy)phenyl)-3-hydroxypiperidin-1-yl)isonicotinic acid

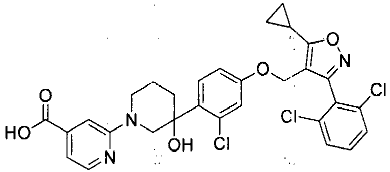

Example 32 was prepared according to the procedure described in general procedure 2 to provide the racemic title compound (18 mg, 35%). ¹H NMR (300 MHz, DMSO-*d*₆) δ 13.28 (s, 1H), 8.25 - 8.48 (m, 1H), 7.80 (m, 1H), 7.44 - 7.68 (m, 4H), 6.91 (d, *J* = 2.6 Hz, 1H), 6.79 (dd, *J* = 8.8, 2.7 Hz, 1H), 6.51 (d, *J* = 9 Hz, 1H), 5.50 (s, 1H), 4.92 (s, 2H), 3.83 (d, *J* = 11.3 Hz, 1H), 3.75 (s, 1H), 3.51 - 3.58 (m, 1H), 2.64 (q, *J* = 10.7, 9.9 Hz, 1H), 2.20 - 2.38 (m, 2H), 2.24 (d, *J* = 9.1 Hz, 2H), 1.27 - 1.06 (m, 4H); (M+H): 614.3.

### Example 33: 6-(3-(2-chloro-4-((3-(2,6-dichlorophenyl)-5-(2-fluoropropan-2-yl)isoxazol-4-yl)methoxy)phenyl)-3-hydroxypyrrolidin-1-yl)nicotinic acid

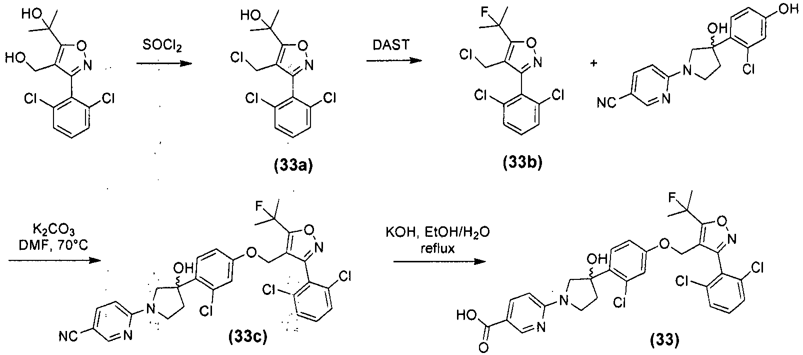

### Step1: 2-(4-(chloromethyl)-3-(2,6-dichlorophenyl)isoxazol-5-yl)propan-2-ol (33a)

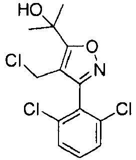

To a solution of 2-(3-(2,6-dichlorophenyl)-4-(hydroxymethyl)isoxazol-5-yl)propan-2-ol (6.8 g, 226 mmol) in dichloromethane (120 mL) was added SOCl₂ (17.2 mL, 237 mmol) at 0°C. The mixture was stirred at room temperature for 15 min, quenched with saturated aqueous NaCO₃ and extracted with EtOAc (3 x 150 mL). The combined organic layer was washed with brine, dried over Na₂SO₄, concentrated and purified by column chromatography (PE/EtOAc = 30:1) to afford 2-(4-(chloromethyl)-3-(2,6-dichlorophenyl)isoxazol-5-yl)propan-2-ol **33a.** ¹H-NMR (CDCl₃, 300 MHz): δ 7.47-7.37 (m, 3H), 4.51 (s, 2H), 2.43 (s, 1H), 1.75 (s, 6H).

### Step2: 4-(chloromethyl)-3-(2,6-dichlorophenyl)-5-(2-fluoropropan-2-yl)isoxazole (33b)

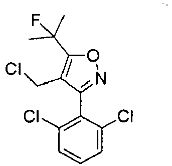

To a solution of 2-(4-(chloromethyl)-3-(2,6-dichlorophenyl)isoxazol-5-yl)propan-2-ol (**33a**, 2.80 g, 9.1 mmol) in dichloromethane (80 mL) was added DAST (1.5 mL, 11.4 mmol). The mixture was stirred at 0°C for 1.5 h, quenched with saturated aqueous NaHCO₃ and extracted with EtOAc (3 x 100 mL). The combined organic layer was washed with brine, dried over Na₂SO₄, concentrated and purified by column chromatography (PE/EtOAc = 30:1) to afford 4-(chloromethyl)-3-(2,6-dichlorophenyl)-5-(2-fluoropropan-2-yl)isoxazole **33b.** ¹H-NMR (CDCl₃, 300 MHz): δ 7.47-7.37 (m, 3H), 4.43 (s, 2H), 1.87 (d, J = 22.2 Hz, 6H). MS (ESI+) *m*/*z* 321.9 (M + H).

### Step3: 6-(3-(2-chloro-4-((3-(2,6-dichlorophenyl)-5-(2-fluoropropan-2-yl)isoxazol-4-yl)methoxy)phenyl)-3-hydroxypyrrolidin-1-yl)nicotinonitrile (33c)

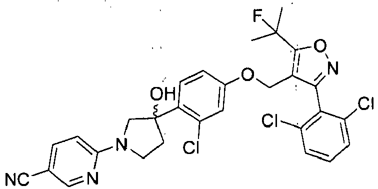

To a solution of 6-(3-(2-chloro-4-hydroxyphenyl)-3-hydroxypyrrolidin-1-yl)nicotinonitrile (100 mg, 0.32 mmol) and 4-(chloromethyl)-3-(2,6-dichlorophenyl)-5-(2-fluoropropan-2-yl)isoxazole (33b, 112 mg, 0.35 mmol) in DMF (10 mL) was added K₂CO₃ (48 mg, 0.35 mmol) at room temperature. The mixture was stirred at 70°C overnight, quenched with water (30 mL) and extracted with EtOAc (3 x 50 mL). The combined organic layer was washed with brine (100 mL), dried over Na₂SO₄ and concentrated in vacuum to afford crude 6-(3-(2-chloro-4-((3-(2,6-dichlorophenyl)-5-(2-fluoropropan-2-yl)isoxazol-4-yl)methoxy)phenyl)-3-hydroxypyrrolidin-1-yl)nicotinonitrile **33c** (191 mg) MS (ESI+) *m*/*z* 600.9 (M + H).

### Step 4, Example 33: racemic 6-(3-(2-chloro-4-((3-(2,6-dichlorophenyl)-5-(2-fluoropropan-2 yl)isoxazol-4-yl)methoxy)phenyl)-3-hydroxypyrrolidin-1-yl)nicotinic acid (33)

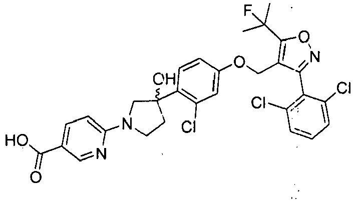

To a solution of 6-(3-(2-chloro-4-((3-(2,6-dichlorophenyl)-5-(2-fluoropropan-2-yl)isoxazol-4-yl)methoxy)phenyl)-3-hydroxypyrrolidin-1-yl)nicotinonitrile (**33c**, 191 mg, 0.32 mmol) in EtOH (10 mL) and water (5 mL) was added KOH (179 mg, 3.2 mmol) at room temperature. The reaction was stirred at reflux overnight, concentrated in vacuum and acidified to pH = 6 with 1N HCl, filtered and purified by preparative-HPLC to afford racemic 6-(3-(2-chloro-4-((3-(2,6-dichlorophenyl)-5-(2-fluoropropan-2-yl)isoxazol-4-yl)methoxy)phenyl)-3-hydroxypyrrolidin-1-yl)nicotinic acid **33** (34 mg, 17%). ¹H NMR (CD₃OD, 300 MHz): δ 8.69 (d, J = 1.8 Hz, 1H), 8.05 (dd, J = 2.1, 8.7 Hz, 1H), 7.56-7.45 (m, 4H), 6.77 (d, J = 2.4 Hz, 1H), 6.71 (dd, J = 2.6, 9.0 Hz, 1H), 6.53 (d, J = 9.0 Hz, 1H), 4.99 (s, 2H), 4.04-3.95 (m, 2H), 3.79-3.70 (m, 2H), 2.85-2.77 (m, 1H), 2.37-2.31 (m, 1H), 1.88 (d, J = 22.2 Hz, 6H). MS (ESI+) *m*/*z* 620.1 (M + H).

### Example 34: racemic 6-(3-(2-chloro-4-((3-(2,6-dichlorophenyl)-5-(2-hydroxypropan-2-yl)isoxazol-4-yl)methoxy)phenyl)-3-hydroxypyrrolidin-1-yl)nicotinic acid

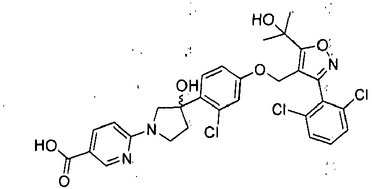

This compound was synthesized according to the procedure described for Example 33, using 2-(4-(chloromethyl)-3-(2,6-dichlorophenyl)isoxazol-5-yl)propan-2-ol **33a** as starting material. ¹H NMR (CD₃OD, 300 MHz): δ 8.68 (d, J = 2.1 Hz, 1H), 8.05 (dd, J = 2.1, 9.0 Hz, 1H), 7.55-7.44 (m, 4H), 6.78 (d, J = 2.4 Hz, 1H), 6.71 (dd, J = 2.4, 9.0 Hz, 1H), 6.55 (d, J = 9.0 Hz, 1H), 5.14 (s, 2H), 4.06-3.94 (m, 2H), 3.78-3.70 (m, 2H), 2.85-2.77 (m, 1H), 2.37-2.31 (m, 1H), 1.69 (s, 6H). MS (ESI+) *m*/*z* 618.0 (M + H).

### Example 35: racemic 6-(3-(2-chloro-4-((4-cyclopropyl-1-(2,6-dichlorophenyl)-1H-1,2,3-triazol-5-yl)methoxy)phenyl)-3-hydroxypyrrolidin-1-yl)nicotinic acid

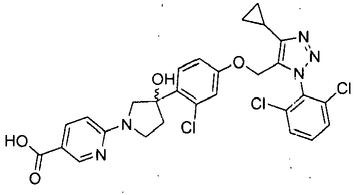

This compound was synthesized according to the procedure described for Example 33, using appropriate starting materials. ¹H NMR (CD₃OD, 300 MHz): δ 8.68 (d, J = 2.1 Hz, 1H), 8.05 (dd, J = 2.1, 9.0 Hz, 1H), 7.66-7.57 (m, 4H), 6.91 (d, J = 2.4 Hz, 1H), 6.81 (dd, J = 2.7, 9.0 Hz, 1H), 6.51 (d, J = 8.7 Hz, 1H), 5.16 (s, 2H), 4.04-3.94 (m, 2H), 3.77-3.69 (m, 2H), 2.87-2.76 (m, 1H), 2.36-2.30 (m, 1H), 2.15-2.09 (m, 1H), 1.11-1.05 (m, 4H). MS (ESI+) *m*/*z* 600.0 (M + H).

### Example 36: racemic 6-(3-2-chloro-4-((5-cyclopropyl-3-((2,6-dimethylphenoxy)methyl)isoxazol-4-yl)methoxy)phenyl)-3-hydroxypyrrolidin-1-yl)nicotinic acid

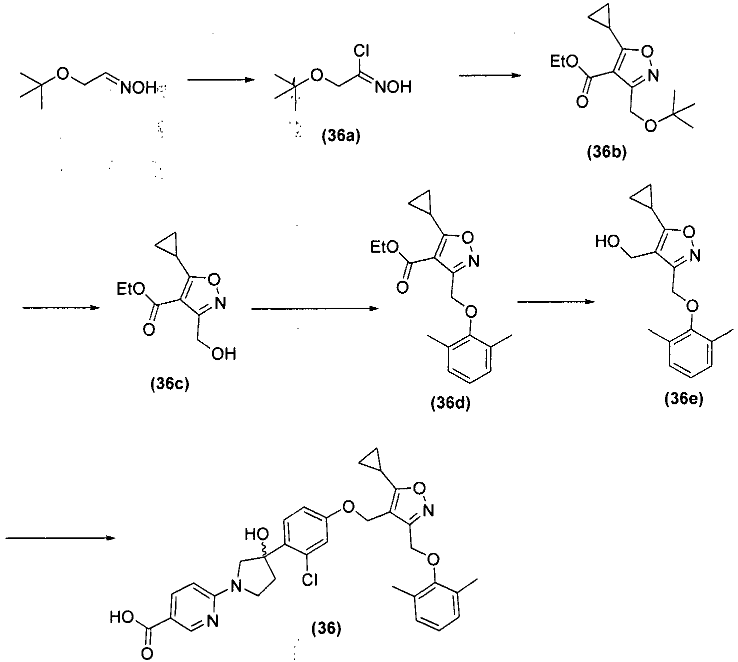

### Step1: 2-(tert-butoxy)-N-hydroxyacetimidoyl chloride (36a)

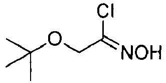

To a solution of 2-(*tert*-butoxy)acetaldehyde oxime (24.1 g, 184 mmol; prepared as described in WO2009/005998) in DMF (600 mL) was added N-chlorosuccinimide (23.7 g, 184 mmol) at 0°C. The mixture was stirred for 1 h, poured into Et₂O (800 mL) and washed with brine (450 mL). The organic layer was dried over MgSO₄ and concentrated to give the crude 2-(tert-butoxy)-N-hydroxyacetimidoyl chloride **36a,** which was used directly in the next step.

### Step2: Ethyl 3-(tert-butoxymethyl)-5-cyclopropylisoxazole-4-carboxylate (36b)

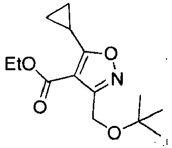

To a solution of ethyl 3-cyclopropyl-3-oxopropanoate (31.6 g, 203 mmol) in THF (600 mL) was added a solution of NaOCH₃ (0.5 M, 10.9 g, 203 mmol) in MeOH at 0°C. After stirring for 5 min, a solution of 2-(tert-butoxy)-N-hydroxyacetimidoyl chloride (**36a**, 27.9 g, 169 mmol) in THF (200 mL) was added dropwise. The mixture was allowed to warm to room temperature and stirred overnight, poured into Et₂O (800 mL), washed with brine (450 mL) and concentrated to give the crude ethyl 3-(tert-butoxymethyl)-5-cyclopropylisoxazole-4-carboxylate **36b,** which was used directly in the next step.

### Step3: Ethyl 5-cyclopropyl-3-(hydroxymethyl)isoxazole-4-carboxylate (36c)

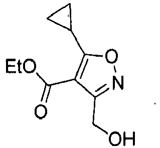

To a solution of ethyl 3-(tert-butoxymethyl)-5-cyclopropylisoxazole-4-carboxylate (**36b,** 38.4 g, 144 mmol) in dichloromethane (600 mL) was added TFA (100 mL) at room temperature. The mixture was stirred at room temperature for 2 h, concentrated and adjusted to basic pH with aq. NaHCO₃. The mixture was extracted with EtOAc (3 x 300 mL). The combined organic layer was washed with brine (400 mL), dried over MgSO₄, concentrated and purified by column chromatography (PE/EtOAc = 10:1) to afford ethyl 5-cyclopropyl-3-(hydroxymethyl)isoxazole-4-carboxylate **36c.** ¹H NMR (300 MHz, DMSO-d₆): δ 4.64 (s, 2H), 4.26 (q, J = 7.2 Hz, 2H), 2.79-2.70 (m, 1H), 1.29 (t, J = 7.2 Hz, 3H), 1.23-1.13 (m, 4H).

### Step4: Ethyl 5-cyclopropyl-3-((2,6-dimethylphenoxy)methyl)isoxazole-4-carboxylate (36d)

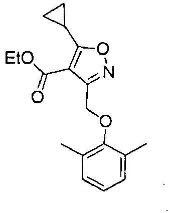

To a solution of ethyl 5-cyclopropyl-3-(hydroxymethyl)isoxazole-4-carboxylate (**36c**, 16.1 g, 76.3 mmol), 2,6-dimethylphenol (9.3 g, 76.3 mmol) and PPh₃ (20 g, 76.3 mmol) in toluene (500 mL) was added DIAD (15.4 g, 76.3 mmol) at 0°C. The mixture was stirred at 90°C for 2 h, cooled, concentrated and purified by column chromatography (PE/EtOAc = 15:1) to afford ethyl 5-cyclopropyl-3-((2,6-dimethylphenoxy)methyl)isoxazole-4-carboxylate **36d.** ¹H-NMR (300 MHz, DMSO-d₆): δ 7.03 (d, J = 7.8, Hz, 2H), 6.96-6.87 (m, 1H), 5.03 (s, 2H), 4.25 (q, J = 7.2 Hz, 2H), 2.82-2.76 (m, 1H), 2.18 (s, 6H), 2.14 (s, 3H), 1.28-1.17 (m, 4H).

### Step5: (5-cyclopropyl-3-((2,6-dimethylphenoxy)methyl)isoxazol-4-yl)methanol (36e)

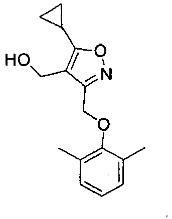

To a solution of LiAlH₄ (2.9 g, 77.6 mmol) in THF (250 mL) was added ethyl 5-cyclopropyl-3-((2,6-dimethylphenoxy)methyl)isoxazole-4-carboxylate (**36d**, 16.3 g, 51.7 mmol) at 0°C. The mixture was stirred at room temperature for 1 h, diluted with water (100 mL) and extracted with EtOAc (3 x 100 mL). The combined organic layer was washed with brine (200 mL), dried over MgSO₄, concentrated and purified by column chromatography (PE/EtOAc = 8:1) to afford (5-cyclopropyl-3-((2,6-dimethylphenoxy)methyl)isoxazol-4-yl)methanol **36e.** ¹H-NMR (300 MHz, DMSO-d₆): δ 7.04 (d, J = 7.5, Hz, 2H), 6.97-6.92 (m, 1H), 5.07 (br s, 1H), 4.85 (s, 2H), 4.46 (s, 2H), 2.30-2.26 (m, 1H), 2.22 (s, 6H), 1.10-0.96 (m, 4H). MS (ESI+) *m*/*z* 256.1 (M - H₂O + H).

### Step 6, Example 36: racemic 6-(3-(2-chloro-4-((5-cyclopropyl-3-((2,6-dimethylphenoxy)methyl)isoxazol-4-yl)methoxy)phenyl)-3-hydroxypyrrolidin-1-yl)nicotinic acid (36)

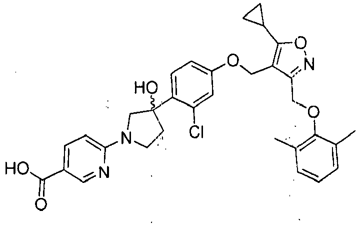

This compound was synthesized according to the procedure as described for Example 33, using (5-cyclopropyl-3-((2,6-dimethylphenoxy)methyl)isoxazol-4-yl)methanol **36e** as starting material. ¹H NMR (CD₃OD, 300 MHz): δ 8.69 (d, J = 1.5 Hz, 1H), 8.05 (dd, J = 1.5, 6.9 Hz, 1H), 7.66 (d, J = 6.6 Hz, 1H), 7.12 (d, J = 1.8 Hz, 1H), 6.99-6.89 (m, 4H), 6.56 (d, J = 6.9 Hz, 1H), 5.06 (s, 2H), 4.91 (s, 2H), 4.14-4.00 (m, 2H), 3.79-3.71 (m, 2H), 2.88-2.81 (m, 1H), 2.43-2.39 (m, 1H), 2.28-2.24 (1H), 2.18 (s, 6H), 1.16-1.09 (m, 4H). MS (ESI+) *m*/*z* 589.7 (M + H).

### Example 37: racemic 6-(3-(2-chloro-4-((4-(2,6-dichlorophenyl)-1-isopropyl-1H-1,2,3-triazol-5-yl)methoxy)phenyl)-3-hydroxypyrrolidin-1-yl)nicotinic acid (35)

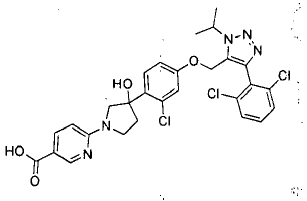

This analog was synthesized according to the procedure as described for Example 33, using (4-(2,6-dichlorophenyl)-1-isopropyl-1H-1,2,3-triazol-5-yl)methanol (prepared as described in WO2011/020615) as starting material. ¹H NMR (CD₃OD, 300 MHz): δ 8.68 (d, J = 2.1 Hz, 1H), 8.04 (dd, J = 2.1, 8.7 Hz, 1H), 7.60-7.43 (m, 4H), 6.88 (d, J = 2.4 Hz, 1H), 6.80 (dd, J = 2.4, 8.7 Hz, 1H), 6.55 (d, J = 9.0 Hz, 1H), 5.19 (s, 2H), 5.00-4.92 (m, 1H), 4.01 (br s, 2H), 3.81-3.70 (m, 2H), 2.89-2.77 (m, 1H), 2.37-2.30 (m, 1H), 1.70 (d, J = 6.9 Hz, 6H). MS (ESI+) *m*/*z* 601.6 (M + H).

### Example 38: racemic 6-(3-(2-chloro-4-((5-cyclopropyl-3-(2,6-dichlorophenethyl)-2-oxo-2,3-dihydrooxazol-4-yl)methoxy)phenyl)-3-hydroxypyrrolidin-1-yl)nicotinic acid (36)

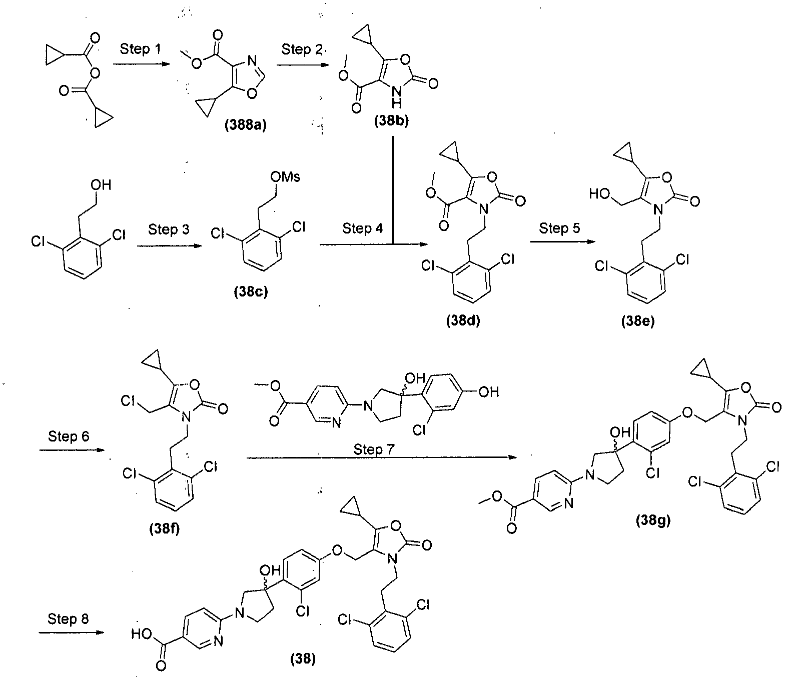

### Step1: methyl 5-cyclopropyloxazole-4-carboxylate (38a)

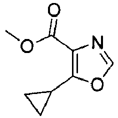

To a solution of methyl 2-isocyanoacetate (72.7 g, 729 mmol) and DBU (111 g, 729 mmol) in THF (1 L) was added a solution of cyclopropanecarboxylic anhydride (112 g, 729 mmol) in THF (100 mL) portionwise at 5°C. The mixture was stirred at rt overnight, concentrated and purified by flash chromatography (PE/EtOAc = 5:1) to afford methyl 5-cyclopropyloxazole-4-carboxylate **38a.** ¹H-NMR (CDCl₃, 300 MHz): δ 1.04-1.18 (m, 4H), 2.74-2.79 (m, 1H), 3.91 (s, 3H), 7.60 (s, 1H).

### Step2: methyl 5-cyclopropyl-2-oxo-2,3-dihydrooxazole-4-carboxylate (38b)

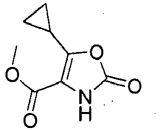

A solution of methyl 5-cyclopropyloxazole-4-carboxylate (**36a**, 36.4 g, 218 mmol) and TsOH·H₂O (82.9 g, 436 mmol) in MeOH (600 mL) was heated to reflux overnight. The mixture was cooled to rt and concentrated in vacuo. The residue was triturated with Et₂O and filtered to afford the crude methyl 2-amino-3-cyclopropyl-3-oxopropanoate (62.8 g, 191 mmol) which was dissolved in THF (1.5 L) and TEA (77.2 g, 764 mmol). Then triphosgene (19.9 g, 67 mmol) was added to the mixture at -50°C for 1 h. The solution was diluted with Et₂O (500 mL) and saturated aqueous NH₄Cl (300 mL) was added. The aqueous phase was separated and extracted with Et₂O (3 x 1 L). The combined organic extracts were washed with brine (500 mL), dried over Na₂SO₄, concentrated and purified by flash chromatography (PE/EtOAc = 5:1) to afford methyl 5-cyclopropyl-2-oxo-2,3-dihydrooxazole-4-carboxylate **38b.** ¹H-NMR (CDCl₃, 300 MHz): δ 0.99-1.11 (m, 4H), 2.41-2.50 (m, 1H), 3.84 (s, 3H), 8.57 (s, 1H).

### Step3: 2,6-dichlorophenethyl methanesulfonate (38c)

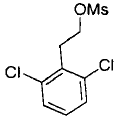

To a solution of 2-(2,6-dichlorophenyl)ethanol (37.3 g, 195 mmol) and TEA (32.7 g, 235 mmol) in DCM (700 mL) was added MsCI (26.9 g, 235 mmol) dropwise at 0°C. After addition, the solution was stirred at rt overnight, diluted with water (200 mL) and extracted with DCM (3 x 400 mL). The combined organic layer was dried over Na₂SO₄, filtered, concentrated and purified by flash chromatography (PE/EtOAc = 5:1) to afford 2,6-dichlorophenethyl methanesulfonate 38c. ¹H-NMR (300 MHz, CDCl₃): δ 2.95 (s, 3H), 3.43 (t, J = 7.5 Hz, 2H), 4.41 (t, J = 7.5 Hz, 2H), 7.12-7.17 (m, 1H), 7.31 (d, J = 8.4 Hz, 2H).

### Step4: methyl 5-cyclopropyl-3-(2,6-dichlorophenethyl)-2-oxo-2,3-dihydrooxazole-4-carboxylate (38d)

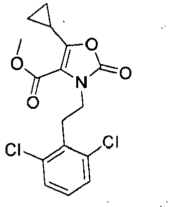

To a solution of methyl 5-cyclopropyl-2-oxo-2,3-dihydrooxazole-4-carboxylate (**38b**, 23.5 g, 129 mmol) in DMF (800 mL) was added NaH (5.7 g, 142 mmol; 60% in mineral oil) at 0°C under nitrogen. The mixture was stirred for 15 min, then a solution of 2,6-dichlorophenethyl methanesulfonate (**38c**, 41.5 g, 154 mmol) in DMF (400 mL) was added dropwise at 0°C. After addition, the mixture was stirred at 100°C overnight, cooled, diluted with water (1500 mL) and extracted with EtOAc (3 x 700 mL). The combined organic layer was washed with water (2 x 200 mL) and brine (300 mL), dried over Na₂SO₄, filtered and concentrated in vacuum. The residue was washed with PE/EtOAc (5:1) to afford methyl 5-cyclopropyl-3-(2,6-dichlorophenethyl)-2-oxo-2,3-dihydrooxazole-4-carboxylate **38d.** ¹H-NMR (300 MHz, CDCl₃): δ 0.97-1.08 (m, 4H), 2.44-2.49 (m, 1H), 3.31 (t, J = 4.8 Hz, 2H). 3.73 (s, 3H), 4.26 (t, J = 4.8 Hz, 2H), 7.08-7.12 (m, 1H), 7.26-7.28 (m, 2H).

### Step5: 5-cyclopropyl-3-(2,6-dichlorophenethyl)-4-(hydroxymethyl)oxazol-2(3H)-one (38e)

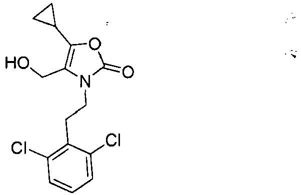

To a solution of methyl 5-cyclopropyl-3-(2,6-dichlorophenethyl)-2-oxo-2,3-dihydrooxazole-4-carboxylate (**38d**, 13.9 g, 39 mmol) in THF (400 mL) was added a solution of LiAlH₄ (16.3 mL, 39 mmol) in THF at 0°C under nitrogen. After addition, the solution was stirred at 0°C for 30 min, sequentially diluted with H₂O (2 mL), 1M NaOH (2 mL) and H₂O (6 mL), flitered and concentrated in vacuum. The residue was washed with PE/EtOAc (2:1) to afford 5-cyclopropyl-3-(2,6-dichlorophenethyl)-4-(hydroxymethyl)oxazol-2(3*H*)-one **38e.** ¹H-NMR (CD₃OD, 300 MHz): δ 0.73-0.77 (m, 2H), 0.83-0.88 (m, 2H), 1.75-1.79 (m, 1H), 3.30-3.38 (m, 2H), 3.95 (t, J = 6.6 Hz, 2H). 4.10 (s, 2H), 7.20-7.25 (m, 1H), 7.37 (d, J = 8.1 Hz, 2H), hydroxyl proton not resolved. LC/MS (ESI): m/z 328.0 (M+H)⁺.

### Step6: (prophetic example) 4-(chloromethyl)-5-cyclopropyl-3-(2,6-dichlorophenethyl)oxazol-2(3H)-one (38f)

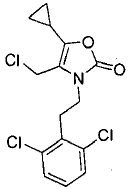

If one were to treat 5-cyclopropyl-3-(2,6-dichlorophenethyl)-4-(hydroxymethyl)oxazol-2(3*H*)-one **38e** similar as described above for Example 33, Step 1, one would obtain 4-(chloromethyl)-5-cyclopropyl-3-(2,6-dichlorophenethyl)oxazol-2(3H)-one.

### Step7: methyl 6-(3-(2-chloro-4-((5-cyclopropyl-3-(2,6-dichlorophenethyl)-2-oxo-2,3-dihydrooxazol-4-yl)methoxy)phenyl)-3-hydroxypyrrolidin-1-yl)nicotinate (36g)

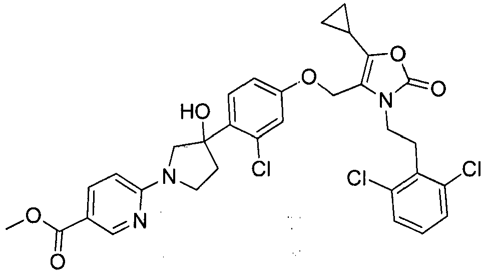

If one were to treat 4-(chloromethyl)-5-cyclopropyl-3-(2,6-dichlorophenethyl)oxazol-2(3H)-one **38f** and methyl 6-(3-(2-chloro-4-hydroxyphenyl)-3-hydroxypyrrolidin-1-yl)nicotinate similar as described above, one would obtain methyl 6-(3-(2-chloro-4-((5-cyclopropyl-3-(2,6-dichlorophenethyl)-2-oxo-2,3-dihydrooxazol-4-yl)methoxy)phenyl)-3-hydroxypyrrolidin-1-yl)nicotinate **38g.**

### Step8, Example 38: racemic 6-(3-(2-chloro-4-((5-cyclopropyl-3-(2,6-dichlorophenethyl)-2-oxo-2,3-dihydrooxazol-4-yl)methoxy)phenyl)-3-hydroxypyrrolidin-1-yl)nicotinic acid (38)

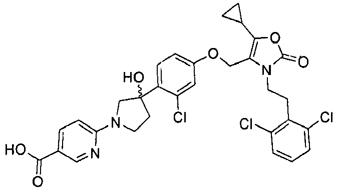

If one were to treat methyl 6-(3-(2-chloro-4-((5-cyclopropyl-3-(2,6-dichlorophenethyl)-2-oxo-2,3-dihydrooxazol-4-yl)methoxy)phenyl)-3-hydroxypyrrolidin-1-yl)nicotinate **38g** similar as described above, one would obtain 6-(3-(2-chloro-4-((5-cyclopropyl-3-(2,6-dichlorophenethyl)-2-oxo-2,3-dihydrooxazol-4-yl)methoxy)phenyl)-3-hydroxypyrrolidin-1-yl)nicotinic acid **38.**

### Example 39: 5-(4-(2-Chloro-4-((5-cyclopropyl-3-(2,6-dichlorophenyl)isoxazol-4-yl)methoxy)phenyl)-4-hydroxypiperidin-1-yl)-1-isopropyl-1H-pyrazole-3-carboxylic acid (39)

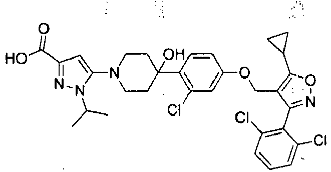

### Step 1: (Z)-Ethyl 2-hydroxy-4-oxo-4-(1,4-dioxa-8-azaspiro[4.5]decan-8-yl)but-2-enoate (39a)

To a suspension of 1-(1,4-dioxa-8-azaspiro[4.5]decan-8-yl)ethanone (4.0 g, 21.6 mmol) in dry THF (100 ml) was added *t*-BuLi (25mL, 1.3M, 32.4mmol) dropwise at -78°C. Then the reaction mixture was allowed to warm to rt, stirred for another 5h, quenched with saturated aq. NH₄Cl. The mixture was extracted with EtOAc (100 mL x 2), the organic layer was washed with H₂O (80 mL) and brine (80 mL), dried with Na₂SO₄, filtered, concentrated and the residue was purified by silica gel column (PE:EtOAc = 5:1 to 2:1) to give the title compound (4.6 g).

### Step 2: Ethyl 1-isopropyl-5-(1,4-dioxa-8-azaspiro[4.5]decan-8-yl)-1H-pyrazole-3-carboxylate (39b)

To a suspension of intermediate **39a** (2.0 g, 7.0 mmol), isopropylhydrazine monohydrochloride (1.0 g, 9.1mmol) and pyridine (3mL) in THF (40 ml) was added Lawesson's reagent (5.7g, 14 mmol) portionwise at rt. The resulting mixture was heated to 60°C and stirred for 16 h. Then EtOAc (50 mL) was added and the mixture was washed with sat. NaHCO₃ (50 m x 2), aq. HCI (1M, 50 mL) and brine (50 mL). The organic phase was dried with Na₂SO₄, filtered, concentrated and the residue was purified by silica gel column (PE:EA = 5:1 to 2:1) to give the title compound (0.7 g).

### Step 3: Ethyl 1-isopropyl-5-(4-oxopiperidin-1-yl)-1H-pyrazole-3-carboxyate (39c)

A suspension of intermediate **39b** (0.70 g, 2.3 mmol, crude) in THF (10 ml) and H₂SO₄ (10%, 10 mL) was stirred for 16 h. The mixture was diluted with EtOAc (50 mL), washed with sat. NaHCO₃ (50 mL x 2), aq. HCI (1M, 50 mL) and brine (50 mL). The organic phase was dried with Na₂SO₄, filtered, concentrated and the residue was purified by silica gel column (PE:EA = 5:1 to 2:1) to give the title compound.(0.61 g).

### Step 4: Ethyl 5-(4-(4-((tert-butyldimethylsilyl)oxy)-2-chlorophenyl)-4-hydroxypiperidin-1-yl)-1-isopropyl-1H-pyrazole-3-carboxylate (39d)

To a round bottom flask was added LiCI (265 mg, 6.3 mmol), dry THF (10 ml) at rt and *i*-PrMgCl (1.9 ml, 2.0 M, 3.8 mmol) under N₂. The mixture was stirred at rt for 10 min, then a solution of (4-bromo-3-chlorophenoxy)(tert-butyl)dimethylsilane (1.1 g, 3.2 mol) in dry THF (2 ml) was added dropwise and stirring was continued at rt for another 1 hour. After that, the above reaction mixture was added into a solution of intermediate **39c** (0.8 g, 2.9 mmol) in dry THF (10 ml) under N₂, and the mixture was stirred at rt for one hour. The reaction mixture was quenched with H₂O (20 mL), extracted with EtOAc (30 mL x 3). The organic layer was washed with brine (20 mL), dried with Na₂SO₄, filtered and purified by column chromatography on silica gel (EA/PE = 1/20) to give the title compound (350 mg).

### Step 5: Ethyl 5-(4-(2-chloro-4-hydroxyphenyl)-4-hydroxypiperidin-1-yl)-1-isopropyl-1H-pyrazole-3-carboxylate (39e)

To a suspension of **39d** (0.3 g, 0.67 mmol) in THF (5 ml) was added TBAF (277 mg, 1.01 mmol) at rt and the mixture was stirred at rt for 30 min. Then the mixture was quenched with water (20 mL), extracted with EtOAc (20 mL x 3). The organic phase was washed with brine (20 mL), dried with Na₂SO₄, filtered and concentrate to give the title compound, which was used directly in next step without further purification (0.23 g).

### Step 6: Ethyl 5-(4-(2-chloro-4-((5-cyclopropyl-3-(2,6-dichlorophenyl)isoxazol-4-yl)methoxy)phenyl)-4-hydroxypiperidin-1-yl)-1-isopropyl-1H-pyrazole-3-carboxylate (39f)

To a suspension of intermediate **39e** (230 mg, 0.56 mol), (5-cyclopropyl-3-(2,6-dichlorophenyl)isoxazol-4-yl)methanol (158 mg, 0.56 mol) and PPh₃ (239 mg, 1.1 mmol) in toluene (10 ml) was added DIAD (226 mg, 1.1 mmol) dropwise at 0°C. The resulting mixture was stirred at rt for 4 h. The reaction mixture was diluted with H₂O, extracted with EtOAc (20 mL x 3) and the organic layers were concentrated to dryness. The residue was and purified by preparative TLC (EtOAc/PE=1/1) to give the title compound (220 mg).

### Step 7, Example 39: 5-(4-(2-Chloro-4-((5-cyclopropyl-3-(2,6-dichlorophenyl)isoxazol-4-yl)methoxy)phenyl)-4-hydroxypiperidin-1-yl)-1-isopropyl-1H-pyrazole-3-carboxylic acid (39)

A suspension of intermediate **39f** (180 mg, 0.267 mmol) in MeOH (10 mL) and aq. NaOH (10%, 10 mL) was stirred at 30°C for 4h. The mixture was concentrated under reduced pressure, HCI (1M) was added until pH = 2∼3 and the resulting precipitate was filtered off and dried under vaccum to give Example **39** (100 mg). ¹H-NMR (400 MHz, CD₃Cl): δ ppm 7.43-7.39 (m, 3H), 7.36-7.31 (m, 1H), 6.83 (d, J = 2.0 Hz, 1H), 6.72 (dd, J = 8.8 Hz, 2.0 Hz, 1H), 6.51 (s, 1H), 4.81 (s, 2H), 4.71-4.65 (m, 1H), 3.29-3.25 (m, 2H), 2.98-2.93 (m, 2H), 2.2.45-2.37 (m, 2H), 2.17-2.05 (m, 3H), 1.47 (d, J = 6.0 Hz, 6H), 1.32-1.25 (m, 2H), 1.18-1.12 (m, 2H). MS-ESI: m/z 645.3/647.3 [M+H]⁺ 667.3/669.3 [M+Na]⁺.

### Assays

### FRET activity assay

Determination of a ligand mediated cofactor peptide interaction to quantify ligand binding to the nuclear receptor FXR was performed as follows: Preparation of human FXR alpha ligand binding domain: The human FXRalpha LBD was expressed in *E. coli* strain BL21(DE3) as an N-terminally GST tagged fusion protein. The DNA encoding the FXR ligand binding domain was cloned into vector pDEST15 (Invitrogen). Expression was under control of an IPTG inducible T7 promoter. The amino acid boundaries of the ligand binding domain were amino acids 187-472 of Database entry NM_005123 (RefSeq). Expression and purification of the FXR-LBD: An overnight preculture of a transformed *E.coli* strain was diluted 1:20 in LB-Ampicillin medium and grown at 30°C to an optical density of OD₆₀₀=0.4-0.6. Gene expression was then induced by addition of 0.5 mM IPTG. Cells were incubated an additional 6 h at 30°C, 180 rpm. Cells were collected by centrifugation (7000 x g, 7 min, rt). Per liter of original cell culture, cells were resuspended in 10 mL lysis buffer (50 mM Glucose, 50 mM Tris pH 7.9, 1 mM EDTA and 4 mg/mL lysozyme) and left on ice for 30 min. Cells were then subjected to sonication and cell debris removed via centrifugation (22000 x g, 30 min, 4°C). Per 10 mL of supernatant 0.5 mL prewashed Glutathione 4B sepharose slurry (Qiagen) was added and the suspension kept slowly rotating for 1 h at 4°C. Glutathione 4B sepharose beads were pelleted by centrifugation (2000 x g, 15 sec, 4°C) and washed twice in wash buffer (25 mM Tris, 50 mM KCI, 4 mM MgCl₂ and 1M NaCl). The pellet was resuspended in 3 mL elution buffer per liter of original culture (elution buffer: 20 mM Tris, 60 mM KCI, 5 mM MgCl₂ and 80 mM glutathione added immediately prior to use as powder). The suspension was left rotating for 15 min at 4°C, the beads pelleted and eluted again with half the volume of elution buffer than the first time. The eluates were pooled and dialysed overnight in 20 mM Hepes buffer (pH 7.5) containing 60 mM KCI, 5 mM MgCl₂ as well as 1 mM dithiothreitol and 10% (v/v) glycerol. The protein was analysed by SDS-Page.

The method measures the ability of putative ligands to modulate the interaction between the purified bacterial expressed FXR ligand binding domain (LBD) and a synthetic biotinylated peptide based on residues 676-700 of SRC-1 (LCD2, 676-700). The sequence of the peptide used was B-CPSSHSSLTERHKILHRLLQEGSPS-COOH where the N-terminus was biotinylated (B). The ligand binding domain (LBD) of FXR was expressed as fusion protein with GST in BL-21 cells using the vector pDEST15. Cells were lysed by sonication, and the fusion proteins purified over glutathione sepharose (Pharmacia) according to the manufacturers instructions. For screening of compounds for their influence on the FXR-peptide interaction, the Perkin Elmer LANCE technology was applied. This method relies on the binding dependent energy transfer from a donor to an acceptor fluorophor attached to the binding partner of interest. For ease of handling and reduction of background from compound fluorescence LANCE technology makes use of generic fluorophore labels and time resolved detection Assays were done in a final volume of 25 µL in a 384 well plate, in a Tris-based buffer (20 mM Tris-HCI pH 7.5; 60 mM KCI, 5 mM MgCl₂; 35 ng/µL BSA), containing 20-60 ng/well recombinantly expressed FXR-LBD fused to GST, 200-600 nM N-terminally biotinylated peptide, representing SRC1 aminoacids 676-700, 200 ng/well Streptavidin-xlAPC conjugate(Prozyme) and 6-10 ng/well Eu W1024 - antiGST (Perkin Elmer). DMSO content of the samples was kept at 1%. After generation of the assay mix and diluting the potentially FXR modulating ligands, the assay was equilibrated for 1 h in the dark at rt in FIA-plates black 384 well (Greiner). The LANCE signal was detected by a Perkin Elmer VICTOR2VTM Multilabel Counter. The results were visualized by plotting the ratio between the emitted light at 665 and 615 nm. A basal level of FXR-peptide formation is observed in the absence of added ligand. Ligands that promote the complex formation induce a concentration-dependent increase in time-resolved fluorescent signal. Compounds which bind equally well to both monomeric FXR and to the FXR-peptide complex would be expected to give no change in signal, whereas ligands which bind preferentially to the monomeric receptor would be expected to induce a concentration-dependent decrease in the observed signal.

To assess the agonistic potential of the compounds, EC₅₀-values were determined for example compounds as listed below in Table 1 (FRET EC₅₀).

### Mammalian one hybrid (M1H) assay

Determination of a ligand mediated Gal4 promoter driven transactivation to quantify ligand binding mediated activation of FXR was performed as follows: The cDNA part encoding the FXR ligand binding domain was cloned into vector pCMV-BD (Stratagene) as a fusion to the yeast GAL4 DNA binding domain under the control of the CMV promoter. The amino acid boundaries of the ligand binding domain were amino acids 187-472 of Database entry NM_005123 (RefSeq). The plasmid pFR-Luc (Stratagene) was used as the reporter plasmid, containing a synthetic promoter with five tandem repeats of the yeast GAL4 binding sites, driving the expression of the Photinus pyralis (American firefly) luciferase gene as the reporter gene. In order to improve experimental accuracy the plasmid pRL-CMV (Promega) was cotransfected. pRL-CMV contains the constitutive CMV promoter, controlling the expression of the Renilla reniformis luciferase. All Gal4 reporter gene assays were done in HEK293 cells (obtained from DSMZ, Braunschweig, Germany) grown in MEM with L-Glutamine and Earle's BSS supplemented with 10% fetal bovine serum, 0.1 mM nonessential amino acids, 1 mM sodium pyruvate, and 100 units Penicilin/Streptavidin per mL at 37°C in 5% CO₂. Medium and supplements were obtained from Invitrogen. For the assay, 5 x 10⁵ cells were plated per well in 96well plates in 100 µL per well MEM without Phenol Red and L-Glutamine and with Earle's BSS supplemented with 10% charcoal/dextran treated FBS (HyClone, South Logan, Utah), 0.1 mM nonessential amino acids, 2 mM glutamine, 1 mM sodium pyruvate, and 100 units Penicilin/ Streptavidin per mL, incubated at 37 °C in 5% CO₂. The following day the cells were >90% confluence. Medium was removed and cells were transiently transfected using 20 µL per well of a OptiMEM - polyethylene-imine-based transfection-reagent (OptiMEM, Invitrogen; Polyethyleneimine, Aldrich Cat No. 40,827-7) including the three plasmids described above. MEM with the same composition as used for plating cells was added 2-4 h after addition of transfection mixture. Then compound stocks, prediluted in MEM were added (final vehicle concentration not exceeding 0.1%). Cells were incubated for additional 16 h before firefly and renilla luciferase activities were measured sequentially in the same cell extract using a Dual-Light-Luciferase-Assay system (Dyer et al., Anal. Biochem. 2000, 282, 158-161). All experiments were done in triplicates.

To assess the FXR agonistic potency of the example compounds, potency was determined in the M1H assay as listed below in Table 1 (M1H EC₅₀).

**Table 1**

| **Example** | **FRET IC50 (nM)** | **M1H EC50 (nM)** |
|---|---|---|
| 1 | 88.8 | 167.1 |
| 1C | 134.0 | 479.5 |
| 2 | 29.1 | 363.7 |
| 2A | 133.6 | 2049.0 |
| 2B | 23.6 | 217.4 |
| 3 | 183.5 | 1764.6 |
| 4 | 95.5 | 722.2 |
| 5 | 531.5 | 1299.5 |
| 6 | 10.3 | 12.1 |
| 6a | 5.2 | 5.6 |
| 6b | 25.8 | 134.5 |
| 7 | 8.7 | 206.5 |
| 7a | 25.1 | 855.0 |
| 7b | 5.3 | 52.3 |
| 8 | 43.6 | 894.5 |
| 8a | 110.2 | 2518.4 |
| 8b | 33.2 | 1538.2 |
| 9 | 71.5 | 205.3 |
| 9a | 41.1 | 76.6 |
| 9b | 147.8 | 476.6 |
| 10 | 95.0 | 257.7 |
| 11 | 198.9 | 172.3 |
| 12 | 84.2 | 129.0 |
| 13 | 83.3 | 131.6 |
| 14 | 25.8 | 60.6 |
| 15 | 30.5 | 82.7 |
| 16 | 36.7 | 732.9 |
| 17 | 13.2 | 90.9 |
| 18 | 31.2 | 150.5 |
| 19 | 19.9 | 3000.0 |
| 20 | 57.7 | 834.0 |
| 21 | 18.9 | 2419.5 |
| 22f | | 139.0 |
| 22g | | 117.0 |
| 23 | 21.6 | |
| 24 | 26.2 | 207.9 |
| 25 | 819.5 | 1596.0 |
| 26 | 32.1 | 137.2 |
| 27 | 252.7 | 1866.0 |
| 28 | 249.2 | 121.1 |
| 29 | 451.3 | 328.2 |
| 30 | 2220.8 | 3000.0 |
| 31 | 1236.6 | 3000.0 |
| 32 | 187.4 | 314.7 |
| 33 | 26 | 127.7 |
| 34 | 42 | 980 |
| 35 | 7.2 | 68 |
| 36 | 10 | 5.0 |
| 37 | 148 | 1750 |
| 38 | | |
| 39 | 9.5 | 13.0 |

## Claims

1. A compound according to the following Formula (1), an enantiomer, diastereomer, tautomer, solvate or pharmaceutical acceptable salt thereof 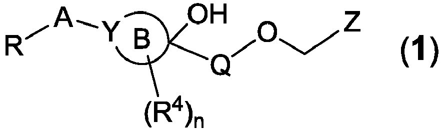 wherein
R is selected from the group consisting of hydrogen, halogen, C₁₋₆-alkyl, C₂₋₆-alkenyl, C₂₋₆-alkynyl, halo-C₁₋₆-alkyl, C₀₋₆-alkylene-R⁷, C₀₋₆-alkylene-O-R⁷, C₀₋₆-alkylene-CN, C₀₋₆-alkylene-NR⁷R⁸, 0-C₃₋₁₀-cycloalkyl, O-C₁₋₆-alkylene-O-R⁷, O-C₃₋₁₀-heterocycloalkyl, C₀₋₆-alkylene-CO₂R⁷, C₀₋₆-alkylene-C(O)R⁷, C₀₋₆-alkylene-C(O)NR⁷R⁸, C₀₋₆-alkylene-C(O)NR⁷SO₂R⁷, C₀₋₆-alkylene-N(R⁷)C(O)R⁷, C₀₋₆-alkylene-SOₓ-R⁷, C₀₋₆-alkylene-SO₃H, C₀₋₆-alkylene-SO₂-NR⁷R⁸, C₀₋₆-alkylene-SO₂-NR⁸COR⁷, C₀₋₆-alkylene-N(R⁷)SO₂-R⁸ and C₀₋₆-alkylene-SO₂-C₃₋₁₀-heterocycloalkyl,
wherein alkylene, cycloalkyl, heterocycloalkyl and the 5- or 6-membered heteroaryl are unsubstituted or substituted by 1 to 4 substituents independently selected from the group consisting of halogen, CN, C₁₋₃-alkyl, halo-C₁₋₃-alkyl, OH, oxo, CO₂H, SO₃H, O-C₁₋₃-alkyl and O-halo-C₁₋₃-alkyl;
R⁷ is independently selected from the group consisting of hydrogen, C₁₋₆-alkyl, halo-C₁₋₆-alkyl, C₀₋₆-alkylene-C₃₋₈-cycloalkyl, C₀₋₆-alkylene-C₃₋₈-heterocycloalkyl, 5- or 6-membered heteroaryl and phenyl, wherein alkyl, alkylene, cyclolalkyl, heterocycloalkyl, phenyl and heteroaryl are unsubstituted or substituted with 1 to 6 substituents independently selected from the group consisting of halogen, CN, OH, oxo, CO₂H, C₁₋₃-alkyl, halo-C₁₋₃-alkyl, O-C₁₋₃-alkyl, O-halo-C₁₋₃-alkyl, SO₃H and SO₂-C₁₋₃-alkyl;
R⁸ is independently selected from the group consisting of hydrogen, C₁₋₆-alkyl, halo-C₁₋₆-alkyl and C₃₋₆-cycloalkyl;
or R⁷ and R⁸ when taken together with the nitrogen to which they are attached may complete a 3- to 8-membered ring containing carbon atoms and optionally containing 1 or 2 heteroatoms selected from O, S or N, wherein the ring is unsubstituted or substituted with 1 to 4 substituents independently selected from the group consisting of fluoro, OH, oxo, C₁₋₄-alkyl and halo-C₁₋₄-alkyl;
A is a 6-10 membered mono- or bicyclic aryl or a 5-10 membered mono- or bicyclic heteroaryl containing 1 to 5 heteroatoms independently selected from the group consisting of N, O and S, wherein aryl and heteroaryl are unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₆alkyl, C₃₋₆-cycloalkyl and halo-C₃₋₆-cycloalkyl;
B is a C₅₋₈-cycloalkyl ring or, if Y is N, then B is a C₄₋₈-heterocycloalkyl or bridged C₄₋₈-heterocycloalkyl containing one nitrogen atom, and wherein the substituent Q is not directly adjacent to substituent A;
Q is selected from the group consisting of phenyl, pyridyl, thiazolyl, thiophenyl, pyrimidyl, oxazolyl, pyrazolyl, imidazolyl and triazolyl, each unsubstituted or substituted with one or two groups independently selected from the group consisting of halogen, C₁₋₄-alkyl, halo-C₁₋₄alkyl, C₁₋₄-alkoxy and halo-C₁₋₄alkoxy;
Y is selected from N, CH or CF;
Z is selected from 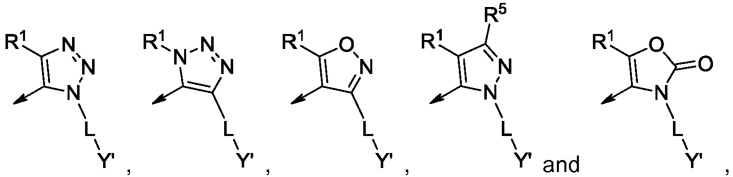 wherein
L is selected from the group consisting of a bond, C₁₋₃-alkylene and C₁₋₃-alkylene-O-;
Y' is selected from phenyl, pyridyl, pyridyl-*N*-oxide, pyrimidyl, pyridinonyl, pyrimidinonyl, C₄₋₈-cycloalkyl and C₄₋₈-heterocycloalkyl, wherein phenyl, pyridyl, pyridyl-*N*-oxide, pyrimidyl, pyridinonyl, pyrimidinonyl, C₄₋₈-cycloalkyl and C₄₋₈-heterocycloalkyl are substituted with R² and R³ and optionally substituted one or two times with a group selected from fluoro, chloro, CN, NH₂, NH(C₁₋₃-alkyl), N(C₁₋₃-alkyl)₂, C₁₋₃-alkyl, fluoro-C₁₋₃-alkyl, OH, C₁₋₃-alkoxy, fluoro-C₁₋₃-alkoxy, C₃₋₆-cycloalkyl and fluoro-C₃₋₆-cycloalkyl;
R¹ is selected from the group consisting of C₁₋₄-alkyl and C₃₋₆-cycloalkyl, wherein C₁₋₄-alkyl is unsubstituted or substituted with 1 to 3 substituents independently selected from the group consisting of fluoro, hydroxy, C₁₋₃-alkoxy and fluoro-C₁₋₃-alkoxy, and C₃₋₆-cycloalkyl is unsubstituted or substituted with 1 to 3 substituents independently selected from the group consisting of fluoro, hydroxy, C₁₋₃-alkyl, fluoro-C₁₋₃-alkyl, C₁₋₃-alkoxy and fluoro-C₁₋₃-alkoxy;
R² and R³ are independently selected from the group consisting of hydrogen, halogen, C₁₋₃-alkyl, halo-C₁₋₃-alkyl, C₁₋₃-alkoxy, halo-C₁₋₃-alkoxy, cyclopropyl and fluoro-cyclopropyl;
R⁴ is independently selected from halogen, C₁₋₃-alkyl, halo-C₁₋₃-alkyl, C₁₋₃-alkoxy, halo-C₁₋₃-alkoxy, C₃₋₆-cycloalkyl and fluoro-C₃₋₆-cycloalkyl;
R⁵ is selected from the group consisting of hydrogen, fluoro, CH₃, CHF₂ and CF₃;
n is selected from 0, 1, 2, 3 and 4; and
x is selected from 0, 1 and 2.

2. The compound according to claim 1 wherein
R is selected from the group consisting of hydrogen, halogen, C₁₋₆-alkyl, C₂₋₆-alkenyl, C₂₋₆-alkynyl, halo-C₁₋₆alkyl, C₀₋₆-alkylene-O-R⁷, C₀₋₆-alkylene-CN, C₀₋₆-alkylene-NR⁷R⁸, O-C₃₋₁₀-cycloalkyl, O-C₁₋₆-alkylene-O-R⁷, O-C₃₋₁₀-heterocycloalkyl, C₀₋₆-alkylene-CO₂R⁷, C₀₋₆-alkylene-C(O)R⁷, C₀₋₆-alkylene-C(O)NR⁷R⁸, C₀₋₆-alkylene-C(O)NR⁷SO₂R⁷, C₀₋₆-alkylene-N(R⁷)C(O)R⁷, C₀₋₆-alkylene-SOₓ-R⁷, C₀₋₆-alkylene-SO₃H, C₀₋₆-alkylene-SO₂-NR⁷R⁸, C₀₋₆-alkylene-SO₂-NR⁸COR⁷, C₀₋₆-alkylene-N(R⁷)SO₂-R⁸ and C₀₋₆-alkylene-SO₂-C₃₋₁₀-heterocycloalkyl,
wherein alkylene, cycloalkyl, heterocycloalkyl and the 5- or 6-membered heteroaryl are unsubstituted or substituted by 1 to 4 substituents independently selected from the group consisting of halogen, CN, C₁₋₃-alkyl, halo-C₁₋₃alkyl, OH, oxo, CO₂H, SO₃H, O-C₁₋₃-alkyl and O-halo-C₁₋₃-alkyl;
R⁷ is independently selected from the group consisting of hydrogen, C₁₋₆-alkyl, halo-C₁₋₃alkyl, C₀₋₆-alkylene-C₃₋₈-cycloalkyl, C₀₋₆-alkylene-C₃₋₈-heterocycloalkyl, 5- or 6-membered heteroaryl and phenyl, wherein alkyl, alkylene, cyclolalkyl, heterocycloalkyl, phenyl and heteroaryl are unsubstituted or substituted with 1 to 6 substituents independently selected from the group consisting of halogen, CN, OH, oxo, CO₂H, C₁₋₃-alkyl, halo-C₁₋₃alkyl, O-C₁₋₃-alkyl, O-halo-C₁₋₃-alkyl, SO₃H and SO₂-C₁₋₃-alkyl;
R⁸ is independently selected from the group consisting of hydrogen, C₁₋₆-alkyl, halo-C₁₋₆alkyl and C₃₋₆-cycloalkyl;
or R⁷ and R⁸ when taken together with the nitrogen to which they are attached may complete a 3- to 8-membered ring containing carbon atoms and optionally containing 1 or 2 heteroatoms selected from O, S or N, wherein the ring is unsubstituted or substituted with 1 to 4 substituents independently selected from the group consisting of fluoro, OH, oxo, C₁₋₄-alkyl and halo-C₁₋₄ alkyl;
A is a 6-10 membered mono- or bicyclic aryl or a 5-10 membered mono- or bicyclic heteroaryl containing 1 to 5 heteroatoms independently selected from the group consisting of N, O and S, wherein aryl and heteroaryl are unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₃alkyl, C₃₋₆-cycloalkyl and halo-C₃₋₆-cycloalkyl;
B is a C₅₋₈-cycloalkyl ring or, if Y is N, then B is a C₅₋₈-heterocycloalkyl containing one nitrogen atom, and wherein the substituent Q is not directly adjacent to substituent A;
Q is selected from the group consisting of phenyl, pyridyl, thiazolyl, thiophenyl, pyrimidyl, oxazolyl, pyrazolyl, imidazolyl and triazolyl, each unsubstituted or substituted with one or two groups independently selected from the group consisting of halogen, C₁₋₄alkyl, halo-C₁₋₄-alkyl, C₁₋₄-alkoxy and halo-C₁₋₄-alkoxy;
Y is selected from N, CH or CF;
Z is selected from 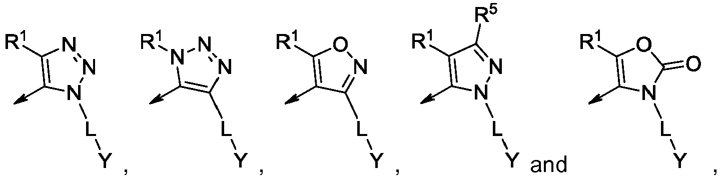 wherein
L is selected from the group consisting of a bond, C₁₋₃-alkylene and C₁₋₃-alkylene-O-;
Y is selected from phenyl, pyridyl, pyridyl-*N*-oxide, pyrimidyl, pyridinonyl, pyrimidinonyl, C₄₋₈-cycloalkyl and C₄₋₈-heterocycloalkyl, wherein phenyl, pyridyl, pyridyl-*N*-oxide, pyrimidyl, pyridinonyl, pyrimidinonyl, C₄₋₈-cycloalkyl and C₄₋₈-heterocycloalkyl are substituted with R² and R³ and optionally substituted one or two times with a group selected from fluoro, chloro, CN, NH₂, NH(C₁₋₃-alkyl), N(C₁₋₃-alkyl)₂, C₁₋₃-alkyl, fluoro-C₁₋₃-alkyl, OH, C₁₋₃-alkoxy, fluoro-C₁₋₃-alkoxy, C₃₋₆-cycloalkyl and fluoro-C₃₋₆-cycloalkyl;
R¹ is selected from the group consisting of C₁₋₄-alkyl and C₃₋₆-cycloalkyl, wherein C₁₋₄-alkyl is unsubstituted or substituted with 1 to 3 substituents independently selected from the group consisting of fluoro, hydroxy, C₁₋₃-alkoxy and fluoro-C₁₋₃-alkoxy, and C₃₋₆-cycloalkyl is unsubstituted or substituted with 1 to 3 substituents independently selected from the group consisting of fluoro, hydroxy, C₁₋₃-alkyl, fluoro-C₁₋₃-alkyl, C₁₋₃-alkoxy and fluoro-C₁₋₃-alkoxy;
R² and R³ are independently selected from the group consisting of hydrogen, halogen, C₁₋₃-alkyl, halo-C₁₋₃-alkyl, C₁₋₃-alkoxy, halo-C₁₋₃-alkoxy, cyclopropyl and fluoro-cyclopropyl;
R⁴ is independently selected from halogen, C₁₋₃-alkyl, halo-C₁₋₃-alkyl, C₁₋₃-alkoxy, halo-C₁₋₃-alkoxy, C₃₋₆-cycloalkyl and fluoro-C₃₋₆-cycloalkyl;
R⁵ is selected from the group consisting of hydrogen, fluoro, CH₃, CHF₂ and CF₃;
n is selected from 0, 1, 2, 3 and 4; and
x is selected from 0, 1 and 2.

3. The compound according to claim 1 or 2 wherein
R is selected from the group consisting of CO₂H, SO₃H, CONR⁷R⁸, tetrazolyl, 1,2,4-oxadiazol-5(4H)-one-3-yl and SO₂NHCOR⁷;
R⁷ is selected from the group consisting of hydrogen, C₁₋₆-alkyl, halo-C₁₋₆alkyl and C₁₋₆-alkylene-R⁹;
R⁸ is selected from the group consisting of hydrogen, C₁₋₆-alkyl and halo-C₁₋₆-alkyl; and
R⁹ is selected from the group consisting of COOH, OH and SO₃H.

4. The compound according to any of claims 1 to 3 wherein
A is selected from the group consisting of phenyl, pyridyl, pyrimidyl, pyrazolyl, indolyl, thienyl, benzothienyl, indazolyl, benzisoxazolyl, benzofuranyl, benzotriazolyl, furanyl, benzothiazolyl, thiazolyl, oxadiazolyl, oxazolyl, naphthyl, quinolyl, isoquinolyl and benzimidazolyl, each unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₃alkyl, C₃₋₆-cycloalkyl and halo-C₃₋₆-cycloalkyl.

5. The compound according to any of claims 1 to 4 wherein R-A is selected from

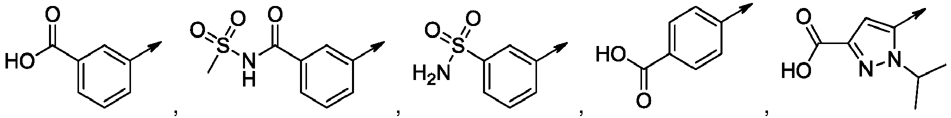

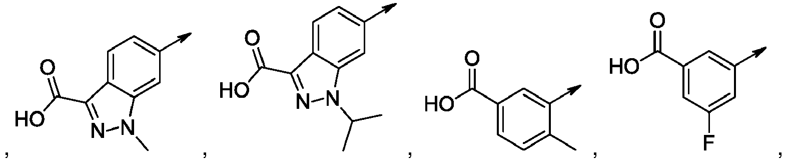

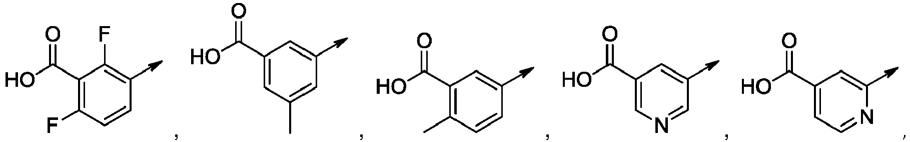

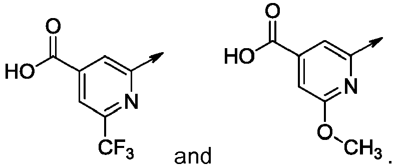

6. The compound according to any of claims 1 to 5 wherein Z is selected from 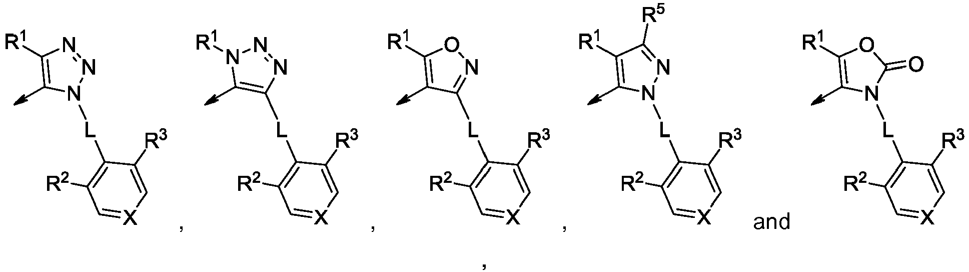 wherein
L is selected from the group consisting of a bond, C₁₋₃-alkylene and C₁₋₃-alkylene-O-;
X is selected from the group consisting of CH, CF, N and NO;
R¹ is selected from the group consisting of C₁₋₄-alkyl and C₃₋₆-cycloalkyl, wherein C₁₋₄-alkyl is unsubstituted or substituted with 1 to 3 substituents independently selected from the group consisting of fluoro, hydroxy, C₁₋₃-alkoxy and fluoro-C₁₋₃-alkoxy, and C₃₋₆-cycloalkyl is unsubstituted or substituted with 1 to 3 substituents independently selected from the group consisting of fluoro, hydroxy, C₁₋₃-alkyl, fluoro-C₁₋₃-alkyl, C₁₋₃-alkoxy and fluoro-C₁₋₃-alkoxy;
R² and R³ are independently selected from the group consisting of hydrogen, halogen, C₁₋₃-alkyl, halo-C₁₋₃alkyl, C₁₋₃-alkoxy, halo-C₁₋₃-alkoxy, cyclopropyl and fluoro-cyclopropyl; and
R⁵ is selected from the group consisting of hydrogen, fluoro, CH₃, CHF₂ and CF₃.

7. The compound according to any of claims 1 to 6 wherein Z is selected from 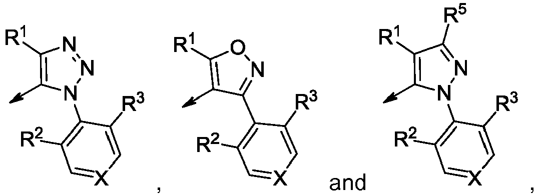 wherein
X is selected from the group consisting of CH, CF, N and NO;
R¹ is selected from the group consisting of CF₃, CHF₂, isopropyl and cyclopropyl, wherein isopropyl and cyclopropyl are unsubstituted or substituted with one or two fluoro or one hydroxy;
R² is selected from the group consisting of fluoro, chloro, CH₃, CHF₂, CF₃, OCHF₂ and OCF₃;
R³ is selected from the group consisting of hydrogen, fluoro, chloro, CH₃, CHF₂, CF₃, OCHF₂ and OCF₃; and
R⁵ is selected from the group consisting of hydrogen, fluoro, CH₃, CHF₂ and CF₃.

8. The compound according to any of claims 1 to 7 wherein 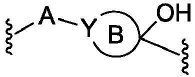 is selected from 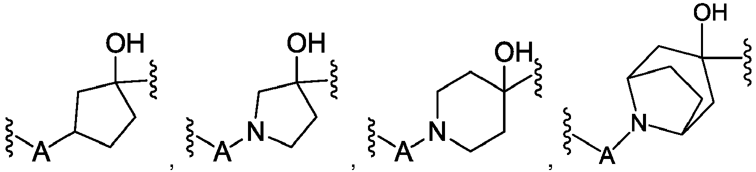 and

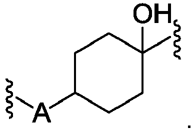

9. The compound according to any of claims 1 to 8 with Formula (2) 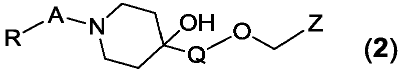 wherein
A is selected from the group consisting of phenyl, pyridyl, pyrimidyl, pyrazolyl, indolyl, thienyl, benzothienyl, indazolyl, benzisoxazolyl, benzofuranyl, benzotriazolyl, furanyl, benzothiazolyl, thiazolyl, oxadiazolyl, oxazolyl, naphthyl, quinolyl, isoquinolyl and benzimidazolyl, each unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₆-alkyl, C₃₋₆-cycloalkyl and halo-C₃₋₆-cycloalkyl;
R is selected from the group consisting of CO₂H, SO₃H, CONR⁷R⁸, tetrazolyl, 1,2,4-oxadiazol-5(4H)-one-3-yl and SO₂NHCOR⁷, wherein
R⁷ is selected from the group consisting of hydrogen, C₁₋₆-alkyl, halo-C₁₋₆-alkyl and C₁₋₆-alkylene-R⁹;
R⁸ is selected from the group consisting of hydrogen, C₁₋₆-alkyl and halo-C₁₋₆-alkyl; and
R⁹ is selected from the group consisting of COOH, OH and SO₃H;
Q is selected from the group consisting of phenyl, pyridyl, thiazolyl, thiophenyl and pyrimidyl, each unsubstituted or substituted with one or two groups independently selected from the group consisting of fluoro, chloro, CH₃, CHF₂ and CF₃;
Z is selected from 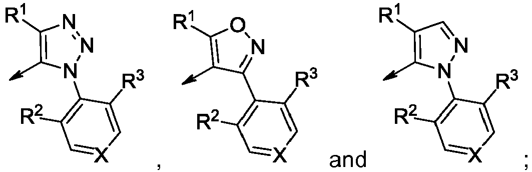
X is selected from the group consisting of CH, N and NO;
R¹ is selected from the group consisting of isopropyl and cyclopropyl, wherein isopropyl and cyclopropyl are unsubstituted or substituted with one or two fluoro or one hydroxy;
R² is selected from the group consisting of fluoro, chloro, CH₃, CHF₂, CF₃, OCHF₂ and OCF₃; and
R³ is selected from the group consisting of hydrogen, fluoro, chloro, CH₃, CHF₂, CF₃, OCHF₂ and OCF₃.

10. The compound according to any of claims 1 to 8 with Formula (3) 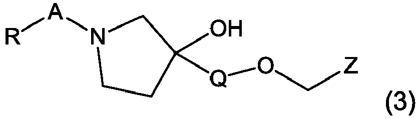 wherein
A is selected from the group consisting of phenyl, pyridyl, pyrimidyl, pyrazolyl, indolyl, thienyl, benzothienyl, indazolyl, benzisoxazolyl, benzofuranyl, benzotriazolyl, furanyl, benzothiazolyl, thiazolyl, oxadiazolyl, oxazolyl, naphthyl, quinolyl, isoquinolyl and benzimidazolyl, each unsubstituted or substituted with one or two groups independently selected from the group consisting of OH, halogen, CN, O-C₁₋₆-alkyl, O-halo-C₁₋₆-alkyl, C₁₋₆-alkyl, halo-C₁₋₃alkyl, C₃₋₆-cycloalkyl and halo-C₃₋₆-cycloalkyl;
R is selected from the group consisting of CO₂H, SO₃H, CONR⁷R⁸, tetrazolyl, 1,2,4-oxadiazol-5(4H)-one-3-yl and SO₂NHCOR⁷, wherein
R⁷ is selected from the group consisting of hydrogen, C₁₋₆-alkyl, halo-C₁₋₆alkyl and C₁₋₆-alkylene-R⁹;
R⁸ is selected from the group consisting of hydrogen, C₁₋₆-alkyl and halo-C₁₋₆-alkyl; and
R⁹ is selected from the group consisting of COOH, OH and SO₃H;
Q is selected from the group consisting of phenyl, pyridyl, thiazolyl, thiophenyl and pyrimidyl, each unsubstituted or substituted with one or two groups independently selected from the group consisting of fluoro, chloro, CH₃, CHF₂ and CF₃;
Z is selected from 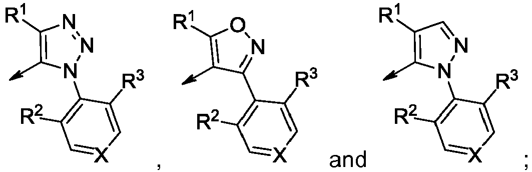
X is selected from the group consisting of CH, N and NO;
R¹ is selected from the group consisting of isopropyl and cyclopropyl, wherein isopropyl and cyclopropyl are unsubstituted or substituted with one or two fluoro or one hydroxy;
R² is selected from the group consisting of fluoro, chloro, CH₃, CHF₂, CF₃, OCHF₂ and OCF₃; and
R³ is selected from the group consisting of hydrogen, fluoro, chloro, CH₃, CHF₂, CF₃, OCHF₂ and OCF₃.

11. The compound according to claim 1 selected from the group consisting of: 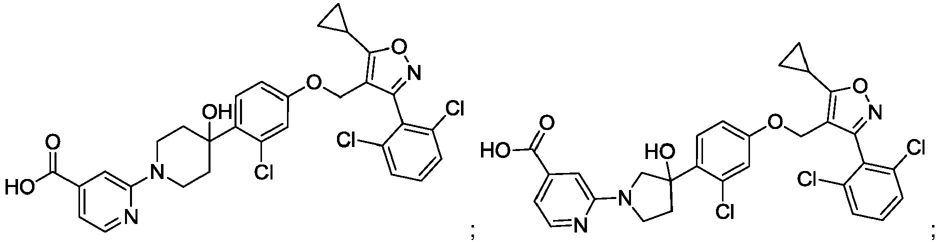 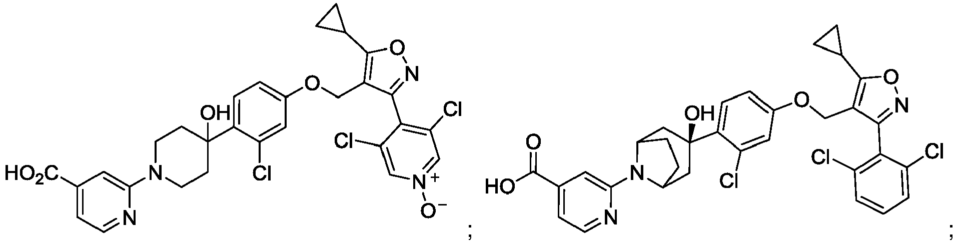 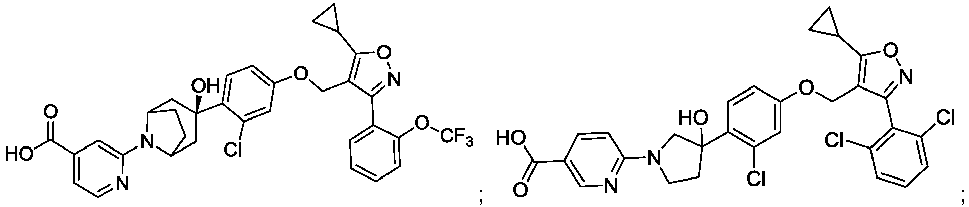 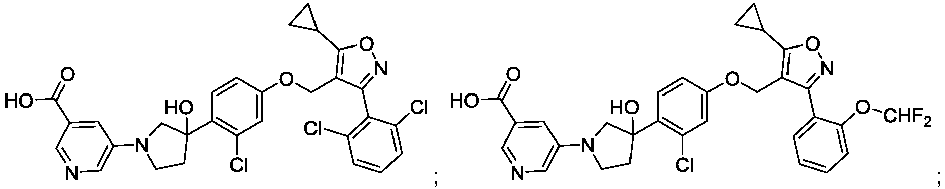 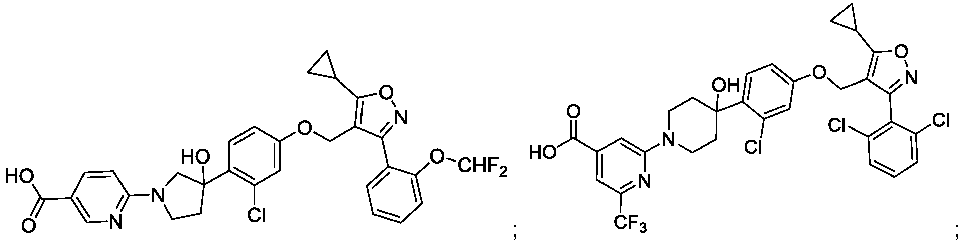 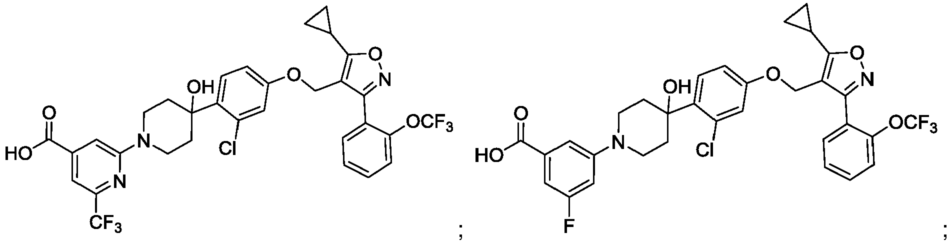 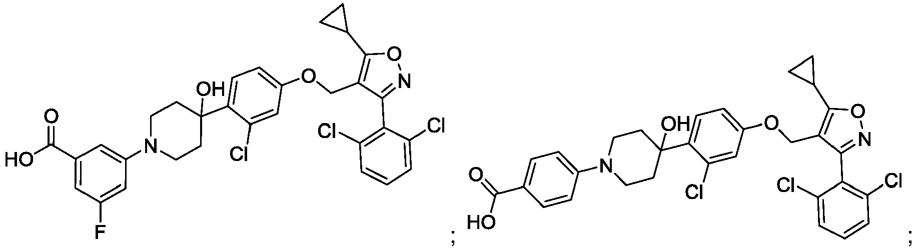 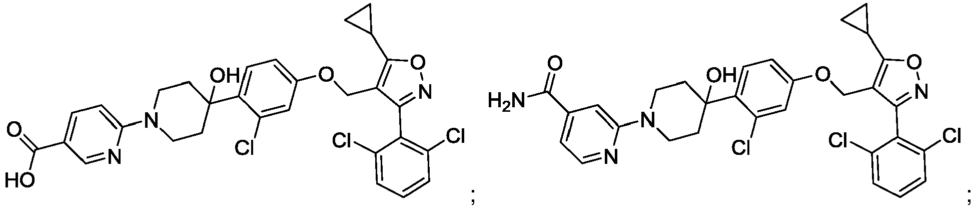 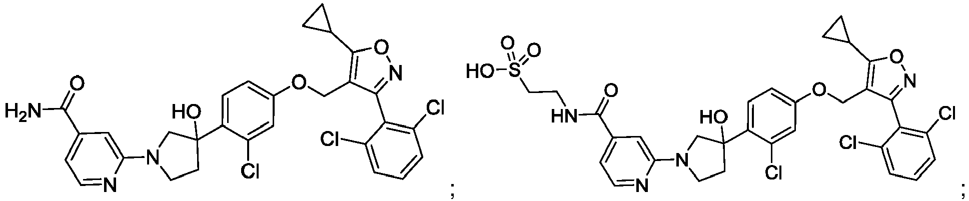 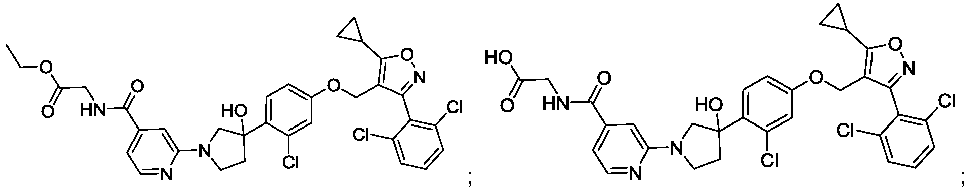 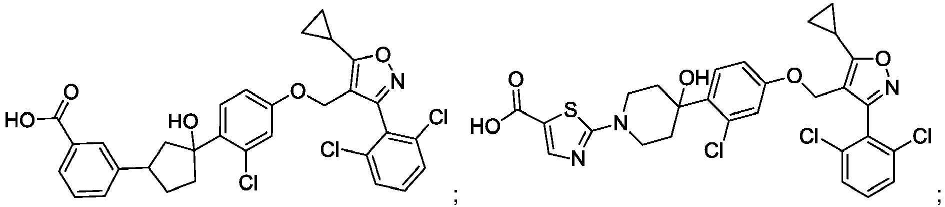 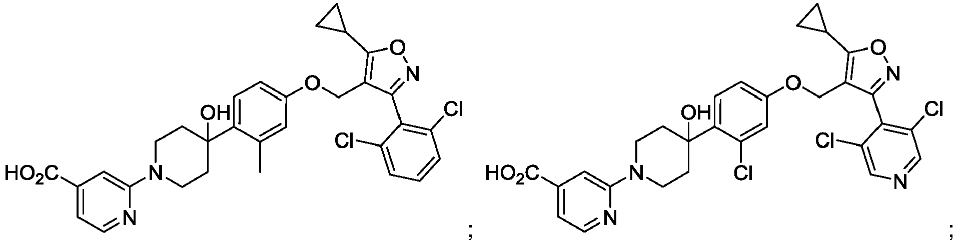 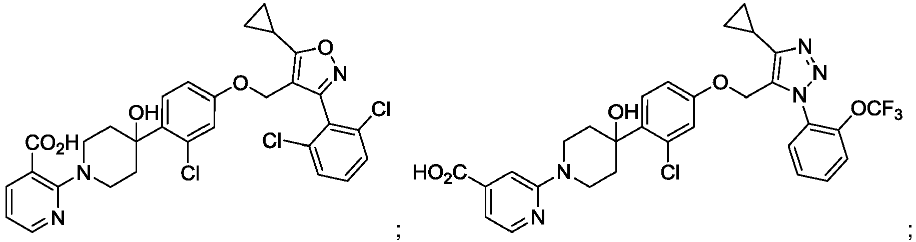 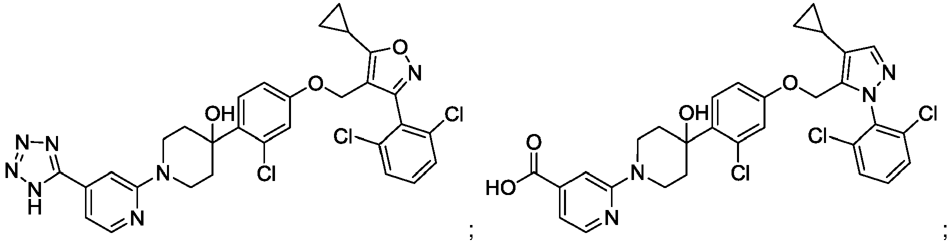 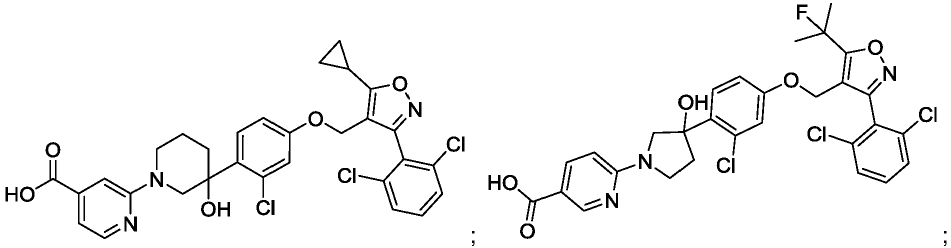 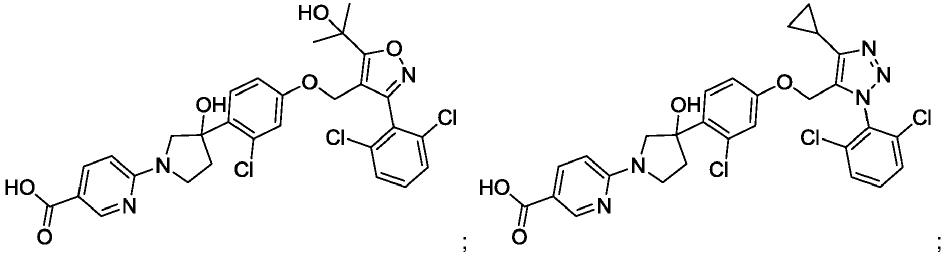 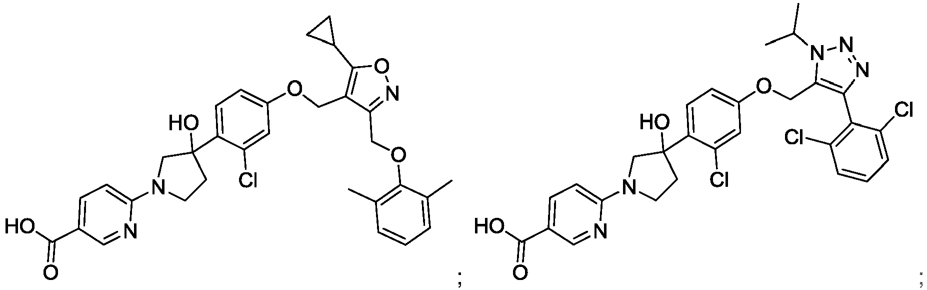 ; and 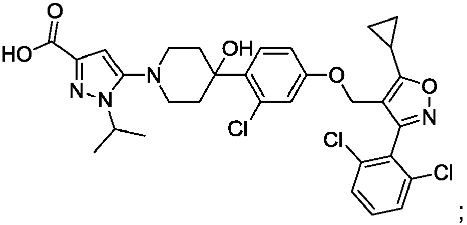 or an enantiomer, diastereomer, tautomer, solvate or pharmaceutical acceptable salt thereof.

12. A compound according to any of claims 1 to 11 for use as a medicament.

13. A compound according to any of claims 1 to 11 for use in the prophylaxis and/or treatment of diseases mediated by FXR.

14. The compound for use according to claim 13 wherein the disease is selected from chronic intrahepatic or some forms of extrahepatic cholestatic conditions;
liver fibrosis;
obstructive or chronic inflammatory disorders of the liver;
liver cirrhosis;
liver steatosis and associated syndromes, cholestatic or fibrotic effects that are associated with alcohol-induced cirrhosis or with viral-borne forms of hepatitis;
liver failure or liver ischemia after major liver resection;
chemotherapy associated steatohepatitis (CASH);
acute liver failure; and
Inflammatory Bowel Diseases.

15. The compound for use according to claim 13 wherein the disease is selected from
lipid and lipoprotein disorders;
Type II Diabetes and clinical complications of Type I and Type II Diabetes, including diabetic nephropathy, diabetic neuropathy, diabetic retinopathy and other observed effects of clinically manifest long term Diabetes;
conditions and diseases which result from chronic fatty and fibrotic degeneration of organs due to enforced lipid and
specifically triglyceride accumulation and subsequent activation of profibrotic pathways, such as Non-Alcoholic Fatty Liver Disease (NAFLD), or Non-Alcoholic Steatohepatitis (NASH);
obesity or metabolic syndrome (combined conditions of dyslipidemia, diabetes or abnormally high body-mass index); and
acute myocardial infarction, acute stroke or thrombosis which occurs as an endpoint of chronic obstructive atherosclerosis.

16. The compound for use according to claim 13 wherein the disease is selected from
non-malignant hyperproliferative disorders and malignant hyperproliferative disorders, specifically of hepatocellular carcinoma, colon adenoma and polyposis, colon adenocarcinoma, breast cancer, pancreas adenocarcinoma, Barrett's esophagus or other forms of neoplastic diseases of the gastrointestinal tract and the liver.

## Patentansprüche

1. Verbindung nach der folgenden Formel **(1),** ein Enantiomer, Diastereomer, Tautomer, Solvat oder pharmazeutisch verträgliches Salz davon 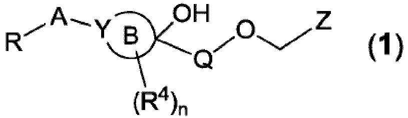 wobei
R aus der Gruppe ausgewählt ist, die aus Wasserstoff, Halogen, C₁₋₆-Alkyl, C₂₋₆-Alkenyl, C₂₋₆-Alkinyl, Halogen-C₁₋₆-alkyl, C₀₋₆-Alkylen-R⁷, C₀₋₆-Alkylen-O-R⁷, C₀₋₆-Alkylen-CN, C₀₋₆-Alkylen-NR⁷R⁸, O-C₃₋₁₀-Cycloalkyl, O-C₁₋₆-Alkylen-O-R⁷, O-C₃₋₁₀-Heterocycloalkyl, C₀₋₆-Alkylen-CO₂R⁷, C₀₋₆-Alkylen-C(O)R⁷, C₀₋₆-Alkylen-C(O)NR⁷R⁸, C₀₋₆-Alkylen-C(O)NR⁷SO₂R⁷, C₀₋₆-Alkylen-N(R⁷)C(O)R⁷, C₀₋₆-Alkylen-SOₓ-R⁷, C₀₋₆-Alkylen-SO₃H, C₀₋₆-Alkylen-SO₂-NR⁷R⁸, C₀₋₆-Alkylen-SO₂-NR⁸COR⁷, C₀₋₆-Alkylen-N(R⁷)SO₂-R⁸ und C₀₋₆-Alkylen-SO₂-C₃₋₁₀-heterocycloalkyl besteht,
wobei Alkylen, Cycloalkyl, Heterocycloalkyl und das 5- oder 6-gliedrige Heteroaryl unsubstituiert oder durch 1 bis 4 Substituenten substituiert sind, die unabhängig aus der Gruppe ausgewählt sind, die aus Halogen, CN, C₁₋₃-Alkyl, Halogen-C₁₋₃-alkyl, OH, Oxo, CO₂H, SO₃H, O-C₁₋₃-Alkyl und O-Halogen-C₁₋₃-alkyl besteht;
R⁷ unabhängig aus der Gruppe ausgewählt ist, die aus Wasserstoff, C₁₋₆-Alkyl, Halogen-C₁₋₆-alkyl, C₀₋₆-Alkylen-C₃₋₈-cycloalkyl, C₀₋₆-Alkylen-C₃₋₈-heterocycloalkyl, 5- oder 6-gliedrigem Heteroaryl und Phenyl besteht, wobei Alkyl, Alkylen, Cycloalkyl, Heterocycloalkyl, Phenyl und Heteroaryl unsubstituiert oder mit 1 bis 6 Substituenten substituiert sind, die unabhängig aus der Gruppe ausgewählt sind, die aus Halogen, CN, OH, Oxo, CO₂H, C₁₋₃-Alkyl, Halogen-C₁₋₃-alkyl, O-C₁₋₃-Alkyl, O-Halogen-C₁₋₃-alkyl, SO₃H und SO₂-C₁₋₃-Alkyl besteht;
R⁸ unabhängig aus der Gruppe ausgewählt ist, die aus Wasserstoff, C₁₋₆-Alkyl, Halogen-C₁₋₆-alkyl und C₃₋₆-Cycloalkyl besteht;
oder R⁷ und R⁸, wenn zusammengenommen mit dem Stickstoff, an den sie gebunden sind, einen 3- bis 8-gliedrigen Ring vervollständigen können, der Kohlenstoffatome enthält und der wahlweise 1 oder 2 Heteroatome, ausgewählt aus O, S oder N, enthält, wobei der Ring unsubstituiert oder mit 1 bis 4 Substituenten substituiert ist, die unabhängig aus der Gruppe ausgewählt sind, die aus Fluor, OH, Oxo, C₁₋₄-Alkyl und Halogen-C₁₋₄-alkyl besteht;
A ein 6- bis 10-gliedriges mono- oder bicyclisches Aryl oder ein 5- bis 10-gliedriges mono- oder bicyclisches Heteroaryl ist, das 1 bis 5 Heteroatome enthält, die unabhängig aus der Gruppe, bestehend aus N, O und S, ausgewählt sind, wobei Aryl und Heteroaryl unsubstituiert oder mit einer oder zwei Gruppen substituiert sind, die unabhängig aus der Gruppe ausgewählt sind, die aus OH, Halogen, CN, O-C₁₋₆-Alkyl, O-Halogen-C₁₋₆-alkyl, C₁₋₆-Alkyl, Halogen-C₁₋₆-alkyl, C₃₋₆-Cycloalkyl und Halogen-C₃₋₆-cycloalkyl besteht;
B ein C₅₋₈-Cycloalkylring ist oder, falls Y N ist, B ein C₄₋₈-Heterocycloalkyl oder verbrücktes C₄₋₈-Heterocycloalkyl ist, das ein Stickstoffatom enthält, und wobei sich der Substituent Q nicht unmittelbar neben Substituent A befindet;
Q aus der Gruppe ausgewählt ist, die aus Phenyl, Pyridyl, Thiazolyl, Thiophenyl, Pyrimidyl, Oxazolyl, Pyrazolyl, Imidazolyl und Triazolyl besteht, jeweils unsubstituiert oder substituiert mit einer oder zwei Gruppen, die unabhängig aus der Gruppe ausgewählt sind, die aus Halogen, C₁₋₄-Alkyl, Halogen-C₁₋₄-alkyl, C₁₋₄-Alkoxy und Halogen-C₁₋₄-alkoxy besteht;
Y aus N, CH oder CF ausgewählt ist;
Z ausgewählt ist aus 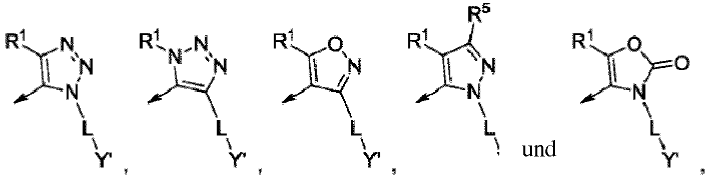 wobei
L aus der Gruppe ausgewählt ist, die aus einer Bindung, C₁₋₃-Alkylen und C₁₋₃-Alkylen-O- besteht;
Y' aus Phenyl, Pyridyl, Pyridyl-*N*-oxid, Pyrimidyl, Pyridinonyl, Pyrimidinonyl, C₄₋₈-Cycloalkyl und C₄₋₈-Heterocycloalkyl ausgewählt ist, wobei Phenyl, Pyridyl, Pyridyl-*N*-oxid, Pyrimidyl, Pyridinonyl, Pyrimidinonyl, C₄₋₈-Cycloalkyl und C₄₋₈-Heterocycloalkyl mit R² und R³ substituiert sind und wahlweise ein- oder zweimal mit einer Gruppe substituiert sind, die aus Fluor, Chlor, CN, NH₂, NH(C₁₋₃-Alkyl), N(C₁₋₃-Alkyl)₂, C₁₋₃-Alkyl, Fluor-C₁₋₃-alkyl, OH, C₁₋₃-Alkoxy, Fluor-C₁₋₃-alkoxy, C₃₋₆-Cycloalkyl und Fluor-C₃₋₆-cycloalkyl ausgewählt ist;
R¹ aus der Gruppe ausgewählt ist, die aus C₁₋₄-Alkyl und C₃₋₆-Cycloalkyl besteht, wobei C₁₋₄-Alkyl unsubstituiert oder mit 1 bis 3 Substituenten substituiert ist, die unabhängig aus der Gruppe ausgewählt sind, die aus Fluor, Hydroxy, C₁₋₃-Alkoxy und Fluor-C₁₋₃-alkoxy besteht, und C₃₋₆-Cycloalkyl unsubstituiert oder mit 1 bis 3 Substituenten substituiert ist, die unabhängig aus der Gruppe ausgewählt sind, die aus Fluor, Hydroxy, C₁₋₃-Alkyl, Fluor-C₁₋₃-alkyl, C₁₋₃-Alkoxy und Fluor-C₁₋₃-alkoxy besteht;
R² und R³ unabhängig aus der Gruppe ausgewählt sind, die aus Wasserstoff, Halogen, C₁₋₃-Alkyl, Halogen-C₁₋₃-alkyl, C₁₋₃-Alkoxy, Halogen-C₁₋₃-alkoxy, Cyclopropyl und Fluor-cyclopropyl besteht;
R⁴ unabhängig aus Halogen, C₁₋₃-Alkyl, Halogen-C₁₋₃-alkyl, C₁₋₃-Alkoxy, Halogen-C₁₋₃-alkoxy, C₃₋₆-Cycloalkyl und Fluor-C₃₋₆-cycloalkyl ausgewählt ist;
R⁵ aus der Gruppe ausgewählt ist, die aus Wasserstoff, Fluor, CH₃, CHF₂ und CF₃ besteht;
n aus 0, 1, 2, 3 und 4 ausgewählt ist; und
x aus 0, 1 und 2 ausgewählt ist.

2. Verbindung nach Anspruch 1, wobei
R aus der Gruppe ausgewählt ist, die aus Wasserstoff, Halogen, C₁₋₆-Alkyl, C₂₋₆-Alkenyl, C₂₋₆-Alkinyl, Halogen-C₁₋₆-alkyl, C₀₋₆-Alkylen-O-R⁷, C₀₋₆-Alkylen-CN, C₀₋₆-Alkylen-NR⁷R⁸, O-C₃₋₁₀-Cycloalkyl, O-C₁₋₆-Alkylen-O-R⁷, O-C₃₋₁₀-Heterocycloalkyl, C₀₋₆-Alkylen-CO₂R⁷, C₀₋₆-Alkylen-C(O)R⁷, C₀₋₆-Alkylen-C(O)NR⁷R⁸, C₀₋₆-Alkylen-C(O)NR⁷SO₂R⁷, C₀₋₆-Alkylen-N(R⁷)C(O)R⁷, C₀₋₆-Alkylen-SOₓ-R⁷, C₀₋₆-Alkylen-SO₃H, C₀₋₆-Alkylen-SO₂-NR⁷R⁸, C₀₋₆-Alkylen-SO₂-NR⁸COR⁷, C₀₋₆-Alkylen-N(R⁷)SO₂-R⁸ und C₀₋₆-Alkylen-SO₂-C₃₋₁₀-heterocycloalkyl besteht,
wobei Alkylen, Cycloalkyl, Heterocycloalkyl und das 5- oder 6-gliedrige Heteroaryl unsubstituiert oder durch 1 bis 4 Substituenten substituiert sind, die unabhängig aus der Gruppe ausgewählt sind, die aus Halogen, CN, C₁₋₃-Alkyl, Halogen-C₁₋₃-alkyl, OH, Oxo, CO₂H, SO₃H, O-C₁₋₃-Alkyl und O-Halogen-C₁₋₃-alkyl besteht;
R⁷ unabhängig aus der Gruppe ausgewählt ist, die aus Wasserstoff, C₁₋₆-Alkyl, Halogen-C₁₋₆-alkyl, C₀₋₆-Alkylen-C₃₋₈-cycloalkyl, C₀₋₆-Alkylen-C₃₋₈-heterocycloalkyl, 5-oder 6-gliedrigem Heteroaryl und Phenyl besteht, wobei Alkyl, Alkylen, Cycloalkyl, Heterocycloalkyl, Phenyl und Heteroaryl unsubstituiert oder mit 1 bis 6 Substituenten substituiert sind, die unabhängig aus der Gruppe ausgewählt sind, die aus Halogen, CN, OH, Oxo, CO₂H, C₁₋₃-Alkyl, Halogen-C₁₋₃-alkyl, O-C₁₋₃-Alkyl, O-Halogen-C₁₋₃-alkyl, SO₃H und SO₂-C₁₋₃-Alkyl besteht;
R⁸ unabhängig aus der Gruppe ausgewählt ist, die aus Wasserstoff, C₁₋₆-Alkyl, Halogen-C₁₋₆-alkyl und C₃₋₆-Cycloalkyl besteht;
oder R⁷ und R⁸, wenn zusammengenommen mit dem Stickstoff, an den sie gebunden sind, einen 3- bis 8-gliedrigen Ring vervollständigen können, der Kohlenstoffatome enthält und der wahlweise 1 oder 2 Heteroatome, ausgewählt aus O, S oder N, enthält, wobei der Ring unsubstituiert oder mit 1 bis 4 Substituenten substituiert ist, die unabhängig aus der Gruppe ausgewählt sind, die aus Fluor, OH, Oxo, C₁₋₄-Alkyl und Halogen-C₁₋₄-alkyl besteht;
A ein 6- bis 10-gliedriges mono- oder bicyclisches Aryl oder ein 5- bis 10-gliedriges mono- oder bicyclisches Heteroaryl ist, das 1 bis 5 Heteroatome enthält, die unabhängig aus der Gruppe, bestehend aus N, O und S, ausgewählt sind, wobei Aryl und Heteroaryl unsubstituiert oder mit einer oder zwei Gruppen substituiert sind, die unabhängig aus der Gruppe ausgewählt sind, die aus OH, Halogen, CN, O-C₁₋₆-Alkyl, O-Halogen-C₁₋₆-alkyl, C₁₋₆-Alkyl, Halogen-C₁₋₆-alkyl, C₃₋₆-Cycloalkyl und Halogen-C₃₋₆-cycloalkyl besteht;
B ein C₅₋₈-Cycloalkylring ist oder, falls Y N ist, B ein C₅₋₈-Heterocycloalkyl ist, das ein Stickstoffatom enthält, und wobei sich der Substituent Q nicht unmittelbar neben Substituent A befindet;
Q aus der Gruppe ausgewählt ist, die aus Phenyl, Pyridyl, Thiazolyl, Thiophenyl, Pyrimidyl, Oxazolyl, Pyrazolyl, Imidazolyl und Triazolyl besteht, jeweils unsubstituiert oder substituiert mit einer oder zwei Gruppen, die unabhängig aus der Gruppe ausgewählt sind, die aus Halogen, C₁₋₄-Alkyl, Halogen-C₁₋₄-alkyl, C₁₋₄-Alkoxy und Halogen-C₁₋₄-alkoxy besteht;
Y aus N, CH oder CF ausgewählt ist;
Z ausgewählt ist aus 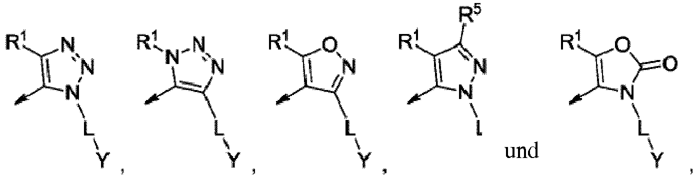 wobei
L aus der Gruppe ausgewählt ist, die aus einer Bindung, C₁₋₃-Alkylen und C₁₋₃-Alkylen-O- besteht;
Y aus Phenyl, Pyridyl, Pyridyl-*N*-oxid, Pyrimidyl, Pyridinonyl, Pyrimidinonyl, C₄₋₈-Cycloalkyl und C₄₋₈-Heterocycloalkyl ausgewählt ist, wobei Phenyl, Pyridyl, Pyridyl-*N-*oxid, Pyrimidyl, Pyridinonyl, Pyrimidinonyl, C₄₋₈-Cycloalkyl und C₄₋₈-Heterocycloalkyl mit R² und R³ substituiert sind und wahlweise ein- oder zweimal mit einer Gruppe substituiert sind, die aus Fluor, Chlor, CN, NH₂, NH(C₁₋₃-Alkyl), N(C₁₋₃-Alkyl)₂, C₁₋₃-Alkyl, Fluor-C₁₋₃-alkyl, OH, C₁₋₃-Alkoxy, Fluor-C₁₋₃-alkoxy, C₃₋₆-Cycloalkyl und Fluor-C₃₋₆-cycloalkyl ausgewählt ist;
R¹ aus der Gruppe ausgewählt ist, die aus C₁₋₄-Alkyl und C₃₋₆-Cycloalkyl besteht, wobei C₁₋₄-Alkyl unsubstituiert oder mit 1 bis 3 Substituenten substituiert ist, die unabhängig aus der Gruppe ausgewählt sind, die aus Fluor, Hydroxy, C₁₋₃-Alkoxy und Fluor-C₁₋₃-alkoxy besteht, und C₃₋₆-Cycloalkyl unsubstituiert oder mit 1 bis 3 Substituenten substituiert ist, die unabhängig aus der Gruppe ausgewählt sind, die aus Fluor, Hydroxy, C₁₋₃-Alkyl, Fluor-C₁₋₃-alkyl, C₁₋₃-Alkoxy und Fluor-C₁₋₃-alkoxy besteht;
R² und R³ unabhängig aus der Gruppe ausgewählt sind, die aus Wasserstoff, Halogen, C₁₋₃-Alkyl, Halogen-C₁₋₃-alkyl, C₁₋₃-Alkoxy, Halogen-C₁₋₃-alkoxy, Cyclopropyl und Fluor-cyclopropyl besteht;
R⁴ unabhängig aus Halogen, C₁₋₃-Alkyl, Halogen-C₁₋₃-alkyl, C₁₋₃-Alkoxy, Halogen-C₁₋₃-alkoxy, C₃₋₆-Cycloalkyl und Fluor-C₃₋₆-cycloalkyl ausgewählt ist;
R⁵ aus der Gruppe ausgewählt ist, die aus Wasserstoff, Fluor, CH₃, CHF₂ und CF₃ besteht;
n aus 0, 1, 2, 3 und 4 ausgewählt ist; und
x aus 0, 1 und 2 ausgewählt ist.

3. Verbindung nach Anspruch 1 oder 2, wobei
R aus der Gruppe ausgewählt ist, die aus CO₂H, SO₃H, CONR⁷R⁸, Tetrazolyl, 1,2,4-Oxadiazol-5(4H)-on-3-yl und SO₂NHCOR⁷ besteht;
R⁷ aus der Gruppe ausgewählt ist, die aus Wasserstoff, C₁₋₆-Alkyl, Halogen-C₁₋₆-alkyl und C₁₋₆-Alkylen-R⁹ besteht;
R⁸ aus der Gruppe ausgewählt ist, die aus Wasserstoff, C₁₋₆-Alkyl und Halogen-C₁₋₆-alkyl besteht; und
R⁹ aus der Gruppe ausgewählt ist, die aus COOH, OH und SO₃H besteht.

4. Verbindung nach einem der Ansprüche 1 bis 3, wobei
A aus der Gruppe ausgewählt ist, die aus Phenyl, Pyridyl, Pyrimidyl, Pyrazolyl, Indolyl, Thienyl, Benzothienyl, Indazolyl, Benzisoxazolyl, Benzofuranyl, Benzotriazolyl, Furanyl, Benzothiazolyl, Thiazolyl, Oxadiazolyl, Oxazolyl, Naphthyl, Chinolyl, Isochinolyl und Benzimidazolyl besteht, jeweils unsubstituiert oder substituiert mit einer oder zwei Gruppen, die unabhängig aus der Gruppe ausgewählt sind, die aus OH, Halogen, CN, O-C₁₋₆-Alkyl, O-Halogen-C₁₋₆-alkyl, C₁₋₆-Alkyl, Halogen-C₁₋₆-alkyl, C₃₋₆-Cycloalkyl und Halogen-C₃₋₆-cycloalkyl besteht.

5. Verbindung nach einem der Ansprüche 1 bis 4, wobei R-A ausgewählt ist aus

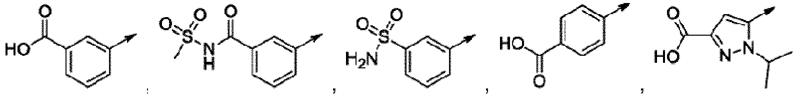

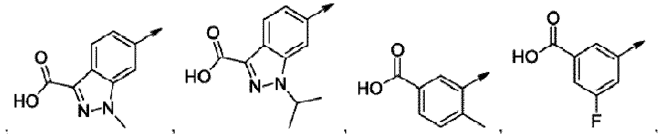

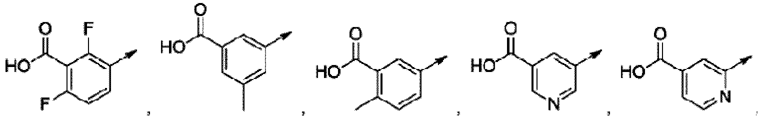

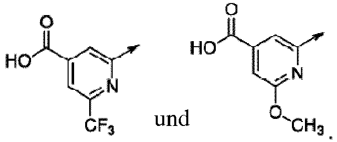

6. Verbindung nach einem der Ansprüche 1 bis 5, wobei Z ausgewählt ist aus 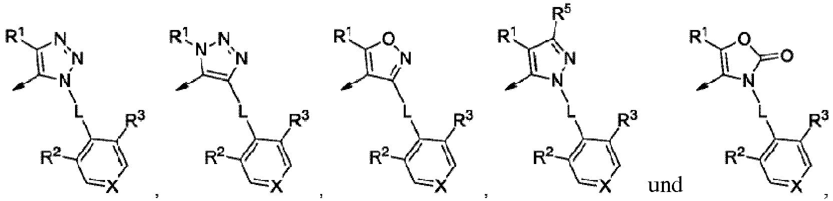 wobei
L aus der Gruppe ausgewählt ist, die aus einer Bindung, C₁₋₃-Alkylen und C₁₋₃-Alkylen-O- besteht;
X aus der Gruppe ausgewählt ist, die aus CH, CF, N und NO besteht;
R¹ aus der Gruppe ausgewählt ist, die aus C₁₋₄-Alkyl und C₃₋₆-Cycloalkyl besteht, wobei C₁₋₄-Alkyl unsubstituiert oder mit 1 bis 3 Substituenten substituiert ist, die unabhängig aus der Gruppe ausgewählt sind, die aus Fluor, Hydroxy, C₁₋₃-Alkoxy und Fluor-C₁₋₃-alkoxy besteht, und C₃₋₆-Cycloalkyl unsubstituiert oder mit 1 bis 3 Substituenten substituiert ist, die unabhängig aus der Gruppe ausgewählt sind, die aus Fluor, Hydroxy, C₁₋₃-Alkyl, Fluor-C₁₋₃-alkyl, C₁₋₃-Alkoxy und Fluor-C₁₋₃-alkoxy besteht;
R² und R³ unabhängig aus der Gruppe ausgewählt sind, die aus Wasserstoff, Halogen, C₁₋₃-Alkyl, Halogen-C₁₋₃-alkyl, C₁₋₃-Alkoxy, Halogen-C₁₋₃-alkoxy, Cyclopropyl und Fluor-cyclopropyl besteht; und
R⁵ aus der Gruppe ausgewählt ist, die aus Wasserstoff, Fluor, CH₃, CHF₂ und CF₃ besteht.

7. Verbindung nach einem der Ansprüche 1 bis 6, wobei Z ausgewählt ist aus 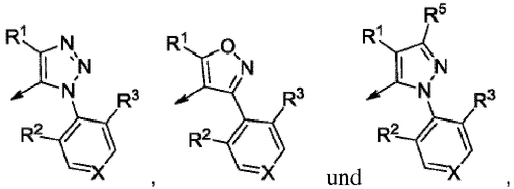 wobei
X aus der Gruppe ausgewählt ist, die aus CH, CF, N und NO besteht;
R¹ aus der Gruppe ausgewählt ist, die aus CF₃, CHF₂, Isopropyl und Cyclopropyl besteht, wobei Isopropyl und Cyclopropyl unsubstituiert oder mit einem oder zwei Fluor oder einem Hydroxy substituiert sind;
R² aus der Gruppe ausgewählt ist, die aus Fluor, Chlor, CH₃, CHF₂, CF₃, OCHF₂ und OCF₃ besteht;
R³ aus der Gruppe ausgewählt ist, die aus Wasserstoff, Fluor, Chlor, CH₃, CHF₂, CF₃, OCHF₂ und OCF₃ besteht; und
R⁵ aus der Gruppe ausgewählt ist, die aus Wasserstoff, Fluor, CH₃, CHF₂ und CF₃ besteht.

8. Verbindung nach einem der Ansprüche 1 bis 7, wobei 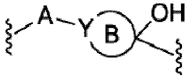 ausgewählt ist aus 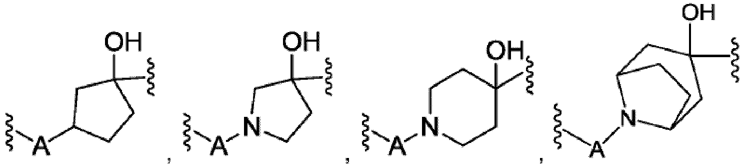 und

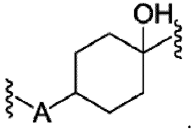

9. Verbindung nach einem der Ansprüche 1 bis 8, mit Formel (2) 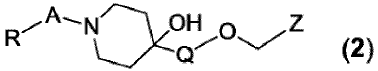 wobei
A aus der Gruppe ausgewählt ist, die aus Phenyl, Pyridyl, Pyrimidyl, Pyrazolyl, Indolyl, Thienyl, Benzothienyl, Indazolyl, Benzisoxazolyl, Benzofuranyl, Benzotriazolyl, Furanyl, Benzothiazolyl, Thiazolyl, Oxadiazolyl, Oxazolyl, Naphthyl, Chinolyl, Isochinolyl und Benzimidazolyl besteht, jeweils unsubstituiert oder substituiert mit einer oder zwei Gruppen, die unabhängig aus der Gruppe ausgewählt sind, die aus OH, Halogen, CN, O-C₁₋₆-Alkyl, O-Halogen-C₁₋₆-alkyl, C₁₋₆-Alkyl, Halogen-C₁₋₆-alkyl, C₃₋₆-Cycloalkyl und Halogen-C₃₋₆-cycloalkyl besteht;
R aus der Gruppe ausgewählt ist, die aus CO₂H, SO₃H, CONR⁷R⁸, Tetrazolyl, 1,2,4-Oxadiazol-5(4H)-on-3-yl und SO₂NHCOR⁷ besteht, wobei
R⁷ aus der Gruppe ausgewählt ist, die aus Wasserstoff, C₁₋₆-Alkyl, Halogen-C₁₋₆-alkyl und C₁₋₆-Alkylen-R⁹ besteht;
R⁸ aus der Gruppe ausgewählt ist, die aus Wasserstoff, C₁₋₆-Alkyl und Halogen-C₁₋₆-alkyl besteht; und
R⁹ aus der Gruppe ausgewählt ist, die aus COOH, OH und SO₃H besteht;
Q aus der Gruppe ausgewählt ist, die aus Phenyl, Pyridyl, Thiazolyl, Thiophenyl und Pyrimidyl besteht, jeweils unsubstituiert oder substituiert mit einer oder zwei Gruppen, die unabhängig aus der Gruppe ausgewählt sind, die aus Fluor, Chlor, CH₃, CHF₂ und CF₃ besteht;
Z ausgewählt ist aus 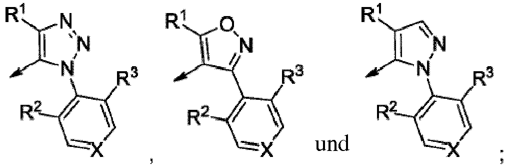
X aus der Gruppe ausgewählt ist, die aus CH, N und NO besteht;
R¹ aus der Gruppe ausgewählt ist, die aus Isopropyl und Cyclopropyl besteht, wobei Isopropyl und Cyclopropyl unsubstituiert oder mit einem oder zwei Fluor oder einem Hydroxy substituiert sind;
R² aus der Gruppe ausgewählt ist, die aus Fluor, Chlor, CH₃, CHF₂, CF₃, OCHF₂ und OCF₃ besteht; und
R³ aus der Gruppe ausgewählt ist, die aus Wasserstoff, Fluor, Chlor, CH₃, CHF₂, CF₃, OCHF₂ und OCF₃ besteht.

10. Verbindung nach einem der Ansprüche 1 bis 8, mit Formel (3) 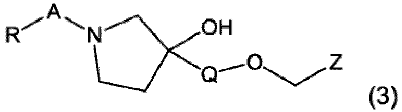 wobei
A aus der Gruppe ausgewählt ist, die aus Phenyl, Pyridyl, Pyrimidyl, Pyrazolyl, Indolyl, Thienyl, Benzothienyl, Indazolyl, Benzisoxazolyl, Benzofuranyl, Benzotriazolyl, Furanyl, Benzothiazolyl, Thiazolyl, Oxadiazolyl, Oxazolyl, Naphthyl, Chinolyl, Isochinolyl und Benzimidazolyl besteht, jeweils unsubstituiert oder substituiert mit einer oder zwei Gruppen, die unabhängig aus der Gruppe ausgewählt sind, die aus OH, Halogen, CN, O-C₁₋₆-Alkyl, O-Halogen-C₁₋₆-alkyl, C₁₋₆-Alkyl, Halogen-C₁₋₆-alkyl, C₃₋₆-Cycloalkyl und Halogen-C₃₋₆-cycloalkyl besteht;
R aus der Gruppe ausgewählt ist, die aus CO₂H, SO₃H, CONR⁷R⁸, Tetrazolyl, 1,2,4-Oxadiazol-5(4H)-on-3-yl und SO₂NHCOR⁷ besteht, wobei
R⁷ aus der Gruppe ausgewählt ist, die aus Wasserstoff, C₁₋₆-Alkyl, Halogen-C₁₋₆-alkyl und C₁₋₆-Alkylen-R⁹ besteht;
R⁸ aus der Gruppe ausgewählt ist, die aus Wasserstoff, C₁₋₆-Alkyl und Halogen-C₁₋₆-alkyl besteht; und
R⁹ aus der Gruppe ausgewählt ist, die aus COOH, OH und SO₃H besteht;
Q aus der Gruppe ausgewählt ist, die aus Phenyl, Pyridyl, Thiazolyl, Thiophenyl und Pyrimidyl besteht, jeweils unsubstituiert oder substituiert mit einer oder zwei Gruppen, die unabhängig aus der Gruppe ausgewählt sind, die aus Fluor, Chlor, CH₃, CHF₂ und CF₃ besteht;
Z ausgewählt ist aus 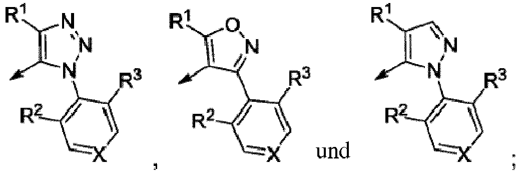
X aus der Gruppe ausgewählt ist, die aus CH, N und NO besteht;
R¹ aus der Gruppe ausgewählt ist, die aus Isopropyl und Cyclopropyl besteht, wobei Isopropyl und Cyclopropyl unsubstituiert oder mit einem oder zwei Fluor oder einem Hydroxy substituiert sind;
R² aus der Gruppe ausgewählt ist, die aus Fluor, Chlor, CH₃, CHF₂, CF₃, OCHF₂ und OCF₃ besteht; und
R³ aus der Gruppe ausgewählt ist, die aus Wasserstoff, Fluor, Chlor, CH₃, CHF₂, CF₃, OCHF₂ und OCF₃ besteht.

11. Verbindung nach Anspruch 1, die aus der Gruppe ausgewählt ist, die besteht aus: 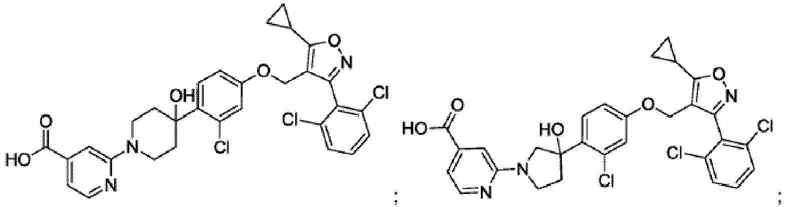 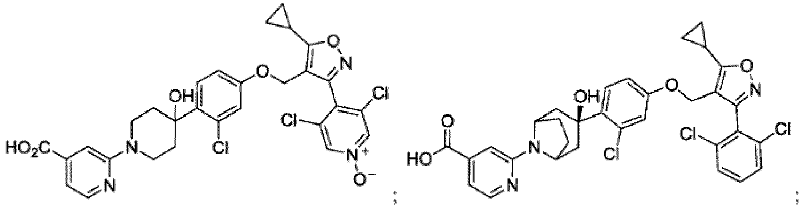 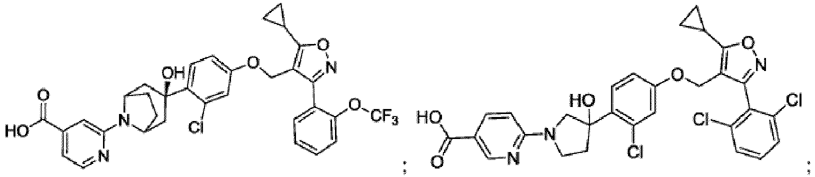 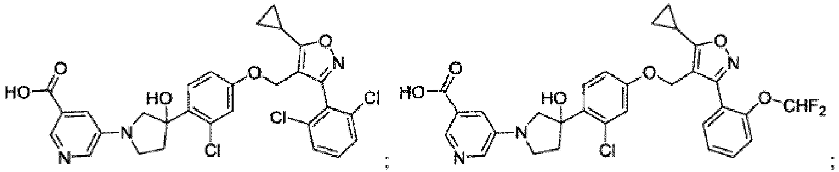 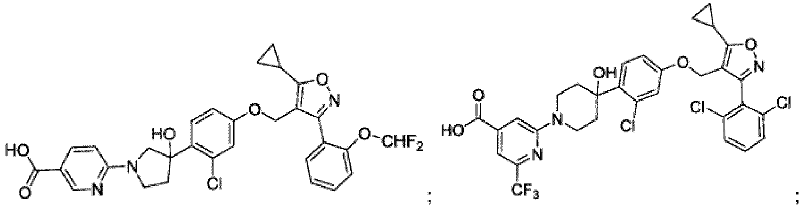 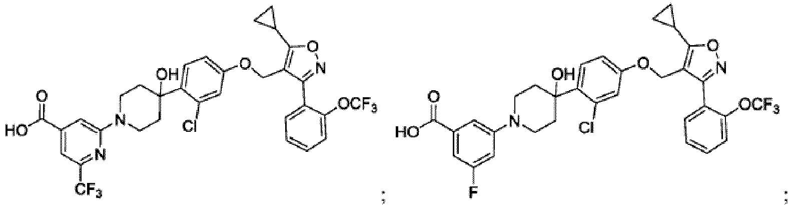 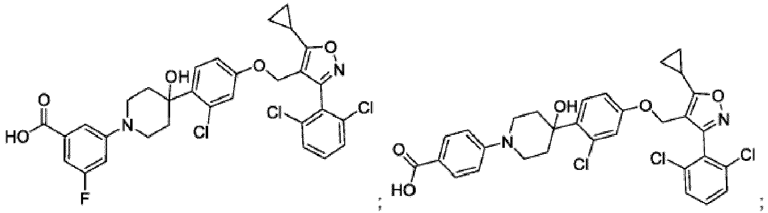 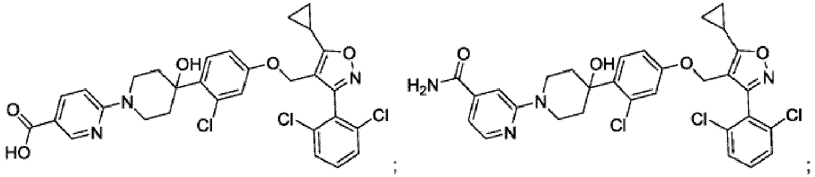 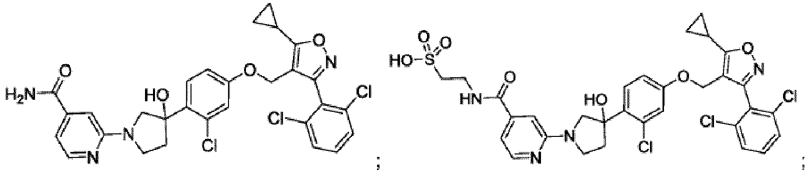 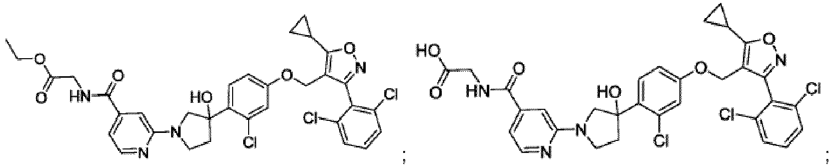 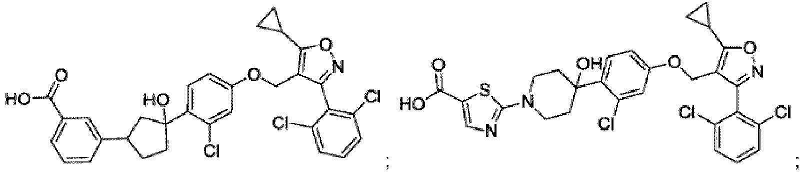 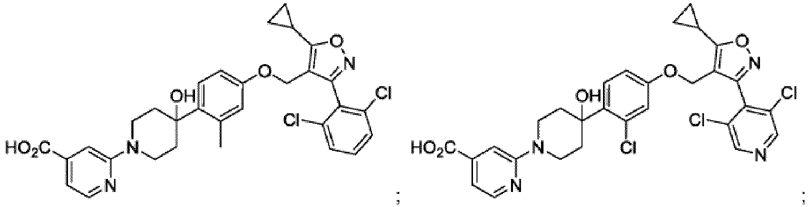 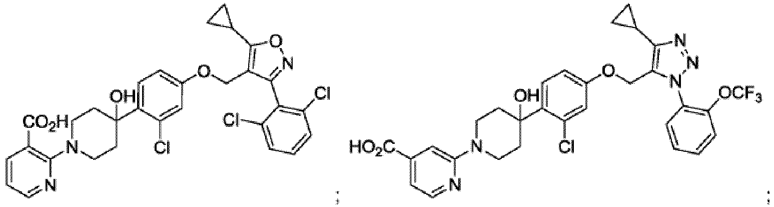 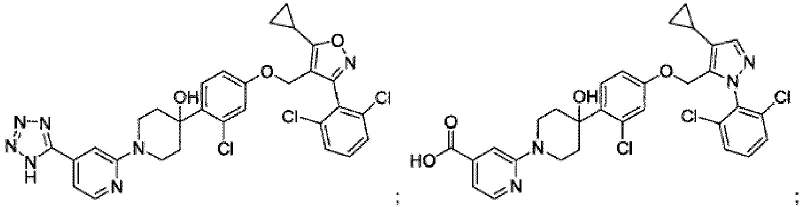 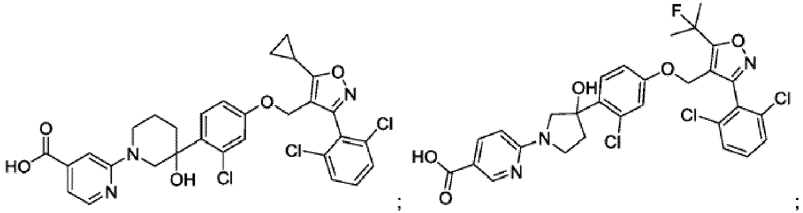 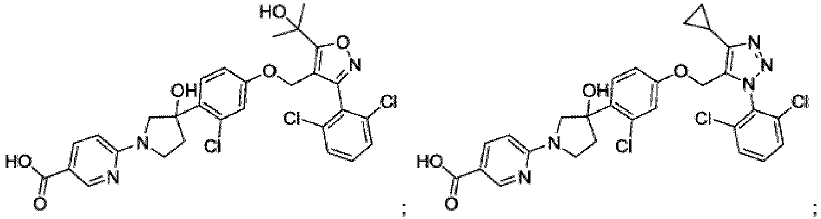 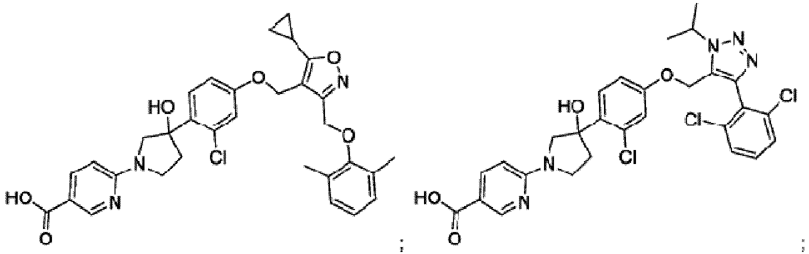 und 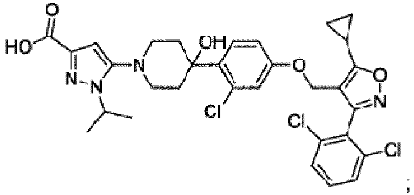 oder ein Enantiomer, Diastereomer, Tautomer, Solvat oder pharmazeutisch verträgliches Salz davon.

12. Verbindung nach einem der Ansprüche 1 bis 11, zur Verwendung als Medikament.

13. Verbindung nach einem der Ansprüche 1 bis 11, zur Verwendung in der Verhütung und/oder Behandlung von durch FXR vermittelten Erkrankungen.

14. Verbindung zur Verwendung nach Anspruch 13, wobei die Erkrankung ausgewählt ist aus
chronischen intrahepatischen oder einigen Formen von extrahepatischen cholestatischen Leiden;
Leberfibrose;
obstruktiven oder chronisch entzündlichen Störungen der Leber;
Leberzirrhose;
Lebersteatose und assoziierten Syndromen, cholestatischen oder fibrotischen Auswirkungen, die mit alkoholinduzierter Zirrhose oder mit durch Viren übertragenen Formen von Hepatitis assoziiert sind;
Leberinsuffizienz oder Leberischämie nach großer Leberresektion;
Chemotherapie-assoziierter Steatohepatitis (CASH);
akuter Leberinsuffizienz; und
chronisch entzündlichen Darmerkrankungen.

15. Verbindung zur Verwendung nach Anspruch 13, wobei die Erkrankung ausgewählt ist aus
Lipid- und Lipoproteinstörungen;
Typ-II-Diabetes und klinischen Komplikationen von Typ-I- und Typ-II-Diabetes, einschließlich diabetischer Nephropathie, diabetischer Neuropathie, diabetischer Retinopathie und sonstiger beobachteter Auswirkungen von klinisch manifestem Langzeitdiabetes ;
Leiden und Erkrankungen, die aus chronischer fettiger und fibrotischer Degeneration von Organen infolge forcierter Lipid- und speziell Triglyceridakkumulation und anschließender Aktivierung profibrotischer Wege resultieren, wie z. B. nicht alkoholischer Fettlebererkrankung (NAFLD) oder nicht alkoholischer Steatohepatitis (NASH);
Obesitas oder metabolischem Syndrom (Kombinationsleiden von Dyslipidämie, Diabetes oder abnorm hohem Body-Mass-Index); und
akutem Herzinfarkt, akutem Schlaganfall oder Thrombose, der bzw. die als Endpunkt chronisch obstruktiver Atherosklerose eintritt.

16. Verbindung zur Verwendung nach Anspruch 13, wobei die Erkrankung ausgewählt ist aus
nicht malignen hyperproliferativen Störungen und malignen hyperproliferativen Störungen, speziell hepatozellulärem Karzinom, Kolonadenom und Polyposis, Adenokarzinom des Kolons, Brustkrebs, Adenokarzinom der Bauchspeicheldrüse, Barrett-Ösophagus oder sonstigen Formen neoplastischer Erkrankungen des Magen-Darm-Trakts und der Leber.

## Revendications

1. Composé selon la Formule (1) suivante, un énantiomère, un diastéréoisomère, un tautomère, un solvate ou un sel pharmaceutiquement acceptable de celui-ci: 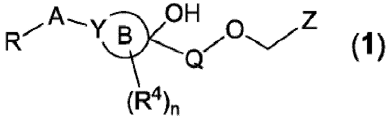 dans lequel:
R est choisi dans le groupe constitué de: hydrogène, halogène, C₁₋₆ alkyle, C₂₋₆ alcényle, C₂₋₆ alcynyle, halo-C₁₋₆ alkyle, C₀₋₆ alkylène-R⁷, C₀₋₆ alkylène-O-R⁷, C₀₋₆ alkylène-CN, C₀₋₆ alkylène-NR⁷R⁸, O-C₃₋₁₀ cycloalkyle, O-C₁₋₆ alkylène-O-R⁷, O-C₃₋₁₀ hétérocycloalkyle, C₀₋₆ alkylène-CO₂R⁷, C₀₋₆ alkylène-C(O)R⁷, C₀₋₆ alkylène-C(O)NR⁷R⁸, C₀₋₆ alkylène-C(O)NR⁷SO₂R⁷, C₀₋₆ alkylène-N(R⁷)C(O)R⁷, C₀₋₆ alkylène-SOₓ-R⁷, C₀₋₆ alkylène-SO₃H, C₀₋₆ alkylène-SO₂-NR⁷R⁸, C₀₋₆ alkylène-SO₂-NR⁸COR⁷, C₀₋₆ alkylène-N(R⁷)SO₂-R⁸ et C₀₋₆ alkylène-SO₂-C₃₋₁₀ hétérocycloalkyle,
dans lequel l'alkylène, le cycloalkyle, l'hétérocycloalkyle et l'hétéroaryle de 5 ou 6 membres sont non substitués ou substitués par 1 à 4 substituants indépendamment choisis dans le groupe constitué de: halogène, CN, C₁₋₃ alkyle, halo-C₁₋₃ alkyle, OH, oxo, CO₂H, SO₃H, O-C₁₋₃ alkyle et O-halo-C₁₋₃ alkyle;
R⁷ est indépendamment choisi dans le groupe constitué de: hydrogène, C₁₋₆ alkyle, halo-C₁₋₆ alkyle, C₀₋₆ alkylène-C₃₋₈ cycloalkyle, C₀₋₆ alkylène-C₃₋₈ hétérocycloalkyle, hétéroaryle de 5 ou 6 membres et phényle, dans lequel l'alkyle, l'alkylène, le cycloalkyle, l'hétérocycloalkyle, le phényle et l'hétéroaryle sont non substitués ou substitués avec 1 à 6 substituants indépendamment choisis dans le groupe constitué de: halogène, CN, OH, oxo, CO₂H, C₁₋₃ alkyle, halo-C₁₋₃ alkyle, O-C₁₋₃ alkyle, O-halo-C₁₋₃ alkyle, SO₃H et SO₂-C₁₋₃ alkyle;
R⁸ est indépendamment choisi dans le groupe constitué de: hydrogène, C₁₋₆ alkyle, halo-C₁₋₆ alkyle et C₃₋₆ cycloalkyle;
ou R⁷ et R⁸ lorsqu'ils sont pris ensemble avec l'azote auquel ils sont attachés peuvent terminer un cycle de 3 à 8 membres contenant des atomes de carbone et contenant optionnellement 1 ou 2 hétéroatomes choisis parmi O, S ou N, dans lequel le cycle est non substitué ou substitué avec 1 à 4 substituants indépendamment choisis dans le groupe constitué de: fluoro, OH, oxo, C₁₋₄ alkyle et halo-C₁₋₄ alkyle;
A est un aryle mono- ou bicyclique de 6-10 membres ou un hétéroaryle mono- ou bicyclique de 5-10 membres contenant 1 à 5 hétéroatomes indépendamment choisis dans le groupe constitué de N, O et S, dans lequel l'aryle et l'hétéroaryle sont non substitués ou substitués avec un ou deux groupes indépendamment choisis dans le groupe constitué de: OH, halogène, CN, O-C₁₋₆ alkyle, O-halo-C₁₋₆ alkyle, C₁₋₆ alkyle, halo-C₁₋₆ alkyle, C₃₋₆ cycloalkyle et halo-C₃₋₆ cycloalkyle;
B est un cycle C₅₋₈ cycloalkyle ou, si Y est N, alors B est un C₄₋₈ hétérocycloalkyle ou un C₄₋₈ hétérocycloalkyle ponté contenant un atome d'azote et dans lequel le substituant Q n'est pas directement adjacent au substituant A;
Q est choisi dans le groupe constitué de: phényle, pyridyle, thiazolyle, thiophényle, pyrimidyle, oxazolyle, pyrazolyle, imidazolyle et triazolyle, chacun étant non substitué ou substitué avec un ou deux groupes indépendamment choisis dans le groupe constitué de: halogène, C₁₋₄ alkyle, halo-C₁₋₄ alkyle, C₁₋₄ alcoxy et halo-C₁₋₄ alcoxy;
Y est choisi parmi N, CH ou CF;
Z est choisi parmi: 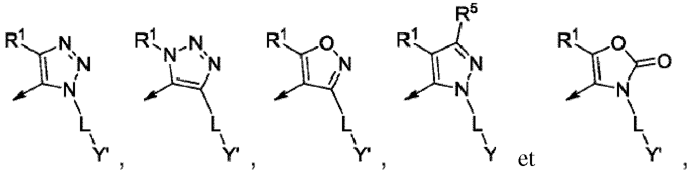 dans lequel:
L est choisi dans le groupe constitué de: une liaison, C₁₋₃ alkylène et C₁₋₃ alkylène-O-;
Y' est choisi parmi: phényle, pyridyle, pyridyl-N-oxyde, pyrimidyle, pyridinonyle, pyrimidinonyle, C₄₋₈ cycloalkyle et C₄₋₈ hérérocycloalkyle, dans lequel le phényle, le pyridyle, le pyridyl-N-oxyde, le pyrimidyle, le pyridinonyle, le pyrimidinonyle, le C₄₋₈ cycloalkyle et le C₄₋₈ hérérocycloalkyle sont substitués avec R² et R³ et optionnellement substitués une ou deux fois avec un groupe choisi parmi: fluoro, chloro, CN, NH₂, NH(C₁₋₃ alkyle), N(C₁₋₃ alkyle)₂, C₁₋₃ alkyle, fluoro-C₁₋₃ alkyle, OH, C₁₋₃ alcoxy, fluoro-C₁₋₃ alcoxy, C₃₋₆ cycloalkyle et fluoro-C₃₋₆ cycloalkyle;
R¹ est choisi dans le groupe constitué de: C₁₋₄ alkyle et C₃₋₆ cycloalkyle, dans lequel le C₁₋₄ alkyle est non substitué ou substitué avec 1 à 3 substituants indépendamment choisis dans le groupe constitué de: fluoro, hydroxy, C₁₋₃ alcoxy et fluoro-C₁₋₃ alcoxy, et le C₃₋₆ cycloalkyle est non substitué ou substitué avec 1 à 3 substituants indépendamment choisis dans le groupe constitué de: fluoro, hydroxy, C₁₋₃ alkyle, fluoro-C₁₋₃ alkyle, C₁₋₃ alcoxy et fluoro-C₁₋₃ alcoxy;
R² et R³ sont indépendamment choisis dans le groupe constitué de: hydrogène, halogène, C₁₋₃ alkyle, halo-C₁₋₃ alkyle, C₁₋₃ alcoxy, halo-C₁₋₃ alcoxy, cyclopropyle et fluoro-cyclopropyle;
R⁴ est indépendamment choisi parmi: halogène, C₁₋₃ alkyle, halo-C₁₋₃ alkyle, C₁₋₃ alcoxy, halo-C₁₋₃ alcoxy, C₃₋₆ cycloalkyle et fluoro-C₃₋₆ cycloalkyle;
R⁵ est choisi dans le groupe constitué de: hydrogène, fluoro, CH₃, CHF₂ et CF₃;
n est choisi parmi 0, 1, 2, 3 et 4; et
x est choisi parmi 0, 1 et 2.

2. Composé selon la revendication 1, dans lequel:
R est choisi dans le groupe constitué de: hydrogène, halogène, C₁₋₆ alkyle, C₂₋₆ alcényle, C₂₋₆ alcynyle, halo-C₁₋₆ alkyle, C₀₋₆ alkylène-O-R⁷, C₀₋₆ alkylène-CN, C₀₋₆ alkylène-NR⁷R⁸, O-C₃₋₁₀ cycloalkyle, O-C₁₋₆ alkylène-O-R⁷, O-C₃₋₁₀ hétérocycloalkyle, C₀₋₆ alkylène-CO₂R⁷, C₀₋₆ alkylène-C(O)R⁷, C₀₋₆ alkylène-C(O)NR⁷R⁸, C₀₋₆ alkylène-C(O)NR⁷SO₂R⁷, C₀₋₆ alkylène-N(R⁷)C(O)R⁷, C₀₋₆ alkylène-SOₓ-R⁷, C₀₋₆ alkylène-SO₃H, C₀₋₆ alkylène-SO₂-NR⁷R⁸, C₀₋₆ alkylène-SO₂-NR⁸COR⁷, C₀₋₆ alkylène-N(R⁷)SO₂-R⁸ et C₀₋₆ alkylène-SO₂-C₃₋₁₀ hétérocycloalkyle,
dans lequel l'alkylène, le cycloalkyle, l'hétérocycloalkyle et l'hétéroaryle de 5 ou 6 membres sont non substitués ou substitués par 1 à 4 substituants indépendamment choisis dans le groupe constitué de: halogène, CN, C₁₋₃ alkyle, halo-C₁₋₃ alkyle, OH, oxo, CO₂H, SO₃H, O-C₁₋₃ alkyle et O-halo-C₁₋₃ alkyle;
R⁷ est indépendamment choisi dans le groupe constitué de: hydrogène, C₁₋₆ alkyle, halo-C₁₋₆ alkyle, C₀₋₆ alkylène-C₃₋₈ cycloalkyle, C₀₋₆ alkylène-C₃₋₈ hétérocycloalkyle, hétéroaryle de 5 ou 6 membres et phényle, dans lequel l'alkyle, l'alkylène, le cycloalkyle, l'hétérocycloalkyle, le phényle et l'hétéroaryle sont non substitués ou substitués avec 1 à 6 substituants indépendamment choisis dans le groupe constitué de: halogène, CN, OH, oxo, CO₂H, C₁₋₃ alkyle, halo-C₁₋₃ alkyle, O-C₁₋₃ alkyle, O-halo-C₁₋₃ alkyle, SO₃H et SO₂-C₁₋₃ alkyle;
R⁸ est indépendamment choisi dans le groupe constitué de: hydrogène, C₁₋₆ alkyle, halo-C₁₋₆ alkyle et C₃₋₆ cycloalkyle;
ou R⁷ et R⁸ lorsqu'ils sont pris ensemble avec l'azote auquel ils sont attachés peuvent terminer un cycle de 3 à 8 membres contenant des atomes de carbone et contenant optionnellement 1 ou 2 hétéroatomes choisis parmi O, S ou N, dans lequel le cycle est non substitué ou substitué avec 1 à 4 substituants indépendamment choisis dans le groupe constitué de: fluoro, OH, oxo, C₁₋₄ alkyle et halo-C₁₋₄ alkyle;
A est un aryle mono- ou bicyclique de 6-10 membres ou un hétéroaryle mono- ou bicyclique de 5-10 membres contenant 1 à 5 hétéroatomes indépendamment choisis dans le groupe constitué de N, O et S, dans lequel l'aryle et l'hétéroaryle sont non substitués ou substitués avec un ou deux groupes indépendamment choisis dans le groupe constitué de: OH, halogène, CN, O-C₁₋₆ alkyle, O-halo-C₁₋₆ alkyle, C₁₋₆ alkyle, halo-C₁₋₆ alkyle, C₃₋₆ cycloalkyle et halo-C₃₋₆ cycloalkyle;
B est un cycle C₅₋₈ cycloalkyle ou, si Y est N, alors B est un C₅₋₈ hétérocycloalkyle contenant un atome d'azote et dans lequel le substituant Q n'est pas directement adjacent au substituant A;
Q est choisi dans le groupe constitué de: phényle, pyridyle, thiazolyle, thiophényle, pyrimidyle, oxazolyle, pyrazolyle, imidazolyle et triazolyle, chacun étant non substitué ou substitué avec un ou deux groupes indépendamment choisis dans le groupe constitué de: halogène, C₁₋₄ alkyle, halo-C₁₋₄ alkyle, C₁₋₄ alcoxy et halo-C₁₋₄ alcoxy;
Y est choisi parmi N, CH ou CF;
Z est choisi parmi: 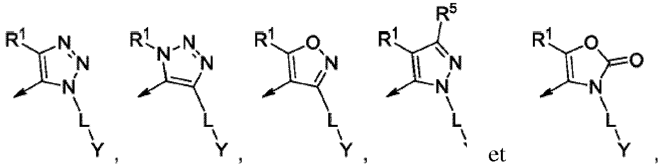 dans lequel:
L est choisi dans le groupe constitué de: une liaison, C₁₋₃ alkylène et C₁₋₃ alkylène-O-;
Y est choisi parmi: phényle, pyridyle, pyridyl-N-oxyde, pyrimidyle, pyridinonyle, pyrimidinonyle, C₄₋₈ cycloalkyle et C₄₋₈ hérérocycloalkyle, dans lequel le phényle, le pyridyle, le pyridyl-N-oxyde, le pyrimidyle, le pyridinonyle, le pyrimidinonyle, le C₄₋₈ cycloalkyle et le C₄₋₈ hérérocycloalkyle sont substitués avec R² et R³ et optionnellement substitués une ou deux fois avec un groupe choisi parmi: fluoro, chloro, CN, NH₂, NH(C₁₋₃ alkyle), N(C₁₋₃ alkyle)₂, C₁₋₃ alkyle, fluoro-C₁₋₃ alkyle, OH, C₁₋₃ alcoxy, fluoro-C₁₋₃ alcoxy, C₃₋₆ cycloalkyle et fluoro-C₃₋₆ cycloalkyle;
R¹ est choisi dans le groupe constitué de: C₁₋₄ alkyle et C₃₋₆ cycloalkyle, dans lequel le C₁₋₄ alkyle est non substitué ou substitué avec 1 à 3 substituants indépendamment choisis dans le groupe constitué de: fluoro, hydroxy, C₁₋₃ alcoxy et fluoro-C₁₋₃ alcoxy, et le C₃₋₆ cycloalkyle est non substitué ou substitué avec 1 à 3 substituants indépendamment choisis dans le groupe constitué de: fluoro, hydroxy, C₁₋₃ alkyle, fluoro-C₁₋₃ alkyle, C₁₋₃ alcoxy et fluoro-C₁₋₃ alcoxy;
R² et R³ sont indépendamment choisis dans le groupe constitué de: hydrogène, halogène, C₁₋₃ alkyle, halo-C₁₋₃ alkyle, C₁₋₃ alcoxy, halo-C₁₋₃ alcoxy, cyclopropyle et fluoro-cyclopropyle;
R⁴ est indépendamment choisi parmi: halogène, C₁₋₃ alkyle, halo-C₁₋₃ alkyle, C₁₋₃ alcoxy, halo-C₁₋₃ alcoxy, C₃₋₆ cycloalkyle et fluoro-C₃₋₆ cycloalkyle;
R⁵ est choisi dans le groupe constitué de: hydrogène, fluoro, CH₃, CHF₂ et CF₃;
n est choisi parmi 0, 1, 2, 3 et 4; et
x est choisi parmi 0, 1 et 2.

3. Composé selon les revendications 1 ou 2, dans lequel:
R est choisi dans le groupe constitué de: CO₂H, SO₃H, CONR⁷R⁸, tétrazolyle, 1,2,4-oxadiazol-5(4H)-one-3-yle et SO₂NHCOR⁷ ;
R⁷ est choisi dans le groupe constitué de: hydrogène, C₁₋₆ alkyle, halo-C₁₋₆ alkyle et C₁₋₆ alkylène-R⁹;
R⁸ est choisi dans le groupe constitué de: hydrogène, C₁₋₆ alkyle et halo-C₁₋₆ alkyle; et
R⁹ est choisi dans le groupe constitué de: COOH, OH et SO₃H

4. Composé selon l'une quelconque des revendications 1 à 3, dans lequel:
A est choisi dans le groupe constitué de: phényle, pyridyle, pyrimidyle, pyrazolyle, indolyle, thiényle, benzothiényle, indazolyle, benzisoxazolyle, benzofuranyle, benzotriazolyle, furanyle, benzothiazolyle, thiazolyle, oxadiazolyle, oxazolyle, naphtyle, quinoléyle, isoquinoléyle et benzimidazolyle, chacun étant non substitué ou substitué avec un ou deux groupes indépendamment choisis dans le groupe constitué de: OH, halogène, CN, O-C₁₋₆ alkyle, O-halo-C₁₋₆ alkyle, C₁₋₆ alkyle, halo-C₁₋₆ alkyle, C₃₋₆ cycloalkyle et halo-C₃₋₆ cycloalkyle.

5. Composé selon l'une quelconque des revendications 1 à 4, dans lequel R-A est choisi parmi:

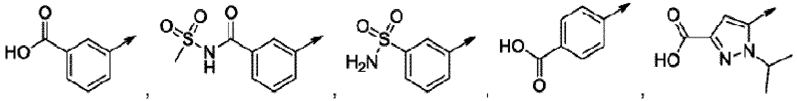

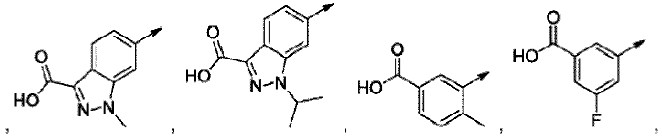

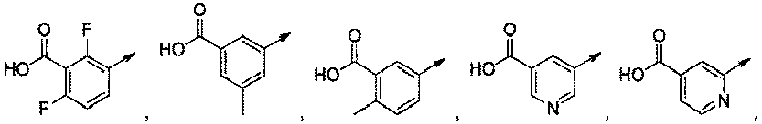

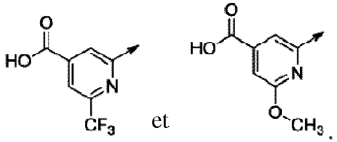

6. Composé selon l'une quelconque des revendications 1 à 5, dans lequel Z est choisi parmi: 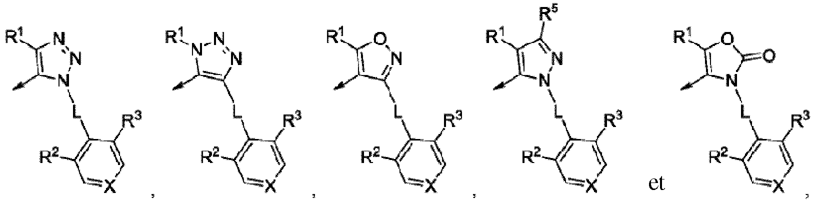 dans lequel:
L est choisi dans le groupe constitué de: une liaison, C₁₋₃ alkylène et C₁₋₃ alkylène-O-;
X est choisi dans le groupe constitué de: CH, CF, N et NO;
R¹ est choisi dans le groupe constitué de: C₁₋₄ alkyle et C₃₋₆ cycloalkyle, dans lequel le C₁₋₄ alkyle est non substitué ou substitué avec 1 à 3 substituants indépendamment choisis dans le groupe constitué de: fluoro, hydroxy, C₁₋₃ alcoxy et fluoro-C₁₋₃ alcoxy, et le C₃₋₆ cycloalkyle est non substitué ou substitué avec 1 à 3 substituants indépendamment choisis dans le groupe constitué de: fluoro, hydroxy, C₁₋₃ alkyle, fluoro-C₁₋₃ alkyle, C₁₋₃ alcoxy et fluoro-C₁₋₃ alcoxy;
R² et R³ sont indépendamment choisis dans le groupe constitué de: hydrogène, halogène, C₁₋₃ alkyle, halo-C₁₋₃ alkyle, C₁₋₃ alcoxy, halo-C₁₋₃ alcoxy, cyclopropyle et fluoro-cyclopropyle; et
R⁵ est choisi dans le groupe constitué de: hydrogène, fluoro, CH₃, CHF₂ et CF₃.

7. Composé selon l'une quelconque des revendications 1 à 6, dans lequel Z est choisi parmi: 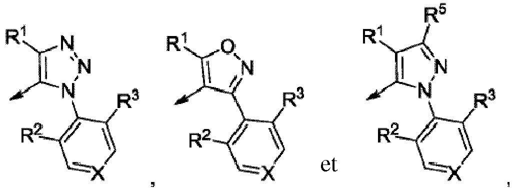 dans lequel:
X est choisi dans le groupe constitué de: CH, CF, N et NO;
R¹ est choisi dans le groupe constitué de: CF₃, CHF₂, isopropyle et cyclopropyle, dans lequel l'isopropyle et le cyclopropyle sont non substitués ou substitués avec un ou deux fluoro ou un hydroxy;
R² est choisi dans le groupe constitué de: fluoro, chloro, CH₃, CHF₂, CF₃, OCHF₂ et OCF₃;
R³ est choisi dans le groupe constitué de: hydrogène, fluoro, chloro, CH₃, CHF₂, CF₃, OCHF₂ et OCF₃; et
R⁵ est choisi dans le groupe constitué de: hydrogène, fluoro, CH₃, CHF₂ et CF₃.

8. Composé selon l'une quelconque des revendications 1 à 7, dans lequel 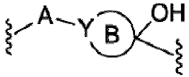 est choisi parmi: 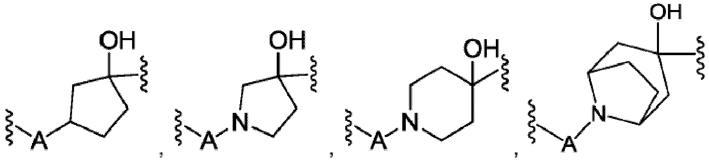 et

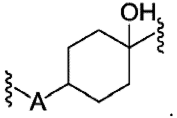

9. Composé selon l'une quelconque des revendications 1 à 8 avec la Formule (2): 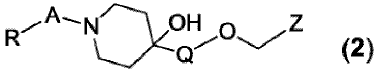 dans lequel:
A est choisi dans le groupe constitué de: phényle, pyridyle, pyrimidyle, pyrazolyle, indolyle, thiényle, benzothiényle, indazolyle, benzisoxazolyle, benzofuranyle, benzotriazolyle, furanyle, benzothiazolyle, thiazolyle, oxadiazolyle, oxazolyle, naphtyle, quinoléyle, isoquinoléyle et benzimidazolyle, chacun étant non substitué ou substitué avec un ou deux groupes indépendamment choisis dans le groupe constitué de: OH, halogène, CN, O-C₁₋₆ alkyle, O-halo-C₁₋₆ alkyle, C₁₋₆ alkyle, halo-C₁₋₆ alkyle, C₃₋₆ cycloalkyle et halo-C₃₋₆ cycloalkyle;
R est choisi dans le groupe constitué de: CO₂H, SO₃H, CONR⁷R⁸, tétrazolyle, 1,2,4-oxadiazol-5(4H)-one-3-yle et SO₂NHCOR⁷, dans lequel:
R⁷ est choisi dans le groupe constitué de: hydrogène, C₁₋₆ alkyle, halo-C₁₋₆ alkyle et C₁₋₆ alkylène-R⁹;
R⁸ est choisi dans le groupe constitué de: hydrogène, C₁₋₆ alkyle et halo-C₁₋₆ alkyle; et
R⁹ est choisi dans le groupe constitué de: COOH, OH et SO₃H;
Q est choisi dans le groupe constitué de: phényle, pyridyle, thiazolyle, thiophényle et pyrimidyle, chacun étant non substitué ou substitué avec un ou deux groupes indépendamment choisis dans le groupe constitué de: fluoro, chloro, CH₃, CHF₂ et CF₃;
Z est choisi parmi: 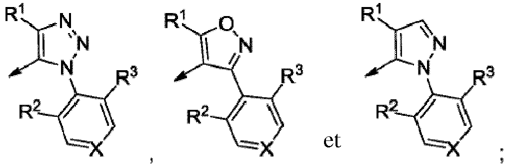 dans lequel:
X est choisi dans le groupe constitué de: CH, N et NO;
R¹ est choisi dans le groupe constitué de: isopropyle et cyclopropyle, dans lequel l'isopropyle et le cyclopropyle sont non substitués ou substitués avec un ou deux fluoro ou un hydroxy;
R² est choisi dans le groupe constitué de: fluoro, chloro, CH₃, CHF₂, CF₃, OCHF₂ et OCF₃; et
R³ est choisi dans le groupe constitué de: hydrogène, fluoro, chloro, CH₃, CHF₂, CF₃, OCHF₂ et OCF₃.

10. Composé selon l'une quelconque des revendications 1 à 8 avec la Formule (3): 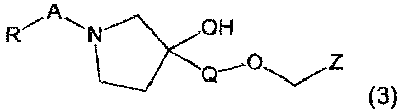 dans lequel:
A est choisi dans le groupe constitué de: phényle, pyridyle, pyrimidyle, pyrazolyle, indolyle, thiényle, benzothiényle, indazolyle, benzisoxazolyle, benzofuranyle, benzotriazolyle, furanyle, benzothiazolyle, thiazolyle, oxadiazolyle, oxazolyle, naphtyle, quinoléyle, isoquinoléyle et benzimidazolyle, chacun étant non substitué ou substitué avec un ou deux groupes indépendamment choisis dans le groupe constitué de: OH, halogène, CN, O-C₁₋₆ alkyle, O-halo-C₁₋₆ alkyle, C₁₋₆ alkyle, halo-C₁₋₆ alkyle, C₃₋₆ cycloalkyle et halo-C₃₋₆ cycloalkyle;
R est choisi dans le groupe constitué de: CO₂H, SO₃H, CONR⁷R⁸, tétrazolyle, 1,2,4-oxadiazol-5(4H)-one-3-yle et SO₂NHCOR⁷, dans lequel:
R⁷ est choisi dans le groupe constitué de: hydrogène, C₁₋₆ alkyle, halo-C₁₋₆ alkyle et C₁₋₆ alkylène-R⁹;
R⁸ est choisi dans le groupe constitué de: hydrogène, C₁₋₆ alkyle et halo-C₁₋₆ alkyle; et
R⁹ est choisi dans le groupe constitué de: COOH, OH et SO₃H;
Q est choisi dans le groupe constitué de: phényle, pyridyle, thiazolyle, thiophényle et pyrimidyle, chacun étant non substitué ou substitué avec un ou deux groupes indépendamment choisis dans le groupe constitué de: fluoro, chloro, CH₃, CHF₂ et CF₃;
Z est choisi parmi: 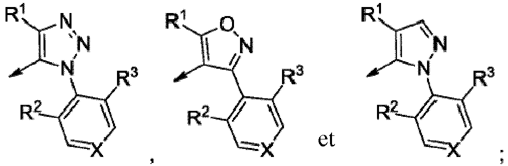 dans lequel:
X est choisi dans le groupe constitué de: CH, N et NO;
R¹ est choisi dans le groupe constitué de: isopropyle et cyclopropyle, dans lequel l'isopropyle et le cyclopropyle sont non substitués ou substitués avec un ou deux fluoro ou un hydroxy;
R² est choisi dans le groupe constitué de: fluoro, chloro, CH₃, CHF₂, CF₃, OCHF₂ et OCF₃; et
R³ est choisi dans le groupe constitué de: hydrogène, fluoro, chloro, CH₃, CHF₂, CF₃, OCHF₂ et OCF₃.

11. Composé selon la revendication 1 choisi dans le groupe constitué de: 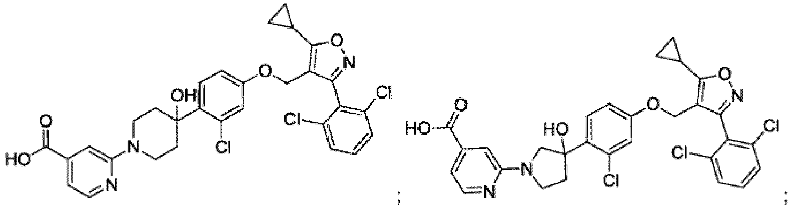 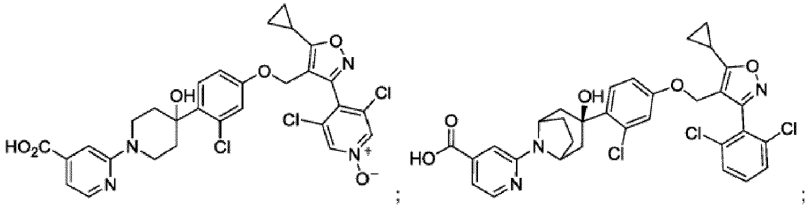 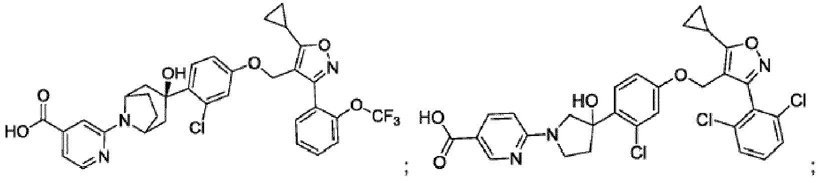 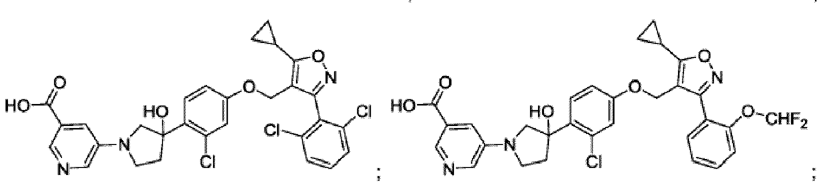 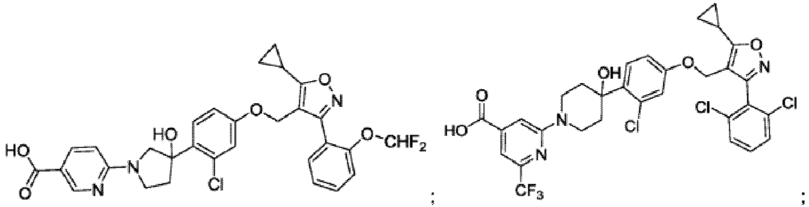 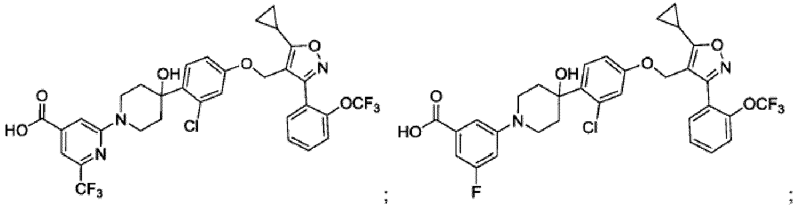 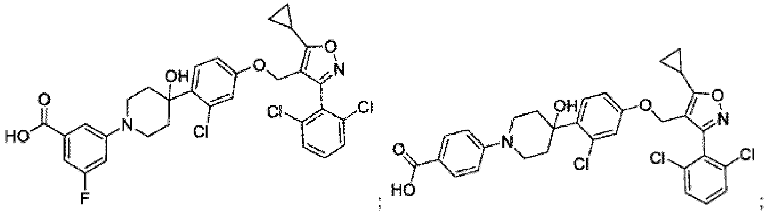 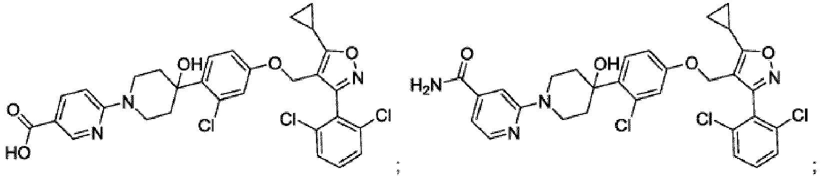 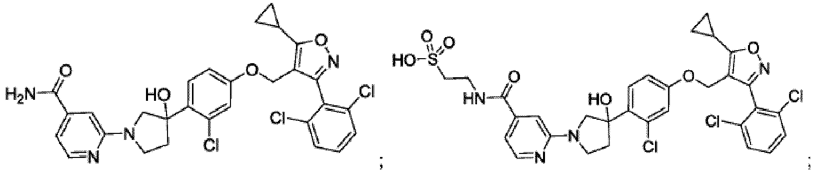 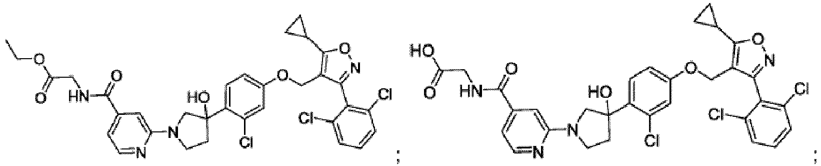 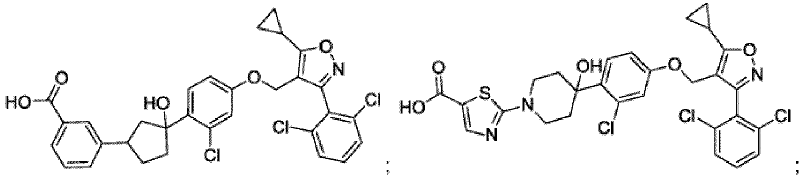 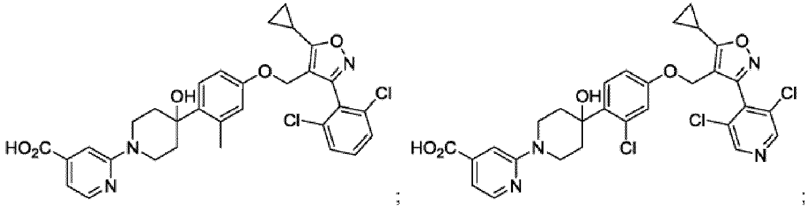 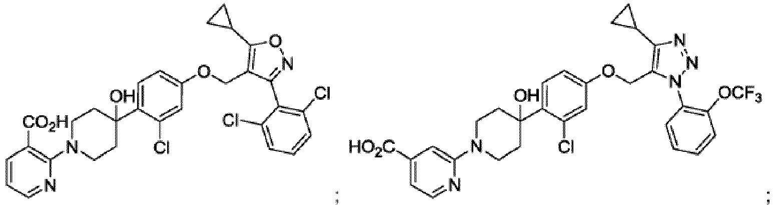 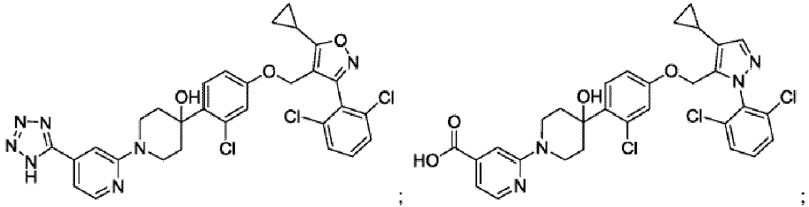 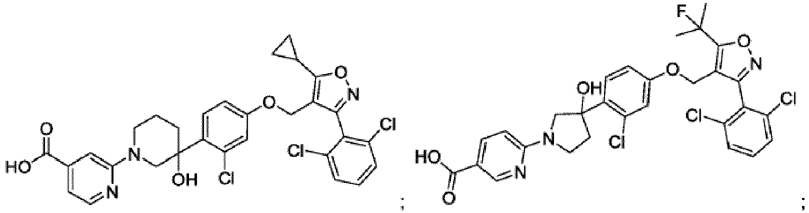 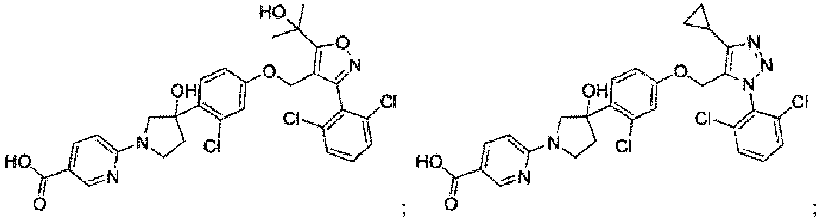 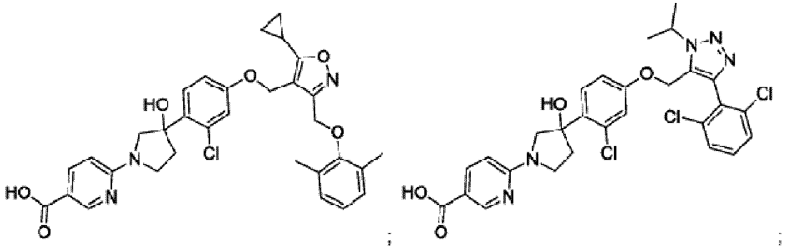 et 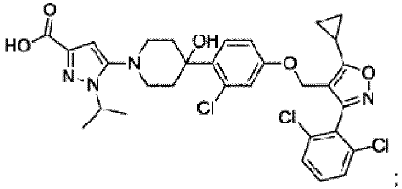 ou un énantiomère, un diastéréoisomère, un tautomère, un solvate ou un sel pharmaceutiquement acceptable de ceux-ci.

12. Composé selon l'une quelconque des revendications 1 à 11 pour une utilisation en tant que médicament.

13. Composé selon l'une quelconque des revendications 1 à 11 pour une utilisation dans la prophylaxie et/ou le traitement de maladies induites par FXR.

14. Composé pour une utilisation selon la revendication 13, où la maladie est choisie parmi:
états intra-hépatiques chroniques ou certaines formes d'états cholestatiques extra-hépatiques;
fibrose du foie;
troubles inflammatoires obstructifs ou chroniques du foie;
cirrhose du foie;
stéatose du foie et syndromes associés, effets cholestatiques ou fibreux qui sont associés à une cirrhose induite par l'alcool ou à des formes d'origine virale d'hépatite;
insuffisance du foie ou ischémie du foie après une résection majeure du foie;
stéatohépatite associée à une chimiothérapie (CASH);
insuffisance du foie aiguë; et
maladies intestinales inflammatoires.

15. Composé pour une utilisation selon la revendication 13, où la maladie est choisie parmi:
troubles lipidiques et lipoprotéiniques;
diabète de Type II et complications cliniques d'un diabète de Type I et de Type II, incluant néphropathie diabétique, neuropathie diabétique, rétinopathie diabétique et d'autres effets observés d'un diabète à long terme se manifestant cliniquement;
états et maladies qui résultent d'une dégénérescence graisseuse et fibreuse chronique d'organes due à une accumulation forcée de lipides et spécifiquement de triglycérides et une activation subséquente de voies pro-fibreuses, telles que maladie du foie gras non alcoolique (NAFLD) ou stéatohépatite non alcoolique (NASH);
obésité ou syndrome métabolique (états combinés de dyslipidémie, diabète ou indice de masse corporelle anormalement élevé); et
infarctus aigu du myocarde, attaque aiguë ou thrombose qui se produit comme un point final d'athérosclérose obstructive chronique.

16. Composé pour une utilisation selon la revendication 13, où la maladie est choisie parmi:
troubles hyperprolifératifs non malins et troubles hyperprolifératifs malins, spécifiquement de carcinome hépatocellulaire, adénome du côlon et polypose, adénocarcinome du côlon, cancer du sein, adénocarcinome du pancréas, oesophage de Barrett ou d'autres formes de maladies néoplasiques du tractus gastro-intestinal et du foie.
